# EUROPEAN PATENT SPECIFICATION

(11) **EP 4 236 931 B1**
(45) Date of publication and mention of the grant of the patent: **09.10.2024**
(21) Application number: 21811683.8
(22) Date of filing: 29.10.2021
(51) Int. Cl.: A61K 31/15, A61K 31/198, A61K 31/352, A61K 31/4045, A61K 38/21, A61P 11/00

(54) **COMPOSITIONS COMPRISING TETRAHYDROCANNABINOL FOR TREATING ACUTE RESPIRATORY FAILURE AND/OR ACUTE RESPIRATORY DISTRESS SYNDROME**
ZUSAMMENSETZUNGEN ENTHALTEND TETRAHYDROCANNABINOL ZUR BEHANDLUNG VON AKUTER ATEMINSUFFIZIENZ UND/ODER AKUTEM ATEMNOTSYNDROM
COMPOSITIONS COMPRENANT DU TETRAHYDROCANNABINOL DESTINÉES AU TRAITEMENT D'INSUFFISANCE RESPIRATOIRE AIGUË ET/OU DU SYNDROME DE DÉTRESSE RESPIRATOIRE AIGUË

(30) Priority: 29.10.2020 US 202063107201 P; 30.10.2020 US 202063107873 P; 21.12.2020 US 202063128755 P; 07.01.2021 US 202163134919 P; 20.08.2021 US 202163235659 P; 06.10.2021 US 202163252912 P
(43) Date of publication of application: 06.09.2023
(73) Proprietor: Nagy, Aurangzeb Nafees, Las Vegas, Nevada 89117 (US)
(72) Inventor: Nagy, Aurangzeb Nafees, Las Vegas, Nevada 89117 (US)
(74) Representative: Wildhack & Jellinek Patentanwälte
(86) International application number: PCT/US2021/057433
(87) International publication number: WO 2022/094330

(56) References cited:
- WO-A1-2020/188551
- WO-A1-2021/191888
- WO-A1-2021/209998
- WO-A1-2021/224924
- WO-A1-2021/231810
- CLINICALTRIALS.GOV: "NCT04342663 A Double-blind, Placebo-controlled Clinical Trial of Fluvoxamine for Symptomatic Individuals With COVID-19 Infection | Results Posted", 9 July 2021 (2021-07-09), pages 1 - 15, XP093092044, Retrieved from the Internet <URL:https://clinicaltrials.gov/study/NCT04342663?tab=results#results-overview> [retrieved on 20231016]
- MOHAMMED AMIRA ET AL: "[Delta]9-Tetrahydrocannabinol Prevents Mortality from Acute Respiratory Distress Syndrome through the Induction of Apoptosis in Immune Cells, Leading to Cytokine Storm Suppression", INTERNATIONAL JOURNAL OF MOLECULAR SCIENCES, vol. 21, no. 17, 1 January 2020 (2020-01-01), pages 6244, XP055896316, Retrieved from the Internet <URL:https://www.ncbi.nlm.nih.gov/pmc/articles/PMC7503745/pdf/ijms-21-06244.pdf> DOI: 10.3390/ijms21176244
- RAMLALL VIJENDRA ET AL: "Melatonin is significantly associated with survival of intubated COVID-19 patients", MEDRXIV, 18 October 2020 (2020-10-18), pages 1 - 37, XP055896548, Retrieved from the Internet <URL:https://www.medrxiv.org/content/10.1101/2020.10.15.20213546v1.full.pdf> [retrieved on 20220301], DOI: 10.1101/2020.10.15.20213546
- TANAKA TOSHIO ET AL: "Immunotherapeutic implications of IL-6 blockade for cytokine storm", IMMUNOTHERAPY, FUTURE MEDICINE, LONDON, vol. 8, no. 8, 1 July 2016 (2016-07-01), pages 959 - 970, XP008184780, ISSN: 1750-7448, [retrieved on 20160706], DOI: 10.2217/IMT-2016-0020
- NAGARKATTI PRAKASH ET AL: "Use of Cannabinoids to Treat Acute Respiratory Distress Syndrome and Cytokine Storm Associated with Coronavirus Disease-2019", FRONTIERS IN PHARMACOLOGY, vol. 11, 6 November 2020 (2020-11-06), XP055896198, DOI: 10.3389/fphar.2020.589438
- RAJ VINIT ET AL: "Assessment of antiviral potencies of cannabinoids against SARS-CoV-2 using computational and in vitro approaches", INTERNATIONAL JOURNAL OF BIOLOGICAL MACROMOLECULES, ELSEVIER BV, NL, vol. 168, 5 December 2020 (2020-12-05), pages 474 - 485, XP086448442, ISSN: 0141-8130, [retrieved on 20201205], DOI: 10.1016/J.IJBIOMAC.2020.12.020
- ZHOU QIONG ET AL: "Interferon-alpha2b Treatment for COVID-19", FRONTIERS IN IMMUNOLOGY, vol. 11, 15 May 2020 (2020-05-15), Lausanne, CH, XP055849492, ISSN: 1664-3224, DOI: 10.3389/fimmu.2020.01061
- RANGEL-MÉNDEZ JORGE-AARÓN; ROSA-ESTER MOO-PUC: "N-acetylcysteine as a potential treatment for COVID-19", FUTURE MICROBIOLOGY, vol. 15, no. 11, 1 July 2020 (2020-07-01), GB, pages 959 - 962, XP055759564, ISSN: 1746-0913, DOI: 10.2217/fmb-2020-0074
- HOERTEL NICOLAS ET AL: "SSRIs and SNRIs and Risk of Death or Intubation in COVID-19: Results from an Observational Study", MEDRXIV, 14 July 2020 (2020-07-14), pages 1 - 37, XP055926695, Retrieved from the Internet <URL:https://www.medrxiv.org/content/10.1101/2020.07.09.20143339v1.full.pdf> [retrieved on 20220531], DOI: 10.1101/2020.07.09.20143339
- CLINICALTRIALS.GOV: "A Double-blind, Placebo-controlled Clinical Trial of Fluvoxamine for Symptomatic Individuals With COVID-19 Infection", 13 April 2020 (2020-04-13), pages 1 - 8, XP055928475, Retrieved from the Internet <URL:https://clinicaltrials.gov/ct2/show/NCT04342663> [retrieved on 20220607]

## Description

This Patent Cooperation Treaty application claims the benefit of priority of United States Provisional Applications 63/107,201 filed on October 29, 2020, 63/107,873 filed October 30, 2020, 63/128,755 filed on December 21, 2020, 63/134,919 filed on January 7, 2021, 63/235,659 filed on August 20, 2021, and 63/252,912 filed October 6, 2021.

### FIELD OF THE INVENTION

The present invention relates generally to methods, combinations, and compositions for use in treating acute respiratory failure (ARF) and/or acute respiratory distress syndrome (ARDS) caused, for example, by viral infections such as COVID-19, using tetrahydrocannabinol (THC) and compositions or combinations including same.

### BACKGROUND

Acute respiratory failure, as seen in patients with acute respiratory distress syndrome (ARDS) caused by viral infections such as COVID-19, can be serious.

Coronavirus Disease 2019 (COVID-19) is a pandemic severely impacting the health and lives of the entire human population on the planet. From an estimated first infection in the Fall of 2019 to November 2020, more than 55 million cases of COVID-19 have been confirmed worldwide, and more than 1.3 million deaths attributable to COVID-19 have been recorded. Severe Acute Respiratory Syndrome Coronavirus 2 (SARS-CoV-2) is a member of the Coronaviridae family, genus Betacoronavirus, which causes COVID-19. SARS-CoV-2 has an estimated basic reproductive number (R₀) of between 1.4 and 5.7 secondary infections per infected index case, making it a highly contagious and rapidly spreading virus.

There are six other coronaviruses that are known to infect humans. Four of these cause the common cold, and the other two cause potentially lethal diseases. These other two coronaviruses are severe acute respiratory syndrome coronavirus (SARS-CoV) and Middle East respiratory syndrome coronavirus (MERS-CoV). These two viruses are believed to have originated in animal populations.

SARS-CoV-2 is a positive-sense, single-stranded RNA virus with linear RNA that is currently believed to primarily spread from infected human patients via respiratory droplets. The full genome of SARS-CoV-2 is about 30,000 bases in length and has been sequenced from RNA extracted from patient samples. SARS-CoV-2 includes four structural proteins: spike or S protein; envelope or E protein; membrane or M protein; and nucleocapsid or N protein. The spike, E, and M proteins enclose the viral genetic core, which includes the linear RNA. The RNA is tightly packed within the core by the N proteins. The spike protein is the portion of the virus that is thought to interact with cell membrane surface proteins in target cells to allow the virus to enter those cells. The spike protein is disposed on the surface/envelope of the virus.

On day zero of infection with the SARS-CoV-2 virus, the virus infects the epithelium of the nasal sinuses. It then travels down to the lungs, and by about day two, the viral load in the lungs is similar to that in the nasal passages. By about day four, the infectious process of the lungs is well underway with typical symptoms of viral lower respiratory infection (dry cough, sore throat, shortness of breath, fever, etc.). By about day seven, the viral activity has typically mostly been suppressed by patients with a normally functioning immune system, although the viral RNA continues to be detectable as far as 21 days from onset of symptoms. Age correlates positively with poor outcome, likely due to immune senescence normally found in the aged.

SARS-CoV-2 seems to affect some people more severely than others. While most infected patients have mild symptoms, others have high morbidity and may die. Much speculation exists as to why this occurs. Experts surmise that 50% of the reason is genetic. Patients with type-A blood or with certain HLA types are more susceptible; however, these observations do not adequately explain the difference.

The COVID-19 disease process branches into three pathways at day seven. A large percentage of patients will begin to recover. A second population of patients may develop cytokine storm. A third population of patients does not recover from the disseminated viral infection, develops diffuse organ failure, and succumbs.

In the cytokine storm pathway, cytotoxic immune cells (*e.g*., cytotoxic T lymphocytes and natural killer cells) attack target cells infected with the SARS-CoV-2 virus, which are presenting viral proteins on their surfaces. Normally, the cytotoxic immune cells will produce a protein called perforin that penetrates the target cell membrane. The cytotoxic immune cells will then release multiple cytotoxic agents into the perforated target cell, which cause apoptosis. Simultaneously, the cytotoxic immune cells secrete other cytokines (*e.g*., transforming growth factor beta (TGF-β), Interleukin 6 (IL-6)) that summon macrophages to the site in order to clean up the debris formed during apoptosis.

In 10-15% of the population, one of the two copies of the perforin gene is defective, making these patients susceptible to perforin malfunction. In the right circumstances, these patients will experience impairment in the ability of the cytotoxic immune cells to utilize perforin to kill the target cell infected with the virus. However, the cytotoxic immune cells will continue to secrete cytokines to summon macrophages to the area. The macrophages that are summoned to the area, although initially summoned for cleanup, also release their own cytokines. In the resulting cytokine storm, the macrophages increase the deposition of fibrin. This macrophage induced fibrin secretion, along with extracellular matrix secreted by fibroblasts, leads to areas of pulmonary fibrosis/restriction in COVID-19, and the ground-glass appearance seen on chest X-rays. A build-up of TGF-β causes the epithelial cells lining the lungs to convert into connective tissue fibroblasts. The combination of excess fibrin and fibroblasts may increase inappropriate blood clotting, which has been seen frequently in COVID-19. Accordingly, patients with COVID-19 may have an increased disposition to develop stroke and inappropriate blood clotting in the small vessels of the extremities.

The virus infects many cells rapidly starting in the nasal passages and then the lungs. The virus progresses rapidly because the molecular target for the virus that allows entry into cells is so widely expressed on so many cells and cell types. In most individuals, the body's natural immune system is able to clear the virus. In a few individuals, the body fails to clear the virus and a cytokine storm develops, which often goes on to kill the patient. Currently the cytokine storm is treated with dexamethasone and IL-6 antagonists while the virus itself is treated with remdesivir.

Cytokine storm occurs when cytotoxic CD8+ T cell or natural killer (NK) cell lines attach to a virus-infected cell, and the virus-infected cell fails to die. Specifically, after NK cells and CD8+ T-cells bind to a cell infected with the SARS-CoV-2 virus, they release a molecule called perforin that makes a perforation in the target cell, and other toxins from the CD8+ T-cells or NK cells enter the target cell causing the cell to undergo apoptosis and die. During this process, the NK and CD8+ T-cells secrete cytokines until the target cell undergoes apoptosis, and then they stop secreting pro-inflammatory cytokines and switch to producing anti-inflammatory cytokines. In some patients the perforin does not function properly (5 to 15% of the population carries defective perforin genes). The target cell then never undergoes apoptosis, so the NK cell and the CD8+ T-cells keep producing pro-inflammatory cytokines, causing the cytokine storm.

The SARS-CoV-2 virus passes into the bloodstream in the area of the alveolar capillaries due to direct attack on the vessels by the virus, oxidative damage to endothelial cells caused by released exotoxin vesicles from white blood cells, and due to the increased permeability of the blood vessels caused by the inflammatory response. The virus then disseminates through the blood to the intestinal mucosa, kidneys, and spleen with demonstrable presence of virus and damage in those areas by day four after infection.

One theory of infection is that the SARS-CoV-2 spike protein binds to type 2 angiotensin-converting enzyme (ACE2) receptors on target human cells to facilitate SARS-CoV-2 entry into those cells. ACE2 receptors are present in most cells in the human body, but they are highly expressed on cilia-containing cell membranes, including those of lung alveolar type II cells, epithelial cells of the gastrointestinal system, and renal tubule epithelium in the kidney. ACE2 receptors normally cleave angiotensin two creating smaller protein fragments that have anti-inflammatory properties. When large numbers of SARS-CoV-2 virus are present, much of the ACE2 receptor is bound, reducing cleavage of angiotensin two, which builds up. According to some theories, the loss of the anti-inflammatory protein fragment cleavage products contributes to the aggressive inflammatory response cause by the SARS-CoV-2 virus. According to other theories, other effects of the buildup of angiotensin two include cardiomyopathy (SARS-CoV-2 virus binding to cardiocyte ACE2 receptors) and stroke (SARS-CoV-2 virus binding to cerebrovascular endothelial cells ACE2 receptors).

After the SARS-CoV-2 virus binds to the human ACE2 receptor, surface proteases, such as transmembrane serine protease 2 (TMPRSS2), facilitate proteolytic activation, fusion, and internalization of the SARS-CoV-2 virus into endosomes in the target cell. After the SARS-CoV-2 virus has been internalized into target cell endosomes, lysosomal cathepsin (CTSL) proteins release the SARS-CoV-2 virus into the cytoplasm of the target cell to further the infection. The binding, internalization, and release of the SARS-CoV-2 virus in the target cell thereby facilitate SARS-CoV-2 infection of the target cells. Kovalchuk et al. report using Cannabis Sativa to reduce the levels of ACE2 receptors in oral, lung, and intestinal epithelial tissues to prevent entry of SARS-CoV-2, and, thereby, to treat or prevent cytokine storm that may lead to ARDS³⁸.

The list of reported symptoms of COVID-19 from sources such as the United States Centers for Disease Control and Prevention (CDC) is growing and evolving. A large number of varied symptoms have been reported from different groups of patients worldwide. COVID-19 symptoms can range from mild to severe disease. COVID-19 symptoms generally appear between 2 to 14 days after exposure to SARS-CoV-2 virus particles. Common symptoms of COVID-19 include cough, shortness of the breath, fever, and fatigue. Other symptoms such as headache, chills, muscle or joint aches, and sore throat can be seen in a number of patients. Impairment of taste and smell has also been reported. Liver enzyme abnormalities and a tendency to form blood clots may occur during infection.

Patients with severe or critical disease often show evidence of a cytokine release syndrome (cytokine storm) with manifestations of progressive pneumonia, respiratory failure, kidney failure, or hypotension, frequently resulting in death. The evidence of cytokine storms in severely or critically ill patients includes high levels of cytokines (*e.g*., interleukin-6) in the blood of such patients. As described herein, during a cytokine storm, a patient's body begins to attack their own cells and tissues in addition to fighting infection.

According to some theories, cytokine storm in certain patients infected with the SARS-CoV-2 virus results from the virus' ability to quickly replicate in infected cells. These cells respond by releasing large amounts of cytokines to kill themselves to prevent spread of the disease. Unfortunately, the cytokine storm resulting from the large amounts of cytokine proteins also kills neighboring cells. In SARS-CoV-2 virus infections, much of the cell death occurs in lung tissue, resulting in damage to the gas transferring surfaces of the lungs, which can be exacerbated by becoming filled with fluids (*i.e*., "waterlogged"). Diffuse alveolar damage can result from cell death in lung tissue and can include the deposition of hyaline membranes made up of dead cells, proteins, and surfactant. Hyaline membrane deposition can limit gas exchange in the lungs. This lung damage can result in pneumonia with the patient dying from an inability to manage gas exchange (*e.g*., lack of oxygen, too much carbon dioxide).

The pulmonary effects of the cytokine storm can lead to acute respiratory failure (ARF), including acute respiratory distress syndrome (ARDS). ARF and/or ARDS are characterized by the above-mentioned pulmonary edema, radiographic findings (*e.g*., diffuse bilateral opacities), and hypoxemia. As such, ARF and/or ARDS can lead to respiratory failure and an increase in mortality. Without an approved drug therapy for ARF and/or ARDS, ARF and/or ARDS treatment is limited to symptomatic management and supportive care (*e.g*., with ventilators). Unfortunately, the mortality rate of ARF and/or ARDS is about 40%.

As described above, a significant portion of the morbidity and mortality caused by COVID-19 is due to ARF and/or ARDS resulting at least partially from the cytokine storm that develops as a result of the infection. IL-6 is a pro-inflammatory cytokine that is a potential therapeutic target for suppression of the cytokine storm (*e.g*., using IL-6 antagonists).

Tocilizumab is a recombinant monoclonal antibody (*i.e*., IgG) directed against the IL-6 receptor. As such, Tocilizumab could bind and physically block the IL-6 receptors on a patient's cells involved in the cytokine storm, thereby disrupting the IL-6 pro-inflammatory pathway, as described by Tanaka et al.⁵ Unfortunately, Tocilizumab has limited bioavailability. Antibodies such as Tocilizumab have a size of about 150 kD, possibly preventing them from passing through the pores in capillaries. This size restriction may limit the activity of antibodies such as Tocilizumab to the intravascular compartment and areas in which blood vessels have already become "leaky," due to the action of inflammatory factors.

Interferon (IFN) includes Type I IFN and Type III IFN. Type I IFN, which includes Interferon-β (IFN-β) and Interferon-α (IFN-α), have broad spectrum antiviral effects. Type III IFN includes Interferon-λ (IFN-λ).

IFN-α (*e.g*., IFN-α2b) has been shown to upregulate expression of major histocompatibility complex class I (MHC I) proteins, which increases presentation of viral antigen peptides. Increased viral antigen peptide presentation in turn increases activation of CD8+ T cell, which then form cytotoxic T lymphocytes (CTLs). Increased CTLs enhance CTL-mediated apoptosis (*e.g*., by macrophages). IFN-α also increases synthesis of other antiviral mediators, such as protein kinase R and 2'-5' oligoadenylate synthetase (2'-5' A synthetase). IFN-α unfortunately increases blood levels of inflammatory proteins interleukin-6 (IL-6) and C reactive protein (CRP). IFN-α activates expression mechanisms for these antiviral mediators by binding to type I interference receptors, leading to phosphorylation of the receptors, which activate transcription through signal transducers and activators of transcription (STAT). IFN-α2b has been disclosed as a treatment for COVID-19 by Zhou et al.; the exploratory testing showed that the detectable virus in the upper respiratory trace was reduced along with a reduction in elevted blood lievs for the inflammatory markers IL-6 and CRP⁴².

IFN-β (*e.g*., IFN-β-1a) has been shown to upregulate the CD73 enzyme, an enzyme which reduces the severity of ARF and/or ARDS by reducing recruitment of leukocytes to the infected area and minimizing vascular leakage. The CD73 enzyme generates adenosine, which has anti-inflammatory properties, reduces endothelial cell permeability, and increases vascular integrity under hypoxic conditions. The CD73 enzyme generates adenosine by converting ADP (which is pro-thrombotic) and ATP (which is pro-inflammatory).

IFN-λ (*e.g*., IFN-λ-1, IFN-λ-2, IFN-λ-3, and/or IFN-λ-4) has been shown to initiate an early-stage antiviral immune response at epithelial surfaces. IFN-λ and IL-28Rα activate tyrosine kinases (TYK2 and JAK1) to phosphorylate IL-28Rα, which bind to STAT proteins leading to generate various antiviral proteins. IFN-λ is thought to induce less IL-6 production than IFN-α or IFN-β.

Because one of the primary sites of COVID-19 infection leading to morbidity and mortality is a patient's lungs, delivering medications to patients suffering from COVID-19 infection via an inhalatory pathway can efficiently treat COVID-19 patients. Unfortunately, COVID-19 can lead to diffuse alveolar damage, which can include buildup of hyaline membranes in the alveoli that reduces the ability of the alveoli to absorb medications delivered via an inhalatory pathway.

THC has been used in mice to treat enterotoxin-induced ARDS in studies by Nagarkatti et al. from the University of South Carolina School of Medicine reported in Frontiers of Pharmacology.²⁴ Investigators from the same medical school tested delta-9 tetrahydrocannabinol treatment for enterotoxin-induced ARDS in a similar mouse model, which showed the treatment prevented mortality in one hundred percent of the mice³⁵.

Raj et al. reported findings that suggested that cannabidiol and Delta-9 tetrahydrocannabinol were possible drugs that might be used to treat patients with COVID-19.³⁶

Ramlall et al. studied melatonin treatment of patients after intubation. Melatonin exposure after intubation was significantly associated with a positive outcome in COVID-19 patients and non-COVID-19 patients.³⁷

Molad reported use of a cannabinoid in combination with a pyrazine in a specific formulation for treating respiratory symptoms in mammals. The respiratory symptoms included COVID-19 and ARDS³⁹.

Another usage of a cannabis-related composition is reported by Baban et al., who used cannabidiol to reduce ARDS caused by COVID-19⁴⁰.

Another treatment for respiratory distress, including from coronavirus and ARDS, was disclosed by losub-Ami et al. In this disclosure, cannabidiolic acid ester alone or in combination with one or more additional cannabinoid comounds was used for treating respiratory distress.⁴¹

Rangel-Mendez et al, proposed the use of N-acetylcysteine as a potential treatment, preventative,or adjuvant against COVID-19⁴³.

Another use for cannabinoid combinations has been disclosed by Gedo in PCT Application Number WO 2020/188551. Gedo reports that administering cannabinoid combinations improves the efficacy of treatment of patients with autism spectrum disorder and associated disorders.⁴⁴

Hoertel et al. hypothesized that SSRIs and SNRls might be effective to treat COVID-19 patients, due to their anti-inflammatory and potential antiviral efffects. Their study showed that exposure to escitalopram, fluoxetine, and venlafaxine was significantly associated with lower risk of intubation or death.⁴⁵

Lenze of Washington School of Medicine initiated a ransomized study on April 8, 2020, to test treatment of COVID-19 patients with fluvoxamine.⁴⁶ Lenze has published research results on the usage of fluvoxamine to treat COVID-19 at ClinicalTrials.Gov: NCTo432663, "A Double-blind, Placebo-controlled Clinical Trial of Fluvoxamine for Symptomatic Individuals with COVID-19 Infrection | Results Posted," 9 July 2021 (2021-07-09), pages 1-15, , URL: https://clinicaltrials.gov/study/NCT04342663?tab=results.

Accordingly, there is a need for treatment of ARF and/or ARDS, optionally resulting from COVID-19 infection.

### SUMMARY

The invention is set out in the appended set of claims. Subject-matter in relation to 'analogs, derivatives, intermediates, metabolites or fragments' is not within the scope of the invention. Any references in the description to methods of treatment refer to the compounds, pharmaceutical compositions, and medicaments of the present invention for use in a method for treatment of the human (or animal) body for therapy.

Embodiments are directed to compositions, combination treatments, and methods for treating ARF and/or ARDS (optionally caused by COVID-19) using THC compositions. In particular, some embodiments are directed to methods for treating ARF and/or ARDS (optionally caused by COVID-19) using compositions or combinations including THC, derivative, intermediate, metabolite, or fragment thereof, and/or combinations thereof and a fluvoxamine, derivative, intermediate, metabolite, or fragment thereof, and/or combinations thereof; and, optionally, an interferon (IFN), derivative, intermediate, metabolite, or fragment thereof, and/or combinations thereof; and, optionally, an acetylcysteine, derivative, intermediate, metabolite, or fragment thereof, and/or combinations thereof.

A first aspect, not covered by the claims, includes a composition comprising a tetrahydrocannabinol (THC) or analog thereof, wherein the analog is a derivative, intermediate, metabolite, or fragment thereof, and/or combinations thereof, for use in treating a subject with late-stage acute respiratory failure (ARF) and/or acute respiratory distress syndrome (ARDS).

Another aspect includes a composition comprising a tetrahydrocannabinol (THC) or analog thereof, wherein the analog is a derivative, intermediate, fragment or metabolite (such as 11-OH-delta-9-tetrahydrocannabinol) thereof, and/or combinations thereof and a fluvoxamine or analog thereof, wherein the analog is a derivative, intermediate, metabolite, or fragment thereof, and/or combinations thereof. In some embodiments, the THC, derivative, intermediate, metabolite, or fragment thereof, is selected from the group consisting of THCA (Tetrahydrocannabinolic acid), THC (Tetrahydrocannabinol), THCVA (Tetrahydrocannabivarin), CBG (Cannabigerol), THCV (Tetrahydrocannabivarin), and combinations thereof. In some embodiments, the THC, derivative, intermediate, metabolite, or fragment thereof is Δ9-THC. In further embodiments, the THC is dronabinol.

Another aspect includes a combination treatment comprising: a composition comprising a tetrahydrocannabinol (THC) or analog thereof, wherein the analog is a derivative, intermediate, metabolite, or fragment thereof, and/or combinations thereof; and a composition comprising a fluvoxamine or analog thereof, wherein the analog is a derivative, intermediate, metabolite, or fragment thereof, and/or combinations thereof.

Another aspect includes a method of treating ARF and/or ARDS in a subject in need thereof, the method comprising administering to the subject compositions as described herein, for example, a composition comprising a THC and/or analog and a fluvoxamine and/or analog described herein or a combination treatment described herein.

Another aspect includes use of compositions as described herein for example a composition comprising a THC and/or analog and a fluvoxamine and/or analog described herein or a combination treatment described herein for treating ARF and/or ARDS in a subject.

Another aspect includes use of compositions as described herein for example a composition comprising a THC and/or analog and a fluvoxamine and/or analog described herein or a combination treatment described herein in the manufacture of a medicament for treating ARF and/or ARDS in a subject.

Another aspect comprises a package comprising one or more of THC and/or an analog thereof, a fluvoxamine and/ or an analog thereof, an acetylcysteine and/or an analog thereof, and/or an IFN and/or analog thereof. In an embodiment, the package further comprises, bronchodilator, and a sterile vial. In another embodiment, the package comprises any one of the compositions described herein, for example, a composition comprising a THC and/or analog and a fluvoxamine and/or analog described herein, optionally in a sterile vial.

In one embodiment, a method of treating acute respiratory failure syndrome (ARF) (optionally caused by COVID-19) in a subject in need thereof includes administering to the subject a therapeutically effective amount of a composition or combination described herein. The composition or combination includes a tetrahydrocannabinol (THC), derivative, intermediate, metabolite or fragment thereof, and/or combinations thereof. The THC may be in the composition or combination in an amount of from about 0.5 mg/mL to about 30 mg/mL. In some embodiments, the composition or combination also includes an interferon (IFN), derivative, intermediate, metabolite or fragment thereof, and/or combinations thereof. In other embodiments, the composition or combination further includes a fluvoxamine, derivative, intermediate, metabolite or fragment thereof, and/or combinations thereof. In some embodiments, the composition includes an acetylcysteine, derivative, intermediate, metabolite, or fragment thereof, and/or combinations thereof. In some embodiments, the composition or combination may be administered using a nebulizer. In other embodiments, the composition or combination may be administered via an intramuscular injection. In other embodiments, the composition may be administered intravenously, orally (*e.g*., as an oral solution, capsule, combustible, inhalatory, and/or food product), and/or via a suppository. The dose required in any route in order to achieve the same blood concentration of THC as obtained with 0.5 mg-30 mg dronabinol taken orally twice daily would be the amount required for that route of administration. This can be determined with bioavailability studies in animals and then in people by standard methods. For example, the blood concentration of THC and its active metabolites associated with 5 mg dronabinol orally BID (twice daily) would be one dose known to be useful, and intravenous, oral, transdermal, rectal, sublingual, nasal or other dosing routes could be dosed with an amount of drug that creates a similar blood concentration of THC. Similarly, for compositions or combinations involving fluvoxamine, the dose of drug given by any given route would be adjusted to achieve the same blood concentration of fluvoxamine as would be obtained for example by giving about 25 mg - about 450 mg, optionally about 50 mg to about 300 mg, optionally about 50 mg to about 200 mg, optionally about 75 mg to about 200 mg orally twice daily.

In another embodiment, a method of treating acute respiratory failure syndrome (ARF) and/or ARDS (optionally caused by COVID-19) in a subject in need thereof includes administering to the subject a therapeutically effective amount of a composition or combination. In some embodiments, the composition or combination includes a tetrahydrocannabinol (THC), derivative, intermediate, metabolite, or fragment thereof, and/or combinations thereof. The THC may be in the composition or combination in an amount of from about 0.5 mg to about 30 mg, if taken orally, or other, if taken via another route, an amount that would achieve the same blood concentration levels of THC and its active metabolites as orally administered 0.5 mg up about 30 mg would achieve, taking into account the different pharmacokinetics of different routes of administration.

In some embodiments, the composition or combination also includes a fluvoxamine, derivative, intermediate, metabolite or fragment thereof, and/or combinations thereof. In some embodiments, the composition or combination further includes an acetylcysteine, derivative, intermediate, metabolite or fragment thereof, and/or combinations thereof. In some embodiments, the composition may be administered using a nebulizer. In some embodiments, the composition may be administered via an intramuscular injection. In some embodiments, the composition may be administered intravenously, orally (*e.g*., as an oral solution), and/or via a suppository.

In another embodiment, a method of inhalatorily treating acute respiratory failure (ARF) and/or ARDS (optionally caused by COVID-19) in a subject in need thereof may include orally administering to the subject a therapeutically effective amount of a composition or combination using a nebulizer or as an oral solution. In some embodiments, the composition or combination includes a tetrahydrocannabinol (THC), derivative, intermediate, metabolite, or fragment thereof, and/or combinations thereof. In some embodiments, the composition or combination also includes an interferon (IFN), derivative, intermediate, metabolite, or fragment thereof, and/or combinations thereof. In some embodiments, the composition or combination includes a fluvoxamine, derivative, intermediate, metabolite, or fragment thereof, and combinations thereof. In some embodiments, the composition or combination includes an acetylcysteine, derivative, intermediate, metabolite, or fragment thereof, and/or combinations thereof. In some embodiments, the composition or combination may be administered using a nebulizer.

In one or more embodiments, the THC, derivative, intermediate, metabolite, or fragment thereof is present in the composition or combination in a concentration of from about 0.5 mg/mL to about 30 mg/mL. The THC, derivative, intermediate, metabolite, or fragment thereof may be present in the composition from about 5 mg/mL to about 10 mg/mL. A therapeutically effective amount of THC, derivative, intermediate, metabolite, or fragment thereof, and combinations thereof may be about 2.5 mg twice per day (B.I.D.) to about 10 mg twice per day (B.I.D.) A therapeutically effective amount of THC, derivative, intermediate, metabolite, or fragment thereof, and combinations thereof may be about 5 mg twice per day (B.I.D.), about 3.33 mg three times per day (T.I.D.), and/or about 1.67 mg three times per day (T.I.D.) The IFN may be interferon α (IFN-α), interferon β (IFN-β), and/or interferon-λ (IFN-λ). The IFN-α may be IFN-α2b. A therapeutically effective amount of IFN, derivative, intermediate, metabolite, or fragment thereof, and combinations thereof is from about 5 IU (via inhalation) per day or about 9 MIU (via intramuscular injection) per week of IFN-α, from about 6 MIU to about 8 MIU (via inhalation) per day of IFN-β, and/or about 3 IU twice a day (B.I.D.) (via inhalation) or about 180 µg per week (via intramuscular injection) of IFN-λ. A therapeutically effective amount of fluvoxamine, derivative, intermediate, metabolite, or fragment thereof, and combinations thereof may be about 150 mg of fluvoxamine twice per day (B.I.D.), about 100 mg of fluvoxamine twice per day (B.I.D.), and/or about 50 mg of fluvoxamine twice per day (B.I.D) or about 100 mg of fluvoxamine three times per day (T.I.D.), and/or about 50 mg of fluvoxamine three times per day (T.I.D.) A therapeutically effective amount of acetylcysteine, derivative, intermediate, metabolite, or fragment thereof, and combinations thereof may be from about 1 mL to about 10 mL of a 10% solution 3 to 4 times per day. A therapeutically effective amount of acetylcysteine, derivative, intermediate, metabolite, or fragment thereof, and combinations thereof may be from about 2 mL to about 20 mL of a 20% solution 4 to 12 times per day. A therapeutically effective amount of acetylcysteine, derivative, intermediate, metabolite, or fragment thereof, and combinations thereof may be about 600 mg twice per day (B.I.D.) The method may also include administering to the subject a therapeutically effective amount of a bronchodilator about 10 to about 15 minutes before administering the composition or combination, and up to 12 times per day. The ARF may include acute respiratory distress syndrome (ARDS) caused by COVID-19 in the subject.

In still another embodiment, a composition or combination includes a tetrahydrocannabinol (THC), derivative, intermediate, metabolite, or fragment thereof, and combinations thereof; and further includes a fluvoxamine, derivative, intermediate, metabolite, or fragment thereof, and combinations thereof. In another embodiment, the composition or combination also includes an interferon (IFN), derivative, intermediate, metabolite, or fragment thereof, and combinations thereof. Moreover, in another embodiment, the composition or combination includes an acetylcysteine, derivative, intermediate, metabolite, or fragment thereof, and combinations thereof. The composition or combination may be administered using a nebulizer.

In one or more embodiments, the THC, derivative, intermediate, metabolite, or fragment thereof is selected from the group consisting of THCA, THC, THCVA, THCV, CBG, 11-OH-delta-9-tetrahydrocannabinol and combinations thereof. The THC, derivative, intermediate, metabolite, or fragment thereof may be Δ9-THC. The THC, derivative, intermediate, metabolite, or fragment thereof may be present in the composition or combination from in an amount of from about 0.5 mg/mL to about 30 mg/mL. The THC, derivative, intermediate, metabolite, or fragment thereof may be present in the composition or combination in an amount of from about 5 mg/mL to about 10 mg/mL. The IFN may be interferon α (IFN-α), interferon β (IFN-β), and/or interferon-λ (IFN-λ). The IFN-α may be IFN-α2b. A therapeutically effective amount of IFN, derivative, intermediate, metabolite, or fragment thereof, and combinations thereof is from about 5 IU (via inhalation) per day or about 9 MIU (via intramuscular injection) per week of IFN-α, from about 6 MIU to about 8 MIU (via inhalation) per day of IFN-β, and/or about 3 IU twice a day (B.I.D.) (via inhalation) or about 180 µg per week (via intramuscular injection) of IFN-λ. The acetylcysteine, derivative, intermediate, metabolite, or fragment thereof may be present in the composition or combination in an amount of about 600 mg. The fluvoxamine, derivative, intermediate, metabolite, or fragment thereof, and combinations thereof may be present in the composition or combination in an amount of fluvoxamine of about 25 mg to about 450 mg, for example 75 mg to about 300 mg, to be administered for example twice per day (B.I.D.) up to a total of 900 mg daily, up to a total of 600 mg daily or up to a total of about 300 mg daily. The composition or combination may also include a pharmaceutical acceptable excipient. The dose ranges would be altered, depending on the route of administration, in order to maintain blood concentrations of active ingredients and their active metabolites at ranges similar to those achieved with oral dronabinol 0.5 mg to 30 mg taken for example twice daily, oral fluvoxamine 25 mg to 450 mg, or other ranges described herein, taken for example twice or three times daily, and the amounts of interferon taken by the routes mentioned above, for THC, fluvoxamine, and the interferons above respectively.

In another aspect, the present disclosure includes a composition or combination comprising a tetrahydrocannabinol (THC) or analog thereof and fluvoxamine or analog thereof, wherein the analog is a derivative, intermediate, metabolite, or fragment thereof, salt, solvate of any of thereof and/or combinations thereof.

In another aspect, the present disclosure includes the composition or combination as described herein for use in the treatment of late-stage stage acute respiratory failure (ARF) and/or acute respiratory distress syndrome (ARDS) caused for example by cytokine storm.

In an embodiment, the present disclosure includes a method of treating late-stage stage acute respiratory failure (ARF) and/or acute respiratory distress syndrome (ARDS) caused by cytokine storm, the method comprising administering THC or analog thereof, and fluvoxamine or analog thereof in a subject in need thereof, wherein the analog is a derivative, intermediate, metabolite, or fragment thereof, salt, solvate of any of thereof and/or combinations thereof.

In another aspect, the present disclosure includes a composition or combination comprising THC or analog thereof and fluvoxamine or analog thereof for use in the manufacture of a medicament for the treatment of late-stage stage acute respiratory failure (ARF) and/or acute respiratory distress syndrome (ARDS) caused by cytokine storm, wherein the analog is a derivative, intermediate, metabolite, or fragment thereof, salt, solvate of any of thereof and/or combinations thereof.

The aforementioned and other embodiments of the invention are described in the Detailed Description which follows.

### BRIEF DESCRIPTION OF THE DRAWINGS

The foregoing and other aspects of embodiments are described in further detail with reference to the accompanying drawings, in which the same elements in different figures are referred to by common reference numerals, wherein:
Fig. 1 is a disease severity versus time graph depicting phases of COVID-19 disease in a patient according to some embodiments.
Fig. 2 is another disease severity versus time graph depicting phases of COVID-19 disease in a patient according to some embodiments.
Fig. 3A depicts a graph showing the comparison of average age in treated and untreated cohorts.
Fig. 3B depicts a graph showing the comparison of gender in treated versus vs control cohorts.
Fig. 3C depicts a graph showing the comparison of the average number of comorbidities in treated and untreated cohorts.
Fig. 4A depicts a graph showing mortality rates in treated and untreated cohorts.
Fig. 4B depicts a graph showing the average length (LOS) in treated and untreated cohorts.
Fig. 4C depicts a graph showing the average early peak CRP in treated and untreated cohorts.

In order to better appreciate how to obtain the above-recited and other advantages and objects of various embodiments, a more detailed description of embodiments is provided with reference to the accompanying drawings. It should be noted that the drawings are not drawn to scale and that elements of similar structures or functions are represented by like reference numerals throughout. It will be understood that these drawings depict only certain illustrated embodiments and are not therefore to be considered limiting of scope of embodiments.

### DETAILED DESCRIPTION OF EMBODIMENTS

Unless otherwise defined, scientific and technical terms used in connection with the present disclosure shall have the meanings that are commonly understood by those of ordinary skill in the art. Further, unless otherwise required by context, singular terms shall include pluralities and plural terms shall include the singular. For example, the term "a cell" includes a single cell as well as a plurality or population of cells. Generally, nomenclatures utilized in connection with, and techniques of, cell and tissue culture, molecular biology, and protein and oligonucleotide or polynucleotide chemistry and hybridization described herein are those well-known and commonly used in the art (see, *e.g*., Green and Sambrook, 2012).

The definitions and embodiments described in particular sections are intended to be applicable to other embodiments herein described for which they are suitable as would be understood by a person skilled in the art.

The recitation of numerical ranges by endpoints herein includes all numbers and fractions subsumed within that range (*e.g*., 1 to 5 includes 1, 1.5, 2, 2.75, 3, 3.90, 4, and 5). It is also to be understood that all numbers and fractions thereof are presumed to be modified by the term "about."

Further, the definitions and embodiments described in particular sections are intended to be applicable to other embodiments herein described for which they are suitable as would be understood by a person skilled in the art. For example, in the following passages, different aspects of the disclosure are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

The compounds described herein may exist in different tautomeric forms and it is intended that any tautomeric forms which the compounds form, as well as mixtures thereof, are included within the scope of the present disclosure.

The compounds described herein may further exist in varying polymorphic forms and it is contemplated that any polymorphs, or mixtures thereof, which form are included within the scope of the present disclosure.

The term "subject" as used herein includes all members of the animal kingdom including mammals, and suitably refers to humans. Thus, the methods and uses of the pre-sent disclosure are applicable to both human therapy and veterinary applications. Patient and subject are used interchangeably herein.

The term "pharmaceutically acceptable" means compatible with the treatment of subjects.

The term "pharmaceutically acceptable carrier" means a non-toxic solvent, dispersant, excipient, adjuvant or other material which is mixed with the active ingredient in order to permit the formation of a pharmaceutical composition, i.e., a dosage form capable of administration to a subject.

The term "pharmaceutically acceptable salt" means either an acid addition salt or a base addition salt which is suitable for, or compatible with, the treatment of subjects.

An acid addition salt suitable for, or compatible with, the treatment of subjects is any non-toxic organic or inorganic acid addition salt of any basic compound.

A base addition salt suitable for, or compatible with, the treatment of subjects is any non-toxic organic or inorganic base addition salt of any acidic compound.

The term "administered" as used herein means administration of a therapeutically effective amount of one or more compounds or compositions of the disclosure to a cell, tissue, organ or subject.

In an embodiment the pharmaceutically acceptable salt is an acid addition salt or a base addition salt. The selection of a suitable salt may be made by a person skilled in the art (see, for example, S. M. Berge, et al., "Pharmaceutical Salts," J. Pharm. Sci. 1977, 66, 1-19).

An acid addition salt suitable for, or compatible with, the treatment of subjects is any non-toxic organic or inorganic acid addition salt of any basic compound. Basic compounds that form an acid addition salt include, for example, compounds comprising an amine group. Illustrative inorganic acids which form suitable salts include hydrochloric, hydrobromic, sulfuric, nitric and phosphoric acids, as well as acidic metal salts such as sodium monohydrogen orthophosphate and potassium hydrogen sulfate. Illustrative organic acids which form suitable salts include mono-, di- and tricarboxylic acids. Illustrative of such organic acids are, for example, acetic, trifluoroacetic, propionic, glycolic, lactic, pyruvic, malonic, succinic, glutaric, fumaric, malic, tartaric, citric, ascorbic, maleic, hydroxymaleic, benzoic, hydroxybenzoic, phenylacetic, cinnamic, mandelic, salicylic, 2-phenoxybenzoic, p-toluenesulfonic acid and other sulfonic acids such as methanesulfonic acid, ethanesulfonic acid and 2-hydroxyethanesulfonic acid. In an embodiment, the mono- or di-acid salts are formed, and such salts exist in either a hydrated, solvated or substantially anhydrous form. In general, acid addition salts are more soluble in water and various hydrophilic organic solvents, and generally demonstrate higher melting points in comparison to their free base forms. The selection criteria for the appropriate salt will be known to one skilled in the art. Other non-pharmaceutically acceptable salts such as oxalates may be used, for example in the isolation of compounds of the disclosure for laboratory use, or for subsequent conversion to a pharmaceutically acceptable acid addition salt.

A base addition salt suitable for, or compatible with, the treatment of subjects is any non-toxic organic or inorganic base addition salt of any acidic compound. Acidic compounds that form a basic addition salt include, for example, compounds comprising a carboxylic acid group. Illustrative inorganic bases which form suitable salts include lithium, sodium, potassium, calcium, magnesium or barium hydroxide as well as ammonia. Illustrative organic bases which form suitable salts include aliphatic, alicyclic or aromatic organic amines such as isopropylamine, methylamine, trimethylamine, picoline, diethylamine, triethylamine, tripropylamine, ethanolamine, 2-dimethylaminoethanol, 2-diethylaminoethanol, dicyclohexylamine, lysine, arginine, histidine, caffeine, procaine, hydrabamine, choline, betaine, ethylenediamine, glucosamine, methylglucamine, theobromine, purines, piperazine, piperidine, N-ethylpiperidine, and polyamine resins. Exemplary organic bases are isopropylamine, diethylamine, ethanolamine, trimethylamine, dicyclohexylamine, choline, and caffeine. The selection of the appropriate salt may be useful, for example, so that an ester functionality, if any, elsewhere in a compound is not hydrolyzed. The selection criteria for the appropriate salt will be known to one skilled in the art.

The compounds of the present disclosure can be suitably formulated in a conventional manner into compositions using one or more carriers. Accordingly, the present disclosure also includes a composition comprising one or more compounds of the disclosure and a carrier. The compounds of the disclosure can be suitably formulated into pharmaceutical compositions for administration to subjects in a biologically compatible form suitable for administration in vivo. Accordingly, the present disclosure further includes a pharmaceutical composition comprising one or more compounds of the disclosure and a pharmaceutically acceptable carrier. In embodiments of the disclosure the pharmaceutical compositions are used in the treatment of any of the diseases, disorders or conditions described herein.

Also provided in an aspect is a combination for treatment comprising a THC composition comprising a tetrahydrocannabinol (THC) or analog thereof or combinations thereof and a fluvoxamine composition comprising a fluvoxamine or analog or combinations thereof. The THC composition can be any composition described herein that comprises THC, an analog thereof or combinations thereof. The fluvoxamine composition can be any composition described herein that comprises fluvoxamine, an analog thereof or combinations thereof. The combination can be used in the methods and uses described herein.

The compounds of the disclosure can be administered to a subject in a variety of forms depending on the selected route of administration, as will be understood by those skilled in the art. For example, a compound of the disclosure is administered by oral, inhalation, parenteral, buccal, sublingual, nasal, rectal, vaginal, patch, pump, topical or transdermal administration and the pharmaceutical compositions or combinations formulated accordingly. In some embodiments, administration is by means of a pump for periodic or continuous delivery. Conventional procedures and ingredients for the selection and preparation of suitable compositions are described, for example, in Remington's Pharmaceutical Sciences (2000 - 20th edition) and in The United States Pharmacopeia: The National Formulary (USP 24 NF19) published in 1999.

Parenteral administration includes systemic delivery routes other than the gastrointestinal (GI) tract, and includes, for example intravenous, intra-arterial, intraperitoneal, subcutaneous, intramuscular, transepithelial, nasal, intrapulmonary (for example, by use of an aerosol), intrathecal, rectal and topical (including the use of a patch or other transdermal delivery device) modes of administration. Parenteral administration may be by continuous infusion over a selected period of time.

In some embodiments, a compound and/or a composition or combination described herein, is orally administered, for example, with an inert diluent or with an assimilable edible carrier, or it is enclosed in hard or soft shell gelatin capsules, or it is compressed into tablets, or it is incorporated directly into or with a food of the diet. It may be in a combustible or non-combustible format. For example, the compound and/or composition or combination described herein may be in a food product, in combustible forms, as well as non-combustible forms such as heat not burn compositions or compositions for use with vaping device. In some embodiments, THC may be provided as cannabis. In some embodiments, fluvoxamine and/or an analog thereof may be provided separately.

In some embodiments, the compound is incorporated with excipient(s) and used in the form of ingestible tablets, buccal tablets, troches, capsules, caplets, pellets, granules, lozenges, chewing gum, powders, syrups, elixirs, wafers, aqueous solutions, and suspensions. In the case of tablets, carriers that can be used include lactose, corn starch, sodium citrate and salts of phosphoric acid. Pharmaceutically acceptable excipients include binding agents (e.g., pregelatinized maize starch, polyvinylpyrrolidone or hydroxypropyl methylcellulose); fillers (e.g., lactose, microcrystalline cellulose or calcium phosphate); lubricants (e.g., magnesium stearate, talc or silica); disintegrants (e.g., potato starch or sodium starch glycolate); or wetting agents (e.g., sodium lauryl sulphate). In embodiments, the tablets are coated by methods well known in the art. In the case of tablets, capsules, caplets, pellets or granules for oral administration, pH sensitive enteric coatings, such as Eudragits^{™} designed to control the release of active ingredients are optionally used. Oral dosage forms also include modified release, for example immediate release and timed-release, formulations. Examples of modified-release formulations include, for example, sustained-release (SR), extended-release (ER, XR, or XL), time-release or timed-release, controlled-release (CR), or continuous-release (CR or Contin), employed, for example, in the form of a coated tablet, an osmotic delivery device, a coated capsule, a microencapsulated microsphere, an agglomerated particle, e.g., as of molecular sieving type particles, or, a fine hollow permeable fiber bundle, or chopped hollow permeable fibers, agglomerated or held in a fibrous packet. Timed-release compositions are formulated, for example as liposomes or those wherein the active compound is protected with differentially degradable coatings, such as by microencapsulation, multiple coatings, etc. Liposome delivery systems include, for example, small unilamellar vesicles, large unilamellar vesicles and multilamellar vesicles. In some embodiments, liposomes are formed from a variety of phospholipids, such as cholesterol, stearylamine or phosphatidylcholines. For oral administration in a capsule form, useful carriers or diluents include lactose and dried corn starch.

In some embodiments, liquid preparations for oral administration take the form of, for example, solutions, syrups or suspensions, or they are suitably presented as a dry product for constitution with water or other suitable vehicle before use. When aqueous suspensions and/or emulsions are administered orally, the compound and/or the composition or combination described herein, is suitably suspended or dissolved in an oily phase that can be combined with emulsifying and/or suspending agents. If desired, certain sweetening and/or flavoring and/or coloring agents can be added. Such liquid preparations for oral administration can be prepared by conventional means with pharmaceutically acceptable additives such as suspending agents (e.g., sorbitol syrup, methyl cellulose or hydrogenated edible fats); emulsifying agents (e.g., lecithin or acacia); non-aqueous vehicles (e.g., almond oil, oily esters or ethyl alcohol); and preservatives (e.g., methyl or propyl p-hydroxybenzoates or sorbic acid). Useful diluents include lactose and high molecular weight polyethylene glycols.

In some embodiments, the composition comprises melatonin as an additive.

It is also possible to freeze-dry the compounds of the disclosure and use the lyophilizates obtained, for example, for the preparation of products for injection.

In some embodiments, a compound of the disclosure is administered parenterally. For example, solutions of a compound of the disclosure can be prepared in water suitably mixed with a surfactant such as hydroxypropylcellulose. In some embodiments, dispersions are prepared in glycerol, liquid polyethylene glycols, DMSO and mixtures thereof with or without alcohol, and in oils. Under ordinary conditions of storage and use, these preparations contain a preservative to prevent the growth of microorganisms. A person skilled in the art would know how to prepare suitable formulations. For parenteral administration, sterile solutions of the compounds of the disclosure are usually prepared, and the pH's of the solutions are suitably adjusted and buffered. For intravenous use, the total concentration of solutes should be controlled to render the preparation isotonic. For ocular administration, ointments or droppable liquids can be delivered, for example, by ocular delivery systems known to the art such as applicators or eye droppers. In some embodiment, such compositions include mucomimetics such as hyaluronic acid, chondroitin sulfate, hydroxypropyl methylcellulose or polyvinyl alcohol, preservatives such as sorbic acid, EDTA or benzyl chromium chloride, and the usual quantities of diluents or carriers. For pulmonary administration, diluents or carriers will be selected to be appropriate to allow the formation of an aerosol.

In some embodiments, a compound and/or a composition described herein, is formulated for parenteral administration by injection, including using conventional catheterization techniques or infusion. Formulations for injection are, for example, presented in unit dosage form, e.g., in ampoules or in multi-dose containers, with an added preservative. In some embodiments, the compositions or combinations take such forms as sterile suspensions, solutions or emulsions in oily or aqueous vehicles, and contain formulating agents such as suspending, stabilizing and/or dispersing agents. In all cases, the form must be sterile and must be fluid to the extent that easy syringability exists. Alternatively, the compounds of the disclosure are suitably in a sterile powder form for reconstitution with a suitable vehicle, e.g., sterile pyrogen-free water, before use.

In some embodiments, compositions or combination for inhalation, optionally nasal administration, are conveniently formulated as aerosols, combustibles, drops, oils, gels and powders. For intranasal administration or administration by inhalation, the compounds described herein can be conveniently delivered in the form of a solution, dry powder or granular formulation or suspension from a pump spray container that is squeezed or pumped by the patient or as an aerosol spray presentation from a pressurized container or a nebulizer. Aerosol formulations typically comprise a solution or fine suspension of the active substance in a physiologically acceptable aqueous or non-aqueous solvent and are usually presented in single or multidose quantities in sterile form in a sealed container, which, for example, take the form of a cartridge or refill for use with an atomising device such as a vaping device. Alternatively, the sealed container is a unitary dispensing device such as a single dose nasal inhaler or an aerosol dispenser fitted with a metering valve which is intended for disposal after use. Where the dosage form comprises an aerosol dispenser, it can contain a propellant which is, for example, a compressed gas such as compressed air or an organic propellant such as fluorochlorohydrocarbon. Suitable propellants include dichlorodifluoromethane, trichlorofluoromethane, dichlorotetrafluoroethane, heptafluoroalkanes, carbon dioxide or another suitable gas. In the case of a pressurized aerosol, the dosage unit is suitably determined by providing a valve to deliver a metered amount. In some embodiments, the pressurized container or nebulizer contains a solution or suspension of the active compound. Capsules (made, for example, from gelatin) and cartridges for use in an inhaler or insufflator are, for example, formulated containing a powder or granular mix of a compound of the disclosure and a suitable powder base such as lactose or starch. The aerosol dosage forms can also take the form of a pump-atomizer.

In some embodiments, the composition is suitably formulated for inhalation and administration by inhalation can be performed using an apparatus or device such as a nebulizer, aerosol spray apparatus, inhaler, vaping device, and/or ventilator.

The compositions may also be formulated for nasogastric tube administration.

Compositions suitable for buccal or sublingual administration include tablets, lozenges, and pastilles, wherein a compound of the disclosure is formulated with a carrier such as sugar, acacia, tragacanth, or gelatin and glycerine. Compositions for rectal administration are conveniently in the form of suppositories containing a conventional suppository base such as cocoa butter.

Suppository forms of the compounds and/or compositions described herein are useful for vaginal, urethral and rectal administrations. Such suppositories will generally be constructed of a mixture of substances that is solid at room temperature but melts at body temperature. The substances commonly used to create such vehicles include theobroma oil (also known as cocoa butter), glycerinated gelatin, other glycerides, hydrogenated vegetable oils, mixtures of polyethylene glycols of various molecular weights and fatty acid esters of polyethylene glycol. See, for example: Remington's Pharmaceutical Sciences, 16th Ed., Mack Publishing, Easton, PA, 1980, pp. 1530-1533 for further discussion of suppository dosage forms.

In some embodiments a compound and/or composition described herein, is coupled with soluble polymers as targetable drug carriers. Such polymers include, for example, polyvinylpyrrolidone, pyran copolymer, polyhydroxypropylmethacrylamide-phenol, polyhydroxyethylaspartamide-phenol, or polyethyleneoxide-polylysine substituted with palmitoyl residues. Furthermore, in some embodiments, a compound and/or composition described herein, is coupled to a class of biodegradable polymers useful in achieving controlled release of a drug, for example, polylactic acid, polyglycolic acid, copolymers of polylactic and polyglycolic acid, polyepsilon caprolactone, polyhydroxy butyric acid, polyorthoesters, polyacetals, polydihydropyrans, polycyanoacrylates and crosslinked or amphipathic block copolymers of hydrogels.

A compound or composition described herein including pharmaceutically acceptable salts and/or solvates thereof is suitably used independently but will generally be administered in the form of a pharmaceutical composition in which the one or more compounds described herein (the active ingredient) is in association with a pharmaceutically acceptable carrier. Depending on the mode of administration, the pharmaceutical composition can comprise from about 0.05 wt% to about 99 wt% or about 0.10 wt% to about 70 wt%, of the active ingredient, and from about 1 wt% to about 99.95 wt% or about 30 wt% to about 99.90 wt% of a pharmaceutically acceptable carrier, all percentages by weight being based on the total composition.

In an embodiment, effective amounts vary according to factors such as the disease state, age, sex and/or weight of the subject. In a further embodiment, the amount of a given compound or compounds that will correspond to an effective amount will vary depending upon factors, such as the given drug(s) or compound(s), the pharmaceutical formulation, the route of administration, the type of condition, disease or disorder, the identity of the subject being treated, but can nevertheless be routinely determined by one skilled in the art.

In an embodiment, the compounds and/or compositions or combinations described herein are administered at least once a week. However, in another embodiment, the compounds and/or compositions or combinations described herein are administered to the subject from about once per two weeks, once per three weeks or once a month. In another embodiment, the compounds and/or compositions or combinations described herein are administered about once per week to about once daily. In another embodiment, the compounds and/or compositions described herein are administered 2, 3, 4, 5 or 6 times daily. The length of the treatment period depends on a variety of factors, such as the severity of the disease, disorder or condition, the age of the subject, the concentration and/or the activity of the compounds of the disclosure, and/or a combination thereof. It will also be appreciated that the effective dosage of the compound used for the treatment may increase or decrease over the course of a particular treatment regime. Changes in dosage may result and become apparent by standard diagnostic assays known in the art. In some instances, chronic administration is required. For example, the compounds can be administered to the subject in an amount and for duration sufficient to treat the subject.

In an embodiment, the subject is a mammal. In another embodiment, the subject is human.

Compounds and/or compositions or combinations described herein are either used alone or in combination with other known agents useful for treating late-stage acute respiratory failure (ARF) and/or acute respiratory distress syndrome (ARDS) caused by cytokine storm. When used in combination with other agents useful in treating late-stage acute respiratory failure (ARF) and/or acute respiratory distress syndrome (ARDS) caused by cytokine storm, a compound and/or composition or combination described herein can be administered contemporaneously with those agents. As used herein, "contemporaneous administration" of two or more substances or compositions to a subject means providing each of the two or more substances or compositions so that they are both active in the individual at the same time. The exact details of the administration will depend on the pharmacokinetics of the two or more substances in the presence of each other, and can include administering the two or more substances within a few hours of each other, or even administering one or more substance within 24 hours of administration of the other(s), if the pharmacokinetics are suitable. Design of suitable dosing regimens is routine for one skilled in the art. In particular embodiments, two or more substances or compositions will be administered substantially simultaneously, i.e., within minutes of each other, or in a single composition that contains one or more, or all of the substances. It is a further embodiment of the present disclosure that a combination of agents is administered to a subject in a non-contemporaneous fashion. In an embodiment, a compound and/or composition described herein, is administered with another therapeutic agent simultaneously or sequentially in separate unit dosage forms or together in a single unit dosage form. Accordingly, the present disclosure provides in one embodiment a single unit dosage form comprising one or more compounds or compositions described herein, an additional therapeutic agent, and a pharmaceutically acceptable carrier.

The dosage of a compound and/or composition or combination described herein varies depending on factors such as the pharmacodynamic properties of the compound, the mode of administration, the age, health and weight of the recipient, the nature and extent of the symptoms, the frequency of the treatment and the type of concurrent treatment, if any, and the clearance rate of the compound in the subject to be treated. One of skill in the art can determine the appropriate dosage based on the above factors. In some embodiments, a compound or composition or combination described herein, is administered initially in a suitable dosage that is adjusted as required, depending on the subject's clinical response. Dosages will generally be selected to maintain a serum level of the compound of the disclosure from about 0.01 µg/cc to about 1000 µg/cc, or about 0.1 µg/cc to about 100 µg/cc. As a representative example, oral dosages of one or more compounds or compositions described herein will range between about 1 mg per day to about 1000 mg per day for an adult, suitably about 1 mg per day to about 500 mg per day, more suitably about 1 mg per day to about 200 mg per day. For parenteral administration, a representative amount is from about 0.001 mg/kg to about 10 mg/kg, about 0.01 mg/kg to about 10 mg/kg, about 0.01 mg/kg to about 1 mg/kg, or about 0.1 mg/kg to about 1 mg/kg can be administered. For oral administration, a representative amount is from about 0.001 mg/kg to about 10 mg/kg, about 0.1 mg/kg to about 10 mg/kg, about 0.01 mg/kg to about 1 mg/kg, or about 0.1 mg/kg to about 1 mg/kg. For administration in suppository form, a representative amount is from about 0.1 mg/kg to about 10 mg/kg, or about 0.1 mg/kg to about 1 mg/kg.

For dose ranges provided herein, also contemplated are individual amounts within each range. For example, for a range of 100 mg to 150 mg, individual amounts of 100 mg, 101 mg, 102 mg and each 1 mg increment to 150 mg are contemplated. Similarly for concentrations, such as 100 mg/mL to 150 mg/mL, individual concentrations within the range such as 100 mg/mL, 101 mg/mL and each 1 mg/mL increment to 150 mg/mL is contemplated.

In some embodiments, the various compositions or combinations are provided for treating ARF and/or ARDS in a subject afflicted with a COVID-19 infection (e.g., by minimizing the symptoms of the cytokine storm resulting from the COVID-19 infection). These compositions or combinations may be delivered via an inhalatory pathway (e.g., using a nebulizer). Effectively treating ARF and/or ARDS resulting from COVID-19 infection can for example reduce the morbidity and mortality of COVID-19.

### Compositions for Use in Treating ARF and/or ARDS

A first aspect includes a composition comprising a tetrahydrocannabinol (THC) or analog thereof, wherein the analog is a derivative, intermediate, metabolite, or fragment thereof, and/or combinations thereof, for use in treating a subject with late-stage acute respiratory failure (ARF) and/or acute respiratory distress syndrome (ARDS).

In some embodiments, the composition or combination (e.g. the THC composition) comprises less than 2% cannabidiol (CBD) or cannabidiolic acid (CBDA). In some embodiments, the composition does not comprise or is essentially free of CBD or CBDA.

In some embodiments, the THC or analog thereof, such as a derivative, intermediate, metabolite, or fragment thereof, is selected from the group consisting of THCA, THC, THCVA, THCV, CBG, 11-OH-Δ9-THC and combinations thereof. In some embodiments, the THC, derivative, intermediate, metabolite, or fragment thereof is Δ9-THC. In further embodiments, the THC is dronabinol.

In another embodiment, the THC, derivative, intermediate, metabolite, or fragment thereof is present in the composition in a concentration of from about 0.5 mg/mL to about 30 mg/mL. In another embodiment, the THC, derivative, intermediate, metabolite, or fragment thereof is present in the composition in a concentration of from about 5 mg/mL to about 10 mg/mL. In another embodiment, the THC or analog thereof is present in the composition in a concentration of about 6.25 mg/mL. In another embodiment, the THC, derivative, intermediate, metabolite, or fragment thereof is present in the composition in a concentration of about 5 mg/mL. In another embodiment, the analog thereof is present in the composition in a concentration of about 2.5 mg/mL.

In another embodiment, the amount of THC, derivative, intermediate, metabolite, or fragment thereof, and/or combinations thereof present in the composition is about 0.5 mg to about 10 mg, optionally about 2.5 mg to about 10 mg. In another embodiment, the amount of THC or analog thereof present in the composition is about 6.25 mg. In another embodiment, the amount of THC or analog thereof present in the composition is about 5 mg. In another embodiment, the amount of THC or analog thereof, present in the composition is about 2.5 mg.

In another embodiment, the composition further comprises a fluvoxamine or analog thereof, wherein the analog is a derivative, intermediate, metabolite, or fragment thereof, and/or combinations thereof. In another embodiment the fluvoxamine, derivative, intermediate, metabolite, or fragment thereof, and/or combinations thereof is present in the composition in a concentration of about 50 mg/mL to about 150 mg/mL, optionally about 75 mg/mL to about 150 mg/mL, optionally about 50 mg/mL, optionally about 100 mg/mL, optionally about 150 mg/mL.

The fluvoxamine or analog thereof, in different embodiments described herein can be fluvoxamine maleate.

In an embodiment, the fluvoxamine or analog thereof, is present in the composition or combination in an amount of fluvoxamine or analog thereof of about 25 mg to about 450 mg, optionally about 30 mg to about 250 mg, optionally about 50 mg to about 200 mg, optionally about 75 mg to about 180 mg, optionally about 50 mg, optionally about 100 mg, optionally about 134 mg, optionally about 148 mg or optionally about 150 mg. In an embodiment, the amount of fluvoxamine or analog thereof, is about 100 mg. In an embodiment, the amount of fluvoxamine or analog thereof, is about 134 mg. In an embodiment, the amount of fluvoxamine or analog thereof, is about 148 mg.

These amounts are for example for oral administration. Doses delivered by other routes can be adjusted in order to achieve blood concentrations similar to the blood concentrations achieved with the oral dosing just described. It can be appreciated that where an amount is calculated based on fluvoxamine, if an analog, such as a salt, derivative, intermediate, metabolite, or fragment of fluvoxamine is used, the amount can be adjusted by a skilled person based on the molecular weight of fluvoxamine and of the analog, salt, derivative, intermediate, metabolite, or fragment thereof as described herein.

As shown in Example 8, administration of THC (at 2.5 mg twice daily) in combination with fluvoxamine (100 mg twice daily) results in improved outcomes as compared to administration of THC alone or administration of THC at a higher daily dose (5 mg twice daily). The group of patients administered the combination had a higher incidence of co-morbidities than groups of patients administered only THC.

In an embodiment, the composition or combination comprises about 2.5 mg of THC or analog thereof and/or about 100 mg of fluvoxamine or analog thereof.

In an embodiment, the composition or combination comprises about 2.5 mg of THC or analog thereof and/or about 134 mg of fluvoxamine or analog thereof.

In an embodiment, the composition or combination comprises about 2.5 mg of THC or analog thereof and/or about 148 mg of fluvoxamine or analog thereof.

In an embodiment, the composition or combination comprises about 5 mg of THC or analog thereof and/or about 100 mg of fluvoxamine or analog thereof.

In an embodiment, the composition or combination comprises about 5 mg of THC or analog thereof and/or about 134 mg of fluvoxamine or analog thereof.

In an embodiment, the composition or combination comprises about.5 mg of THC or analog thereof and/or about 148 mg of fluvoxamine or analog thereof.

In an embodiment, the composition or combination comprises about 6.25 mg of THC or analog thereof and/or about 100 mg of fluvoxamine or analog thereof.

In an embodiment, the composition or combination comprises about 6.25 mg of THC or analog thereof and/or about 134 mg of fluvoxamine or analog thereof.

In an embodiment, the composition or combination comprises about 6.25 mg of THC or analog thereof and/or about 148 mg of fluvoxamine or analog thereof.

In some embodiments, the composition further comprises an interferon (IFN) or analog thereof, wherein, the analog is a derivative, intermediate, metabolite, or fragment thereof, and/or combinations thereof. In some embodiments, the IFN is interferon α (IFN-α), interferon β (IFN-β), and/or interferon-λ (IFN-λ). In some embodiments, the IFN-α is IFN-α2b.

In some embodiments, the IFN-α is present in the composition in a concentration of about 2.5 IU/mL, the IFN-β is present in the composition in a concentration of about 3 IU/mL or the IFN- λ is present in the composition in a concentration of about 3 IU/mL.

In some embodiments, the IFN-α is present in the composition in an amount of about 2.5 IU, the IFN-β is present in the composition in an amount of about 3 IU or the IFN- λ is present in the composition in an amount of about 3 IU.

In another embodiment, the composition further comprises an acetylcysteine or an analog thereof, wherein the analog is a derivative, intermediate, metabolite, or fragment thereof, and/or combinations thereof. In another embodiment, the acetylcysteine, derivative, intermediate, metabolite, or fragment thereof, and/or combinations thereof is present in the composition in an amount of about 10% to about 20% of the total composition. In another embodiment, the acetylcysteine derivative, intermediate, metabolite, or fragment thereof, and/or combinations thereof is present in the composition in an amount of about 10% of the total composition. In another embodiment, the acetylcysteine derivative, intermediate, metabolite, or fragment thereof, and/or combinations thereof is present in the composition in an amount of about 20% of the total composition. In another embodiment, the composition comprises a concentration of acetylcysteine or analogs thereof of about 600 mg/mL.

In another embodiment, the acetylcysteine derivative, intermediate, metabolite, or fragment thereof, and/or combinations thereof is present in the composition in an amount of about 600 mg.

In another embodiment, the composition or combination further comprises one or more buffering agents, one or more preservatives, one or more antioxidants, one or more pharmaceutically acceptable excipients, carriers, diluents, one or more sweetener agents, one or more flavoring agents, or combinations thereof, optionally oil such as sesame oil or mineral oil. In some embodiments, excipients may include oil, such as mineral or sesame oil, FD&C Yellow No. 6, gelatin, glycerin, purified water, sesame oil, titanium dioxide, iron oxide black, shellac glaze, isopropyl alcohol, n-butyl alcohol, propylene glycol, hydroxypropyl methylcellulose ammonium hydroxide FD&C Blue No. 2 and/or FD&C Red No. 21. In some embodiments, a pharmaceutically acceptable carrier includes nanovesicles. For example, one or more active ingredients may be included in a nanovesicle, or in different nanovesicles and the different nanovesicles are provided in a formulation. In some embodiments, the nanovesicle may be a reverse nanovesicle.

In another embodiment, the composition is for use in treating ARF. In another embodiment, the composition is for use in treating ARDS. In another embodiment, the composition is for use in treating a subject experiencing cytokine storm. In another embodiment, the composition is for use in treating ARF, ARDS and/or cytokine storm in a subject afflicted with COVID-19.

In another embodiment, the subject is hospitalized and/or is hypoxic. In another embodiment, the hypoxic subject has an O₂ saturation of less than about 93%, a PaO₂/FiO₂ ratio of less than 300 mm Hg in room air, or more than about a 30% decrease in PaO₂/FiO₂ ratio in the previous 24 hours. In another embodiment, the hypoxic subject has an O₂ saturation of less than about 93%.

### Compositions

Another aspect includes a composition comprising a tetrahydrocannabinol (THC) or analog thereof, wherein the analog is a derivative, intermediate, metabolite, or fragment thereof, and/or combinations thereof and a fluvoxamine or analog thereof, wherein the analog is a derivative, intermediate, metabolite, or fragment thereof, and/or combinations thereof. In some embodiments, the THC, derivative, intermediate, metabolite, or fragment thereof, is selected from the group consisting of THCA, THC, THCVA, THCV, and combinations thereof. In some embodiments, the THC, derivative, intermediate, metabolite, or fragment thereof is Δ9-THC. In further embodiments, the THC is dronabinol.

In some embodiments, the composition or combination (e.g. the THC composition) comprises less than 2% CBD or CBDA. In some embodiments, the composition does not comprise or is essentially free of CBD or CBDA.

In another embodiment, the THC, derivative, intermediate, metabolite, or fragment thereof is present in the composition in a concentration of from about 0.5 mg/mL to about 30 mg/mL. In another embodiment, the THC, derivative, intermediate, metabolite, or fragment thereof is present in the composition in a concentration of from about 5 mg/mL to about 10 mg/mL. In another embodiment, the THC, derivative, intermediate, metabolite, or fragment thereof is present in the composition in a concentration of about 5 mg/mL.

In another embodiment, the amount of THC, derivative, intermediate, metabolite, or fragment thereof, and/or combinations thereof present in the composition is about 0.5 mg to about 30 mg, about 0.5 mg to about 20 mg, about 0.5 mg to about 10 mg, optionally about 2.5 mg to about 30 mg or about 2.5 mg to about 10 mg. In another embodiment, the amount of THC, derivative, intermediate, metabolite, or fragment thereof, and/or combinations thereof present in the composition is about 10 mg. In another embodiment, the amount of THC, derivative, intermediate, metabolite, or fragment thereof, and/or combinations thereof present in the composition is about 5 mg. In another embodiment, the amount of THC, derivative, intermediate, metabolite, or fragment thereof, and/or combinations thereof present in the composition is about 2.5 mg.

In another embodiment, the composition further comprises a fluvoxamine or analog thereof, wherein the analog is a derivative, intermediate, metabolite, or fragment thereof, and/or combinations thereof. In another embodiment the fluvoxamine, derivative, intermediate, metabolite, or fragment thereof, and/or combinations thereof is present in the composition in a concentration of about 50 mg/mL to about 150 mg/mL, optionally about 75 mg/mL to about 150 mg/mL, optionally about 50 mg/mL, optionally about 100 mg/mL, optionally about 150 mg/mL.

In an embodiment, the fluvoxamine, derivative, intermediate, metabolite, or fragment thereof, and/or combinations thereof is present in the composition in an amount of fluvoxamine of about 50 mg to about 150 mg, optionally about 75 mg to about 150 mg, optionally 50 mg, optionally 100 mg, optionally about 134 mg, optionally about 148 mg or optionally 150 mg. In another embodiment, the fluvoxamine, derivative, intermediate, metabolite, or fragment thereof, and/or combinations thereof is present in the composition in an amount about 25 mg to about 450 mg, optionally about 30 mg to about 250 mg, optionally about 50 mg to about 200 mg, optionally about 75 mg to about 180 mg, optionally about 50 mg, optionally about 100 mg, optionally about 150 mg, optionally about 175 mg, or optionally about 180 mg.

In an embodiment, the fluvoxamine or analog thereof is present in the composition in an amount of fluvoxamine of about 50 mg.

In an embodiment, the fluvoxamine or analog thereof is present in the composition in an amount of fluvoxamine of about 100 mg.

In an embodiment, the fluvoxamine or analog thereof is present in the composition in an amount of fluvoxamine of about 134 mg.

In an embodiment, the fluvoxamine or analog thereof is present in the composition in an amount of fluvoxamine of about 148 mg.

In an embodiment, the fluvoxamine or analog thereof is present in the composition in an amount of fluvoxamine of about 150 mg.

In some embodiments, the composition further comprises an interferon (IFN) or analog thereof, wherein, the analog is a derivative, intermediate, metabolite, or fragment thereof, and/or combinations thereof. In some embodiments, the IFN is interferon α (IFN-α), interferon β (IFN-β), and/or interferon-λ (IFN-λ). In some embodiments, the IFN-α is IFN-α2b.

In some embodiments, the IFN-α is present in the composition in a concentration of about 2.5 IU/mL, the IFN-β is present in the composition in a concentration of about 3 IU/mL or the IFN- λ is present in the composition in a concentration of about 3 IU/mL.

In some embodiments, the IFN-α is present in the composition in an amount of about 2.5 IU, the IFN-β is present in the composition in an amount of about 3 IU or the IFN- λ is present in the composition in an amount of about 3 IU.

In another embodiment, the composition further comprises an acetylcysteine or an analog thereof, wherein the analog is a derivative, intermediate, metabolite, or fragment thereof, and/or combinations thereof. In another embodiment, the acetylcysteine, derivative, intermediate, metabolite, or fragment thereof, and/or combinations thereof is present in the composition in an amount of about 10% to about 20% of the total composition. In another embodiment, the acetylcysteine derivative, intermediate, metabolite, or fragment thereof, and/or combinations thereof is present in the composition in an amount of about 10% of the total composition. In another embodiment, the acetylcysteine derivative, intermediate, metabolite, or fragment thereof, and/or combinations thereof is present in the composition in an amount of about 20% of the total composition.

Percentage amounts can for example be weight/weight, vol/vol or weight/vol. For example, where the composition is a liquid and the component being described is also a liquid, the percentage can for example be vol/vol.

In another embodiment, the composition comprises a concentration of acetylcysteine or analogs thereof of about 600 mg/mL.

In another embodiment, the acetylcysteine derivative, intermediate, metabolite, or fragment thereof, and/or combinations thereof is present in the composition in an amount of about 600 mg.

In another embodiment, the composition or combination further comprises one or more buffering agents, one or more preservatives, one or more antioxidants, one or more pharmaceutically acceptable excipients, carriers, diluents, one or more sweetener agents, one or more flavoring agents, or combinations thereof, optionally oil such as mineral oil or sesame oil. In some embodiments, excipients may include oil, such as mineral or sesame oil, FD&C Yellow No. 6, gelatin, glycerin, purified water, sesame oil, titanium dioxide, iron oxide black, shellac glaze, isopropyl alcohol, n-butyl alcohol, propylene glycol, hydroxypropyl methyl cellulose, ammonium hydroxide, FD&C Blue No. 2 and/or FD&C Red No. 21. In some embodiments, a pharmaceutically acceptable carrier includes nanovesicles. For example, one or more active ingredients can be included in a nanovesicle, or in different nanovesicles and the different nanovesicles are provided in a formulation. In some embodiments, the nanovesicle may be a reverse nanovesicle.

In an embodiment, the composition comprising the THC and the composition comprising the fluvoxamine are formulated for inhalation or oral, intravenous, intranasal, or suppository administration.

### Combination Treatments

Another aspect includes a combination treatment comprising: a composition comprising a tetrahydrocannabinol (THC) or analog thereof, wherein the analog is a derivative, intermediate, metabolite, or fragment thereof, and/or combinations thereof; and a composition comprising a fluvoxamine or analog thereof, wherein the analog is a derivative, intermediate, metabolite, or fragment thereof, and/or combinations thereof.

In an embodiment the THC, derivative, intermediate, metabolite, or fragment thereof is selected from the group consisting of THCA, THC, THCVA, THCV, and combinations thereof. In an embodiment the THC, derivative, intermediate, metabolite, or fragment thereof is Δ9-THC. In an embodiment, the THC is dronabinol.

In some embodiments, the composition comprising a tetrahydrocannabinol (THC) or analog thereof, and/or combinations thereof comprises less than 2% CBD or CBDA. In some embodiments, the composition does not comprise or is essentially free of CBD or CBDA.

In some embodiments, the composition comprising a fluvoxamine or analog thereof, wherein the analog is a derivative, intermediate, metabolite, or fragment thereof, and/or combinations thereof comprises less than 2% CBD or CBDA. In some embodiments, the composition (e.g. the THC composition) does not comprise or is essentially free of CBD or CBDA.

In an embodiment, the composition comprising the THC, derivative, intermediate, metabolite, or fragment thereof, and/or combinations comprises about 0.5 mg to about 10 mg, optionally about 2.5 mg to about 10 mg of THC. In an embodiment, the composition comprising the THC, derivative, intermediate, metabolite, or fragment thereof, and/or combinations comprises about 6.25 mg of THC. In an embodiment, the composition comprising the THC, derivative, intermediate, metabolite, or fragment thereof, and/or combinations comprises about 5 mg of THC. In an embodiment, the composition comprising the THC or analog thereof comprises about 2.5 mg of THC.

In an embodiment, the composition comprising the fluvoxamine, derivative, intermediate, metabolite, or fragment thereof, and/or combinations comprises about 50 mg to about 150 mg, optionally about 50 mg, optionally about 100 mg, optionally about 134 mg, optionally about 148 mg or optionally about 150 mg of fluvoxamine. In an embodiment, the composition comprising the fluvoxamine, derivative, intermediate, metabolite, or fragment thereof, and/or combinations comprises an amount of fluvoxamine of about 25 mg to about 450 mg, optionally about 30 mg to about 250 mg, optionally about 50 mg to about 200 mg, optionally about 75 mg to about 180 mg, optionally about 50 mg, optionally about 100 mg, optionally about 134 mg, optionally about 148 mg or optionally about 150 mg.

In an embodiment, the amount of THC or analog thereof, optionally dronabinol, in the composition is 6.25 mg. In an embodiment, the amount of fluvoxamine or analog thereof optionally fluvoxamine maleate, is about 134 mg.

In an embodiment, the composition comprising the THC, derivative, intermediate, metabolite, or fragment thereof, and/or combinations comprises THC in a concentration of from about 0.5 mg/mL to about 30 mg/mL. In an embodiment, the composition comprising the THC, derivative, intermediate, metabolite, or fragment thereof, and/or combinations comprises THC in a concentration of from about 5 mg/mL to about 10 mg/mL. In an embodiment, the THC, derivative, intermediate, metabolite, or fragment thereof is present in the composition comprising the THC in a concentration of about 5 mg/mL.

Other concentrations between 0.5 mg/mL to 30 mg/mL may also be used, for example 2.5 mg/mL or 6.25 mg/mL.

In an embodiment, the composition comprising the fluvoxamine, derivative, intermediate, metabolite, or fragment thereof, and/or combinations comprises fluvoxamine in a concentration of about 50 mg/mL to about 150 mg/mL, optionally about 75 mg/mL to about 150 mg/mL, optionally about 50 mg/mL, optionally about 100 mg/mL, optionally about 150 mg/mL.

Other concentrations between 50 mg/mL to 150 mg/mL may also be used, for example 134 mg/mL or 148 mg/mL.

In an embodiment, one or both of the composition comprising the THC and the composition comprising the fluvoxamine are formulated for inhalation or oral, intravenous, intranasal, or suppository administration.

In an embodiment, one or both of the composition or combination comprising the THC and the composition or combination comprising fluvoxamine further comprise one or more buffering agents, one or more preservatives, one or more antioxidants, one or more pharmaceutically acceptable excipients, carriers, diluents, one or more sweetener agents, one or more flavoring agents, or combinations thereof, optionally an oil such as sesame or mineral oil. In some embodiments, excipients may include oil, such as mineral or sesame oil, FD&C Yellow No. 6, gelatin, glycerin, purified water, sesame oil, titanium dioxide, iron oxide black, shellac glaze, isopropyl alcohol, n-butyl alcohol, propylene glycol, hydroxypropyl methyl cellulose, ammonium hydroxide, FD&C Blue No. 2 and/or FD&C Red No. 21. In some embodiments, a pharmaceutically acceptable carrier includes nanovesicles. For example, one or more active ingredients may include in a nanovesicle, or in different nanovesicles and the different nanovesicles are provided in a formulation. In some embodiments, the nanovesicle may be reverse nanovesicle.

### Methods of Treating ARF and/or ARDS

Another aspect includes a method of treating ARF and/or ARDS in a subject in need thereof, the method comprising administering to the subject a composition or combination as described herein for example a composition comprising a THC and/or analog and a fluvoxamine and/or analog described herein or a combination treatment described herein.

In some embodiments, the composition or combination (e.g. the THC composition) comprises less than 2% CBD or CBDA. In some embodiments, the composition does not comprise or is essentially free of CBD or CBDA.

In an embodiment, a composition as described herein, for example a composition comprising composition comprising a THC and/or analog and a fluvoxamine and/or analog described herein or a combination treatment described herein, is administered twice per day (B.I.D) in an amount sufficient to deliver a dose of about 2.5 mg to about 10 mg of the THC or analog thereof.

In an embodiment, a composition as described herein, for example a composition comprising a THC and/or analog and a fluvoxamine and/or analog described herein or a combination treatment described herein, is administered twice per day (B.I.D) in an amount sufficient to deliver a dose of about 5 mg of the THC or analog thereof.

In an embodiment, a composition as described herein, for example a composition comprising a THC and/or analog and a fluvoxamine and/or analog described herein or a combination treatment described herein, is administered twice per day (B.I.D) in an amount sufficient to deliver a dose of about 2.5 mg of the THC or analog thereof.

In an embodiment, a composition as described herein, for example a composition comprising a THC and/or analog and a fluvoxamine and/or analog described herein or a combination treatment described herein, is administered three times per day (T.I.D) in an amount sufficient to deliver a dose of about 1mg to about 4 mg of the THC or analog thereof.

In an embodiment, a composition as described herein, for example a composition comprising a THC and/or analog and a fluvoxamine and/or analog described herein or a combination treatment described herein, is administered three times per day (T.I.D) in an amount sufficient to deliver a dose of about 3.33 mg of the THC or analog thereof.

In an embodiment, a composition as described herein, for example a composition comprising a THC and/or analog and a fluvoxamine and/or analog described herein or a combination treatment described herein, is administered three times per day (T.I.D) in an amount sufficient to deliver a dose of about 1.67 mg of the THC or analog thereof.

In an embodiment, a composition as described herein, for example a composition comprising a THC and/or analog and a fluvoxamine and/or analog described herein or a combination treatment described herein, is administered twice per day (B.I.D) for example in an amount sufficient to deliver a dose of about 75 mg to about 150 mg, optionally about 100 mg, about 134 mg, about 148 mg or 150 mg, of the fluvoxamine, or analog thereof. The daily dose can for example be of about 150 mg to about 300 mg, optionally about 200 mg, about 268 mg, about 296 mg or 300 mg, of the fluvoxamine, or analog thereof. In an embodiment, a composition as described herein, for example a composition comprising a THC and/or analog thereof (not covered by the claims) or a composition comprising a THC and/or analog and a fluvoxamine and/or analog described herein or a combination treatment described herein, is administered twice per day (B.I.D) in an amount sufficient to deliver an individual dose or a daily dose of fluvoxamine of about 25 mg to about 900 mg, optionally about 30 mg to about 300 mg, optionally about 50 mg to about 200 mg, optionally about 75 mg to about 180 mg, optionally about 50 mg, optionally about 100 mg, optionally about 134 mg, optionally 148 mg, optionally about 150 mg of the fluvoxamine, derivative, intermediate, metabolite, or fragment thereof, and combinations thereof.

In an embodiment, a composition as described herein, for example a composition comprising a THC and/or analog and a fluvoxamine and/or analog described herein or a combination treatment described herein, is administered three times per day (T.I.D). In some embodiments, the composition or combination administered is in an amount sufficient to deliver a dose of about 50 mg to about 900 mg of the fluvoxamine or analog thereof or a unit dose of about 50 mg to about 450 mg of the fluvoxamine or analog thereof. For example, the unit dose may comprise about 100 mg, about 134 mg, about 148 mg or about 150 mg of fluvoxamine or analog.

As mentioned, the fluvoxamine or analog thereof in the compositions, combinations, methods and uses may be fluvoxamine maleate.

In an embodiment, a composition as described herein, for example a composition comprising a THC and/or analog and a fluvoxamine and/or analog described herein or a combination treatment described herein, is administered three times per day (T.I.D).

For example, the composition or combination administered is in an amount sufficient to deliver a dose of about 100 mg, or about 100 mg to about 250 mg, of the fluvoxamine or analogs thereof. In an embodiment, the composition or combination administered is in an amount sufficient to deliver a dose of about 134 mg of the fluvoxamine or analogs thereof. In an embodiment, the composition or combination administered is in an amount sufficient to deliver a dose of about 148 mg of the fluvoxamine or analogs thereof. In an embodiment, the composition or combination administered is in an amount sufficient to deliver a dose of about 150 mg of the fluvoxamine or analogs thereof.

For example, the composition or combination administered is in an amount sufficient to deliver a dose of about 50 mg of the fluvoxamine or analogs thereof or a dose of about 130 mg to about 200 mg of the fluvoxamine or analogs thereof, or 148 mg.

In an embodiment, the method is for treating ARF. In an embodiment, the method is for treating ARDS. In an embodiment, the method is for treating a subject experiencing cytokine storm. In an embodiment, the method is for treating ARF, ARDS and/or cytokine storm in a subject afflicted with COVID-19.

In an embodiment, the subject is hospitalized and/or is hypoxic. In an embodiment, the hypoxic subject has an O₂ saturation of less than about 93%, a PaO₂/FiO₂ ratio of less than 300 mm Hg in room air, or more than about a 30% decrease in PaO₂/FiO₂ ratio in the previous 24 hours. In an embodiment, the hypoxic subject has an O₂ saturation of less than about 93%.

In an embodiment, compositions as described herein, for example a composition comprising a THC and/or analog and a fluvoxamine and/or analog described herein or a combination treatment described herein, are administered orally, via inhalation, intravenously, intranasally, or via suppository.

In an embodiment, the subject is a human.

In an embodiment, the method further comprises administering a bronchodilator to the subject about 10 minutes to about 15 minutes before administering to the subject a composition as described herein, for example a composition comprising a THC and/or analog and a fluvoxamine and/or analog described herein or a combination treatment described herein.

In an embodiment, the method comprises administering the combination therapy described herein simultaneously, separately or sequentially.

Another aspect includes use of compositions as described herein, for example a composition comprising a THC and/or analog and a fluvoxamine and/or analog described herein or a combination treatment described herein, for treating ARF and/or ARDS in a subject.

In some embodiments, the composition or combination (e.g. the THC composition) comprises less than 2% CBD or CBDA. In some embodiments, the composition does not comprise or is essentially free of CBD or CBDA.

Another aspect includes use of compositions as described herein, for example a composition comprising a THC and/or analog thereof (not covered by the claims) or a composition comprising a THC and/or analog and a fluvoxamine and/or analog described herein or a combination treatment described herein, in the manufacture of a medicament for treating ARF and/or ARDS in a subject.

In some embodiments, the composition or combination (e.g. the THC composition) comprises less than 2% CBD or CBDA. In some embodiments, the composition does not comprise or is essentially free of CBD or CBDA.

In an embodiment, the subject is a human.

In an embodiment, the use is for treating ARF. In an embodiment, the use is for treating ARDS. In an embodiment, the use is for treating a subject experiencing cytokine storm. In an embodiment, the use is for treating ARF, ARDS and/or cytokine storm in a subject afflicted with COVID-19.

In an embodiment, the subject is hospitalized and/or is hypoxic. In some embodiments, the subject is suffering from pulmonary edema, diffuse bilateral opacities in radiographic findings. In an embodiment, the hypoxic subject has an O₂ saturation of less than about 93%, less than about 92%, less than about 91%, or less than about 90% a PaO₂/FiO₂ ratio of less than 300 mm Hg in room air, or more than about a 30%, more than about 40%, more than about 50% decrease, more than about 60% decrease, more than about 70% decrease, more than about 80% decrease, or more than about 90% decrease in PaO₂/FiO₂ ratio in about the previous 24 hours. In an embodiment, the hypoxic subject has an O₂ saturation of less than about 93%.

In some embodiments, compositions or combinations as described herein, for example a composition or combination comprising a THC and/or analog thereof or a composition or combination comprising a THC and/or analog and a fluvoxamine and/or analog described herein, are comprised in tablet form, in a capsule, in a suspension, or a powder.

### Packages

Another aspect comprises a package comprising THC and/or an analog thereof and a fluvoxamine and/ or an analog thereof, and/or an acetylcysteine and/or an analog thereof, and/or an IFN and/or analog thereof. In an embodiment, the package further comprises a bronchodilator, and/or a sterile vial. In another embodiment, the package comprises any compositions or combinations as described herein, for example a composition or combination comprising a THC and/or analog thereof or a composition or combination comprising a THC and/or analog and a fluvoxamine and/or analog described herein, optionally in a sterile vial.

In some embodiments, the composition or combination (e.g. the THC composition) comprises less than 2% CBD or CBDA. In some embodiments, the composition does not comprise or is essentially free of CBD or CBDA.

In some embodiments, compositions or combinations as described herein, for example a composition or combination comprising a THC and/or analog thereof, or a composition or combination comprising a THC and/or analog and a fluvoxamine and/or analog described herein, or a combination treatment described herein, are co-administered with standard of care treatments including glucocorticoid, hydroxychloroquine, azithromycin, Naprosyn, cetirizine, thalidomide, heparin, antivirals, pressor support, oxygen supplementation, ventilation, prone ventilation and extracorporeal membrane oxygenation.

In some embodiments, the THC is Δ9-THC. For example, the FDA approved dronabinol formulation can be used, optionally wherein the carriers and/or excipients can be adjusted. For example, excipients such as glycerin, iron oxide yellow, gelatin and titanium dioxide can be removed from the dronabinol formulation.

In some embodiments, fluvoxamine can be a pharmaceutically acceptable salt thereof. For example, fluvoxamine can be fluvoxamine maleate.

In some embodiments, fluvoxamine and THC can be dissolved or suspended in a suitable carrier such as sesame seed oil. It can be appreciated that because Δ9-THC is hydrophobic, a hydrophobic carrier such as sesame seed oil may be suitable. Fluvoxamine in contrast is more hydrophilic, but can still be suspended in carriers such as sesame seed oil. For example, fluvoxamine can be dissolved or suspended in sesame seed oil in more than about 5 nm or about 5 nm deflocculated particles. Fluvoxamine particles can be obtained by any suitable means known in the art. For example, it can be milled to about 0.5 microns. The fluvoxamine particles can be suspended in sesame seed oil to create a coarse suspension.

As sesame seed oil is currently used in a commercially available Δ9-THC formulation, it is not expected to adversely affect the pharmacokinetic properties of Δ9-THC. It can be appreciated that this formulation would not negatively affect the absorption of fluvoxamine because there are no contraindications to taking fluvoxamine, including fluvoxamine maleate, with food such as sesame seed oil. Further, it is believed that if lipids or oils could interfere with the bioavailability of fluvoxamine, this would have been demonstrated in the original bioavailability studies conducted in humans and resulted in a recommendation to restrict administration of the medication with food.

THC and fluvoxamine can be formulated in a HPMC capsule. For example, each capsule can comprise about 5.25 mg or about 6.25 mg of THC such as Δ9-THC and about 134 mg or about 148 mg of fluvoxamine (e.g., fluvoxamine maleate). It is contemplated that other dosages and unit doses can be used as described herein. It is contemplated that other dosages and unit doses can be used as described herein such as a capsule containing 6.0 mg of THC and 120 mg of fluvoxamine maleate, a capsule containing 6.0 mg of THC and 180 mg of fluvoxamine maleate, or a capsule containing 12.5 mg THC and 180 mg of fluvoxamine maleate. Additionally, a capsule containing 6.25 mg of THC and 134 mg of fluvoxamine maleate or a capsule containing 12.5 mg of THC and 166 mg of fluvoxamine maleate can be used.

Based on the available storage and handling information of fluvoxamine and Δ9-THC, a capsule comprising the composition of Δ9-THC and fluvoxamine as described herein can, for example, be stored at a temperature of about 8°C and 15°C, or alternatively be stored in a refrigerator, while protecting from freezing.

### THC Compositions

In another aspect, the present disclosure includes a composition comprising a tetrahydrocannabinol (THC) or analog thereof and fluvoxamine or analog thereof, wherein the analog is a derivative, intermediate, metabolite, salt, solvate or fragment thereof. The composition which can be referred to as the THC composition, can comprise any of the compositions comprising THC or an analog thereof described herein.

In some embodiments, the THC, derivative, intermediate, metabolite, salt, solvate or fragment thereof, is selected from the group consisting of THCA, THC, THCVA, THCV, and combinations thereof. For example, the THC, derivative, intermediate, metabolite, salt or solvate or fragment thereof is Δ9-THC. For example, the THC is dronabinol.

In some embodiments, the composition or combination (e.g. the THC composition) comprises less than 2% CBD or CBDA. In some embodiments, the composition does not comprise or is essentially free of CBD or CBDA.

In some embodiments, the derivative, intermediate, metabolite, salt, solvate or fragment of fluvoxamine is a salt of fluvoxamine. For example, the salt of fluvoxamine is fluvoxamine maleate.

In some embodiments, the ratio of the THC to fluvoxamine (e.g., fluvoxamine maleate) is about 0.01:1 to about 0.075:1 based on weight. In some embodiments, the ratio of the THC to fluvoxamine maleate is about 0.015:1 to about 0.05:1, about 0.025:1 to about 0.05:1, about 0.03;1 to about 0.04:1, about 0.069:1 to about 0.072:1, or about 0.035:1 based on weight.

In some embodiments, the composition or combination described herein further comprises one or more pharmaceutically acceptable diluents, carriers and/or excipients. For example, the one or more pharmaceutically acceptable diluents, carriers and/or excipients are each independently selected from sesame oil, silicon dioxide, sodium chloride and combinations thereof.

In some embodiments, the composition described herein further comprises sesame oil and silicone dioxide. In some embodiments, the composition further comprises sodium chloride.

In some embodiments, the composition comprises
about 55 wt% to about 70 wt% of sesame oil,
about 1 wt% to about 3 wt% of silicon dioxide,
about 25 wt% to about 40 wt% of fluvoxamine or analog thereof, and
about 0.85% to about 3 wt% of THC or analog thereof.

In some embodiments, the composition comprises
about 58 wt% to about 67 wt% of sesame oil,
about 2 wt% to about 3 wt% of silicon dioxide,
about 29 wt% to about 37 wt% of fluvoxamine or analog thereof, and
about 1 wt% to about 2.5 wt% of THC or analog thereof.

In some embodiments, the composition comprises
about 60 wt% to about 66 wt% of sesame oil,
about 2 wt% to about 2.5 wt% of silicon dioxide,
about 31 wt% to about 35 wt% of fluvoxamine or analog thereof, and
about 1 wt% to about 2.5 wt% of THC or analog thereof.

In some embodiments, the composition comprises
about 64 wt% of sesame oil,
about 2.2 wt% of silicon dioxide,
about 32.6 wt% of fluvoxamine or analog thereof, and
about 1.2 wt% of THC or analog thereof.

In some embodiments, the composition comprises
about 62.7 wt% to about 64.2 wt% of sesame oil,
about 2.2 wt% of silicon dioxide,
about 32.6 wt% of fluvoxamine or analog thereof, and
about 1 wt% to about 2.5 wt% of THC or analog thereof.

In some embodiments, the composition comprises
about 62.7 wt% of sesame oil,
about 2.2 wt% of silicon dioxide,
about 32.6 wt% of fluvoxamine or analog thereof, and
about 2.5 wt% of THC or analog thereof.

In some embodiments, the fluvoxamine or analog thereof is fluvoxamine maleate, and/or the THC or analog thereof is Δ9-THC.

In some embodiments, the composition is in the form of a capsule, optionally the capsule is a soft-gel capsule, optionally a hydroxyporpylmethylcellulose (HPMC) capsule.

### Compositions for use in treatment of ARF and/or ARDS including late-stage ARF and/or late-stage ARDS.

In another aspect, the present disclosure includes the composition or combination as described herein for use in the treatment of acute respiratory failure (ARF) and/or acute respiratory distress syndrome (ARDS), optionally late-stage ARF and/or late-stage ARDS, optionally caused by cytokine storm.

In some embodiments, the composition or combination (e.g. the THC composition) comprises less than 2% CBD or CBDA. In some embodiments, the composition does not comprise or is essentially free of CBD or CBDA.

In some embodiments, the composition described herein, is for use in the treatment of ARF. In some embodiments, the composition described herein, is for use in the treatment of ARDS. In some embodiments, the composition described herein, is for use in the treatment of a cytokine storm. For example, the composition described herein, is for use in the treatment of a subject afflicted with COVID-19.

For example, treatment of a subject afflicted with COVID-19 refers to the treating of the COVID-19 viral infection and/or one or more of the associated symptoms and/or syndromes that can be associated with COVID-19 infection.

In some embodiments, the composition is for use in a subject that is hospitalized and/or hypoxic. For example, the subject has an O₂ saturation of less than about 93%, a PaO₂/FiO₂ ratio of less than 300 mm Hg in room air, or more than about a 30% decrease in PaO₂/FiO₂ ratio in the previous 24 hours. For example, the subject can have an O₂ saturation of less than about 93%.

In some embodiments, the composition is for use twice daily, optionally to achieve a daily dose of about 5 mg to about 25 mg, about 5 mg to about 23 mg, or about 10 mg to about 22 mg of THC, and about 25 mg to 900 mg, about 75 mg to about 600 mg, about 100 mg to about 300 mg, about 100 mg to about 268 mg, about 200 mg to about 268 mg, about 100 mg to about 230 mg, about 210 mg to about 220 mg, or about 213 mg to about 218 mg of fluvoxamine.

It can be appreciated that the actual amount of fluvoxamine or analog thereof used as described herein, e.g. in a composition as described in the present disclosure, and/or administered as described in a method of the present disclosure, depends on the nature of the fluvoxamine or analog thereof. It is understood that the molecular weight of the fluvoxamine analog varies depending on the chemical structure of the fluvoxamine analog, which may affect the actual amount (i.e. weight) of the fluvoxamine analog used as described herein. For example, fluvoxamine has a molecular weight of about 318.335 g/mol, whereas fluvoxamine maleate has a molecular weight of 434.4 g/mol. Accordingly, for example, if an exemplary composition comprises about 100 mg of fluvoxamine, an equivalent composition would comprise about 136.46 mg of fluvoxamine maleate in the case where fluvoxamine is used in its maleate form. For example, if a subject is to be administered about 50 mg of fluvoxamine, in the case where fluvoxamine maleate is used, the subject would be administered about 68 mg of fluvoxamine maleate. A similar calculation can be made by a skilled person for other analogs of fluvoxamine.

It can be appreciated that the actual amount of THC or analog thereof used as described herein, *e.g.* in a composition as described in the present disclosure, and/or administered as described in a method of the present disclosure, depends on the nature of the THC or analog thereof. It is understood that the molecular weight of the THC analog varies depending on the chemical structure of the THC analog, which may affect the actual amount (i.e. weight) of the THC analog used as described herein. For example, Δ9-THC has a molecular weight of 314.5 g/mol, whereas THCA has a molecular weight of 358.478 g/mol. Accordingly, for example, if an exemplary composition comprises about 10 mg of Δ9-THC as the THC component, an analogous composition could comprise about 11.4 mg of THCA in the case where THCA is used as the THC component. For example, if a subject is to be administered 15 mg of Δ9-THC, in the case where THCA is used, the subject could be administered about 17.1 mg of THCA. A similar calculation can be made by a skilled person for other analogs of THC.

In some embodiments, the composition is for oral use.

In another aspect, the present disclosure includes a method of treating late-stage stage acute respiratory failure (ARF) and/or acute respiratory distress syndrome (ARDS) caused by cytokine storm comprising administering THC or analog thereof, and fluvoxamine or analog thereof in a subject in need thereof, wherein the analog is derivative, intermediate, metabolite, or fragment thereof, salt, solvate and/or combinations thereof.

In some embodiments, the method is for treating ARF. In some embodiments, the method is for treating ARDS. In some embodiments, the method is for treating cytokine storm. For example, in some embodiments the method is for treating a subject afflicted with COVID-19.

In some embodiments, the subject is hospitalized and/or hypoxic. For example, the subject can have an O₂ saturation of less than about 93%, a PaO₂/FiO₂ ratio of less than 300 mm Hg in room air, or more than about a 30% decrease in PaO₂/FiO₂ ratio in the previous 24 hours. For example, the subject can have an O₂ saturation of less than about 93%.

In some embodiments, the administering of the THC or analog thereof and fluvoxamine or analog thereof comprises administering a composition described herein.

In some embodiments, the THC or analog thereof and fluvoxamine or analog thereof is administered twice daily, optionally to achieve a daily dose of about 5 mg to about 25 mg, about 5 mg to about 23 mg, or about 10 mg to about 22 mg of THC, and about 25 mg to 900 mg, about 75 mg to about 600 mg, about 100 mg to about 300 mg, about 100 mg to about 268 mg, about 200 mg to about 268 mg, about 100 mg to about 230 mg, about 200 mg to about 230 mg, about 210 mg to about 220 mg, or about 213 mg to about 218 mg of fluvoxamine.

In some embodiments, the THC or analog thereof and fluvoxamine or analog thereof is administered orally.

### Compositions for the Manufacture of a Medicament for Treatment of late-stage ARF and/or ARDS

In another aspect, the present disclosure includes a composition or combination comprising THC or analog thereof and fluvoxamine or analog thereof for use in the manufacture of a medicament for the treatment of late-stage stage acute respiratory failure (ARF) and/or acute respiratory distress syndrome (ARDS) caused by cytokine storm, wherein the analog is a derivative, intermediate, metabolite, or fragment thereof, salt, solvate and/or combinations thereof.

In some embodiments, the composition or combination (e.g. the THC composition) comprises less than 2% CBD. In some embodiments, the composition does not comprise or is essentially free of CBD or CBDA.

In some embodiments, the medicament is for treating ARF. In some embodiments, the medicament is for treating ARDS. In some embodiments, the medicament is for treating a cytokine storm. For example, in some embodiments the medicament is for treating a subject afflicted with COVID-19.

In some embodiments, the medicament is for use in a subject that is hospitalised and/or hypoxic. For example, the subject can have an O₂ saturation of less than about 93%, a PaO₂/FiO₂ ratio of less than 300 mm Hg in room air, or more than about a 30% decrease in PaO₂/FiO₂ ratio in the previous 24 hours. For example, the subject can have an O₂ saturation of less than about 93%.

In some embodiments, the composition or combination is for use in the preparation of a medicament is a composition as described herein.

In some embodiments, the medicament is for use twice daily, optionally to achieve a daily dose of about 5mg to about 25 mg, about 9 mg to about 23 mg, or about 10 mg to about 22 mg of THC, and about 25 mg to 900 mg, about 75 mg to about 600 mg, about 100 mg to about 300 mg, about 100 mg to about 268 mg, about 200 mg to about 268 mg, about 100 mg to about 230 mg, about 200 mg to about 230 mg, about 210 mg to about 220 mg, or about 213 mg to about 218 mg of fluvoxamine.

In some embodiments, the medicament is for oral use.

In some embodiments, the subject is an organ transplant recipient. In some embodiments, the subject is a lung transplant recipient. In other embodiments, the subject is a kidney, heart, corneal, pancreas, intestine, middle ear, skin, bone, bone marrow, heart valve, and/or connective tissue transplant recipient.

### Exemplary Composition or Combinations for Treating ARF and/or ARDS in a Subject Afflicted with a COVID-19 Infection

In some embodiments, a composition or combination for treating ARF and/or ARDS resulting from COVID-19 infection includes a THC, derivative, intermediate, metabolite, or fragment thereof, and combinations thereof and includes Fluvoxamine or a derivative, intermediate, metabolite, or fragment thereof, and combinations thereof. The composition or combination can also include an interferon (IFN), derivative, intermediate, metabolite, or fragment thereof, and combinations thereof. In some embodiments, the composition or combination includes an acetylcysteine or a derivative, intermediate, metabolite, or fragment thereof, and combinations thereof. The composition or combination may be delivered via an inhalatory pathway (e.g., using a nebulizer) to a patient afflicted with COVID-19 and suffering from ARF and/or ARDS. In other embodiments, the composition or combination may also be delivered intravenously, orally (*e.g*., as an oral solution), and/or via a suppository.

In some embodiments, the composition or combination (e.g. the THC composition) comprises less than 2% CBD or CBDA. In some embodiments, the composition does not comprise or is essentially free of CBD or CBDA.

### 1. Tetrahydrocannabinol

In some embodiments, the THC, derivative, or intermediate (including prodrugs) include THC: Δ9-tetrahydrocannabinol-C5 (Δ9-THC-C 5), Δ9-tetrahydrocannabinol-C4 (Δ9-THC-C 4), Δ9-tetrahydrocannabivarin (Δ9-THCV-C 3), Δ9-Tetrahydrocannabiorcol (Δ9-THCO C-1), Δ9-Tetrahydrocannabinolsäure (Δ9 THCA-C-5 A), Δ9-Tetrahydrocannabinolsäure B (Δ9 THCA-C-5 B), Δ9-Tetrahydrocannabinolsäure-C4 (Δ9 THCA-C-4 A and/or B), Δ9-Tetrahydrocannabivarinsäure A (Δ9-THCVA-C 3 A), Δ9-Tetrahydrocannabiorcolsäure (Δ9-THCOA-C 1 A and/or B), (-)-Δ8-trans-(6aR,10aR)-Δ8-tetrahydrocannabinol (Δ8-THC-C 5), (-)-Δ8-trans-(6aR, 10aR)-Tetrahydrocannabinolsäure A (Δ8-THCA-C 5 A); (-)-(6a S,10a R)-Δ9-tetrahydrocannabinol ((-)-cis-Δ9-THC-C 5). In some embodiments, the THC, derivative, or intermediate includes tetrahydrocannabinolic acid (THCA), tetrahydrocannabivarin carboxylic acid (THCVA), tetrahydrocannabivarin (THCV), and combinations thereof. In some embodiments, the THC, derivative, or intermediate is Δ9-tetrahydrocannabinol (Δ9-THC).

In some embodiments, the THC derivative or intermediate is not CBD or CBDA.

In some embodiments, the THC, derivative, intermediate, metabolite, or fragment thereof, and combinations thereof may be present in the composition or combination in an amount of from about 0.5 mg/mL to about 30 mg/mL. In some embodiments, the THC, derivative, intermediate, metabolite, or fragment thereof, and combinations thereof may be present in the composition or combination in an amount of from about 1 mg/mL to about 30 mg/mL, from about 2 mg/mL to about 30 mg/mL, from about 5 mg/mL to about 30 mg/mL, from about 5 mg/mL to about 25 mg/mL, from about 5 mg/mL to about 20 mg/mL, from about 5 mg/mL to about 15 mg/mL, or about 5 mg/mL to about 10 mg/mL In some embodiments, the THC, derivative, intermediate, metabolite, or fragment thereof, and combinations thereof may be present in the composition or combination in an amount of about 0.5 mg/mL, about 0.75 mg/mL, about 1 mg/mL, about 5 mg/mL, about 10 mg/mL, about 15 mL, about 20 mg/mL, about 25 mg/mL, or about 30 mg/mL.

In some embodiments, a therapeutically effective dose of the THC, derivative, intermediate, metabolite, or fragment thereof, and combinations thereof may be from about 2.5 mg twice per day (B.I.D.) to about 10 mg twice per day (B.I.D.) In some embodiments, a therapeutically effective dose of the THC, derivative, intermediate, metabolite, or fragment thereof, and combinations thereof may be about 5 mg twice per day (B.I.D.), about 3.33 mg three times per day (T.I.D.), and/or about 1.67 mg three times per day (T.I.D.) These doses of THC, derivative, intermediate, metabolite, or fragment thereof, and combinations thereof may be delivered intravenously, orally (*e.g*., as an oral solution), via a suppository, and/or via inhalation.

In some embodiments, the THC, derivative, intermediate, metabolite, or fragment thereof, and combination thereof activates the CB2 receptor through a cyclic-GMP mediated mechanism to minimize cytokine storm induced ARF and/or ARDS. In some embodiments, the THC, derivative, intermediate, metabolite, or fragment thereof, and combinations also decreases the amount of pro-inflammatory IFN-γ and TNF-α, and increases anti-inflammatory Tregs, TGF-β, and IL-10. In some embodiments, the THC, derivative, intermediate, metabolite, or fragment thereof, and combinations is also small enough to pass through the pores and capillaries. In some embodiments, the THC, derivative, intermediate, metabolite, or fragment thereof, and combination thereof is also anti-viral by potentiating viral clearing in the spleen through CB2 receptor agonism.

### 2. Interferon

In some embodiments, the IFN, derivative, or intermediate (including prodrugs) include Type I IFN and Type III IFN. Type I IFNs include IFN-α and/or IFN-β. The IFN-α includes IFNα1a, IFNα2a, and IFNα2b, with or without pegylation. The IFN-β includes IFN-β-1a and IFN-β-1b. Type III IFNs include IFN-λ. The IFN-λ includes IFN-λ-1 (IL-29), IFN-λ-2 (IL-28a), IFN-λ-3 (IL-28b), and IFN-λ-4 (similar to IFN-λ-3).

In some embodiments, a therapeutically effective dose of the IFN, derivative, intermediate, metabolite, or fragment thereof, and combinations thereof may be about 5 IU per day or about 9 MIU per week of IFN-α, from about 6 MIU to about 8 MIU per day of IFN-β, and/or about 3 IU twice a day (B.I.D.) (via inhalation) or about 180 µg per week (via intramuscular injection) of IFN-λ. In some embodiments, 5 IU per day of IFN-α may be delivered to the patient. In some embodiments, 9 MIU of IFN-α may be delivered via intramuscular injection of 3 MIU of IFN-α three times per week. In some embodiments, about 6 MIU to about 8 MIU of IFN-β may be delivered daily. In some embodiments, about 3 IU of IFN-λ may be delivered twice a day (B.I.D.) via inhalation. In some embodiments, 180 µg of IFN-λ may be delivered via intramuscular injection weekly.

In some embodiments, IFN-α, a derivative, intermediate, metabolite, or fragment thereof, and combinations thereof reduces the severity of ARF and/or ARDS by upregulating MHC I proteins, which increases presentation of viral antigen peptides, thereby increasing activation of CD8+ T cell to cytotoxic T lymphocytes (CTLs). In some embodiments, increased CTLs enhance CTL-mediated apoptosis by macrophages. IFN-α also increases other antiviral mediators (such as protein kinase R and 2'-5' A synthetase), IL-6, and CRP. In some embodiments, the IFN-α, derivative, intermediate, metabolite, or fragment thereof, and combination thereof is also small enough to pass through the pores and capillaries.

In some embodiments, IFN-β, a derivative, intermediate, metabolite, or fragment thereof, and combination thereof reduces the severity of ARF and/or ARDS by upregulating the CD73 enzyme, reducing recruitment of leukocytes to the infected area, minimizing vascular leakage, reducing endothelial cell permeability, and increasing vascular integrity. In some embodiments, the CD73 enzyme generates adenosine and reduces ADP and ATP. In some embodiments, the IFN-β, derivative, intermediate, metabolite, or fragment thereof, and combinations thereof is also small enough to pass through the pores and capillaries.

In some embodiments, IFN-λ, a derivative, intermediate, metabolite, or fragment thereof, and combination thereof reduces the severity of ARF and/or ARDS by initiating an early-stage antiviral immune response at epithelial surfaces. In some embodiments, IFN-λ and IL-28Rα activate tyrosine kinases (TYK2 and JAK1) to phosphorylate IL-28Rα, which bind to STAT proteins leading to generate various antiviral proteins. In some embodiments, the IFN-λ, derivative, intermediate, metabolite, or fragment thereof, and combination thereof is also small enough to pass through the pores and capillaries.

### 3. Fluvoxamine

Fluvoxamine is a selective serotonin reuptake inhibitor (SSRI) that binds and agonizes a σ-1 non-opioid receptor, potentially modulating the immune response. Fluvoxamine has the strongest σ-1 non-opioid receptor binding of all agents known to bind to the σ-1 non-opioid receptor, but other agents which also bind the receptor and which may have useful effects include 3-MeO-PCP, 4-PPBP, Afobazole, Allylnormetazocine, Anavex 2-73, Arketamine, BD1031, BD1052, Berberine, Citalopram, Cocaine, Dehydroepiandosterone (DHEA), Dehydroepiandosterone sulfate (DHEA-S), Dextromethorphan, Dextrophan, N,N-Dimethyltryptamine, Dimemorfan, Ditolylguanadine, Escitalopram, Fluoxetine, Fluvoxamine, Igmesine, Ketamine, L-687.384, Lamotrigine, Memantine, Methamphetamine, Noscapine, OPC-14523, Opipramol, Pentazocine, Pentoxyverine, Phencyclidine, (+)-3-PPP, PRE-084, Pregnenolone sulfate, SA 4503, Siramesine, UMB23, and UMB82. The σ-1 non-opioid receptor is an endoplasmic reticulum chaperone protein that regulates cytokine production via the endoplasmic reticulum stress sensor inositol-requiring enzyme 1a (IRE1). Fluvoxamine reduces damage from inflammation through the σ-1 non-opioid receptor/IRE1 pathway. Fluvoxamine also reduces viral replication by inhibiting viral particle maturation/release through its action at the σ-1 non-opioid receptor on the endoplasmic reticulum. Moreover, fluvoxamine reduces the severity of the cytokine storm due to action at the σ-1 non-opioid receptor. This is a relatively milder immune modulation effect. Fluvoxamine also has a relatively stronger antiviral effect due to its action on the σ-1 non-ooioid receotors on the endoolasmic reticulum.

In some embodiments, a therapeutically effective oral dose of the Fluvoxamine, derivative, intermediate, metabolite, or fragment thereof, and combinations thereof can be about 150 mg or about 148mg or about 134 mg twice per day (B.I.D.), about 100 mg three times per day (T.I.D.), about 50 mg three times per day (T.I.D.) and/or about 50 mg two times per day (B.I.D.). The dosages given by other routes (*e.g.,* as an IV solution) would be calculated to achieve the same blood concentration of fluvoxamine or an analog thereof as the immediately aforementioned doses of fluvoxamine or analog thereof for example fluvoxamine maleate, would achieve in current commercially available tablet formulations.

Fluvoxamine, a derivative, intermediate, metabolite, or fragment thereof, and combinations thereof reduces the severity of ARF and/or ARDS by acting as a σ-1 non-opioid receptor agonist. Fluvoxamine reduces damage from inflammation and cytokine production through the σ-1 non-opioid receptor/IRE1 pathway. Fluvoxamine also reduces viral replication by inhibiting viral particle maturation/release through its action at the σ-1 non-opioid receptor on the endoplasmic reticulum and via a lysosomotropic effect. Other mechanisms of action also include autophagy modulation and SSRI inhibition of platelet activation.

### 4. Acetylcysteine

In some embodiments, the acetylcysteine, derivative, or intermediate (including prodrugs) includes, but are not limited to, N-acetylcysteine. In some embodiments, the acetylcysteine, derivative, intermediate, metabolite, or fragment thereof, and combinations thereof may be present in the composition or combination in an amount of about 600 mg. In some embodiments, the composition or combination is for administration twice per day (B.I.D.) In some embodiments, the acetylcysteine, derivative, intermediate, metabolite, or fragment thereof, and combinations thereof functions as a mucolytic in the composition or combination to remove at least some of the hyaline membranes in the alveoli to increase absorption of the other components of the composition or combination when the composition or combination is delivered via an inhalatory pathway (*e.g.,* using a nebulizer).

In some embodiments, the composition, or combination may also include one or more buffering agents, one or more preservatives, one or more antioxidants, one or more pharmaceutically acceptable diluents, excipients, carriers, one or more sweetener agents, one or more flavoring agents, or combinations thereof.

### Exemplary Dosing and Delivery

In some embodiments of a dosing regimen of a four component (e.g., acetylcysteine, THC, fluvoxamine, IFN) treatment the dose of acetylcysteine, derivative, intermediate, metabolite, or fragment thereof, and combinations thereof is about 600 mg twice per day (B.I.D.); the dose of THC is about 1.25 mg to about 5 mg twice per day (B.I.D.), about 3.33 mg three times per day (T.I.D.), and/or about 1.67 mg three times per day (T.I.D.); the dose of fluvoxamine is about 50 mg to about 450 mg or about 50 mg to about 250 mg twice per day (B.I.D.), or about 50 mg to 300 mg or about 50 mg to about 175 mg three times per day (T.I.D.), and/or about 50 mg to about 150 mg three times per day (T.I.D.); and the dose of IFN is as follows. In some embodiments, the dose of IFN is 2.5 IU twice per day (B.I.D.) of IFN-α2a; 3 IU twice per day (B.I.D.) of IFN-β-1; or 3 IU twice per day (B.I.D.) of IFN-λ1.

In some embodiments of a dosing regimen of a two component composition (e.g., fluvoxamine, dronabinol), the dose of fluvoxamine is about 25 mg to about 450 mg twice per day (B.I.D.), or about 50 mg to about 150 mg B.I.D., optionally 50 mg B.I.D., optionally 100 mg B.I.D., optionally 134 mg B.I.D. or 148 mg B.I.D. or 150 mg B.I.D.; or about 100 mg three times per day (T.I.D.), or about 50 mg to 300 mg three times per day (T.I.D.); and the dose of THC is about 2.5 mg to about 10 mg, for example 2.5 mg, 5 mg, 6.25 mg or 10 mg twice per day (B.I.D.), optionally about 6.7 mg T.I.D. about 4.2 mg T.I.D., about 3.33 mg three times per day (T.I.D.),, about 2.1 mg T.I.D., about 1.67 mg three times per day (T.I.D.) or about 0.83 mg T.I.D.. An oral formulation can consist of 6.25 mg dronabinol and 134 mg of fluvoxamine maleate dissolved in sesame seed oil encapsulated by a hydroxypropyl methylcellulose capsule colored externally with FD&C Red No. 21 and embossed in iron oxide black with "5.25." Another oral formulation can consist of 5.25 mg dronabinol and 148 mg of fluvoxamine maleate dissolved in sesame seed oil encapsulated by a hydroxypropyl methylcellulose capsule colored externally with FD&C Red No. 21 and embossed in iron oxide black with "5.25." The fluvoxamine may alternatively be deflocculated to particles of ~0.5 microns and suspended rather than be dissolved. The dosages given by other routes (*e.g.,* as an IV solution) would be calculated to achieve the same blood concentration of fluvoxamine as the immediately aforementioned doses fluvoxamine maleate would achieve in current commercially available tablet formulations.

Any of the compositions or combination disclosed herein may be delivered by inhalation (via a nebulizer), orally (*e.g.,* as an oral solution), intravenously, and/or via a suppository for the treatment of ARF and/or ARDS in a subject afflicted with a COVID-19 infection. In some embodiments, one or more of the compounds and/or the compositions may be packaged in nano-vesicles and delivered through the routes described herein.

In some embodiments of a two component composition, a dose of THC is about 2.5 mg, about 5 mg or about 6.25 mg twice per day (B.I.D.), about 4.2 mg T.I.D., about 3.33 mg three times per day (T.I.D.), and/or about 1.67 mg three times per day (T.I.D.)) and a dose of fluvoxamine about 25 mg to about 450 mg twice per day (B.I.D.), about 136 mg B.I.D., about 100 mg three times per day (T.I.D.), and/or about 50 mg three times per day (T.I.D.)) is combined for delivery through the routes described herein. In some embodiments, THC may be mixed with mineral oil to form a suspension and the fluvoxamine may be mixed into the suspension (*e.g.,* by shaking) to form a composition suspension in mineral oil, which is delivered to a patient through the routes described herein. In some embodiments, THC may be mixed with fluvoxamine in suspension, encapsulated in a dissolvable coating for delivery through the routes described herein and controlled release or immediate release.

### Synergistic Effects

Without wishing to be bound by theory, one or more of the following synergistic effects appear. (1) The acetylcysteine breaks up the mucus lining the alveoli allowing medications better access to the targeted cells of the lungs and access to the capillaries so that they can act synergistically as well. (2) The acetylcysteine acts as an anticoagulant that helps prevent the clotting of the microvasculature in the lungs, which would otherwise impair healthy gas exchange and contribute to morbidity and mortality. (3) The IFN ramps up intracellular viral defense mechanisms to help each cell survive viral exposure. One effect is to limit viral replication by halting transcription of the viral mRNA and production of the proteins needed for viral particle maturation and release. IFN accelerates the inflammatory response, but unfortunately also increases secretion of IL-6, which is a critical stimulator of the cytokine storm responsible for significant morbidity and mortality. (4) The THC suppresses the effect of IL-6 by acting on CB2 receptors present on white blood cells. This reduces and/or eliminates the cytokine storm. (5) Fluvoxamine halts viral replication bv inhibiting viral particle maturation/release through its action at the σ-1 non-opioid receptor on the endoplasmic reticulum. (6) The acetylcysteine in the bloodstream helps eliminate free radicals (some of which were caused by IFN, both endogenous and from the treatment). Otherwise, the free radicals may contribute to end organ damage that results in morbidity and mortality.

By combining one or more of the medications described herein, lower dosing is achievable, so for example the incidence of side effects from medications (such as development of serotonin syndrome from THC and fluvoxamine) can be reduced, and, therefore, treatment can be delivered more safely.

For many years, attempts have been made to treat different viral infections and some cancers with IFN. These efforts have largely been abandoned due to the severe side effects of interferon treatment. The treatments herein described allow for the use of IFN while simultaneously reducing its side effects and enhancing its function.

The acetylcysteine enhances function by enabling better access to the lung tissue and by blocking viral RNA replication and budding. The acetylcysteine reduces side effects by protecting and organs from IFN-induced free radical production and reducing the IFN-induced hypercoagulable state.

Without wishing to be bound by theory, THC may reduce side effects by halting the cytokine storm via reducing IL-6 response in white blood cells. Δ9-tetrahydrocannabinol (Δ9-THC) has been shown to have anti-IL-6 activity by activating receptors for THC, cannabinoid receptor (CB2), which is present predominantly in white blood cells through a cyclic-GMP mediated mechanism. Δ9-THC has been shown to decrease mortality in an animal model of cytokine storm induced ARF and/or ARDS. THC treatment may also decrease the amount of the pro-inflammatory cytokines, interferon-γ (IFN-y), and tumor necrosis factor-α (TNF-α). THC treatment also increases regulatory T cells (Tregs) and cytokines from Tregs, tumor growth factor- β (TGF-β) and interleukin-10 (IL-10). THC is also small enough to pass through the pores and capillaries. The side effect profile for Δ9-THC is well-established, and it is FDA approved for use in cancer patients.

It may also counteract the IFN-induced anorexia by its action in the central nervous system. Both effects appear to be mediated by the CB2 receptor, indicating that a primary function of the endocannabinoid system may be to finely balance/temporize the inflammatory effects of IFN.

Fluvoxamine also decreases the cytokine storm by decreasing IL-6 secretion.

The fluvoxamine may enhance function by halting viral reproduction, potentially assisting IFN in this role.

In some embodiments, THC and fluvoxamine, optionally in combination with acetylcysteine, will function well together as a composition or combination for treating ARF and/or ARDS in a subject afflicted with a COVID-19 infection. In some embodiments, the composition or combination may not include IFN. In particular, compositions or combinations including THC and fluvoxamine, and optionally acetylcysteine, may be delivered through an inhalatory mechanism. Without wishing to be bound by theory, such a composition or combination may also exhibit synergistic benefits as follows. (1) Acetylcysteine may allow access to the respiratory epithelium (via its mucolytic activity) and may maintain patency of the pulmonary vasculature (via its anti-thrombogenic activity), which may facilitate the other medications' access to the intravascular space. (2) Without wishing to be bound by theory, the fluvoxamine may stop viral replication via activity at the σ-1non-opioid receptor. Fluvoxamine is an agonist of the σ receptor Additionally, antidepressants have been shown to have antiviral and anti-inflammatory properties due to as yet unknown mechanisms which may prevent release of IL-6 from intracellular compartments. (3) Without wishing to be bound by theory, the THC may stop the cytokine storm via suppression of IL-6 activity as well as through other anti-inflammatory effects. (4) The acetylcysteine may attenuate/prevent end organ damage by assisting with free radical deactivation. Additionally, acetylcysteine has direct antiviral activity.

Without wishing to be bound by theory, THC may reduce the severity of the cytokine storm through action at the CB2 receptor, which suppresses IL-6 production. This is a relatively stronger immune modulation effect. Without wishing to be bound by theory, fluvoxamine may reduce the severity of the cytokine storm due to action at the σ-1 non-opioid receptor. This is a relatively milder immune modulation effect. Fluvoxamine also may have a relatively stronger antiviral effect due to its action on the σ-1 non-opioid receptors on the endoplasmic reticulum.

Without wising to be bound by theory, using both medications together results in a synergistic effect for one or more of the following reasons. THC and fluvoxamine reduce the severity of the cytokine storm by complementary mechanisms. Accordingly, less of each medication can be used. Both THC and fluvoxamine are metabolized by the same liver enzyme. Accordingly, using the medications independent of a fixed ratio in a patient may cause one medication to dangerously accumulate, increasing side effects or causing a potentially fatal event (e.g., serotonin storm). Delivering these two medications in a single composition and/or combination treatment may provide better control of the ratios of these two medications, allowing the ratio to be optimized to provide a safer composition/medication. Fluvoxamine treats anxiety, which is a common side effect of THC. THC treats nausea which is a common side effect of fluvoxamine. Using the two drugs together may reduce the side effects experienced when using either drug by itself. This side effect reduction may increase patient tolerance for the drug and may also allow for higher doses of the drugs to be used if needed.

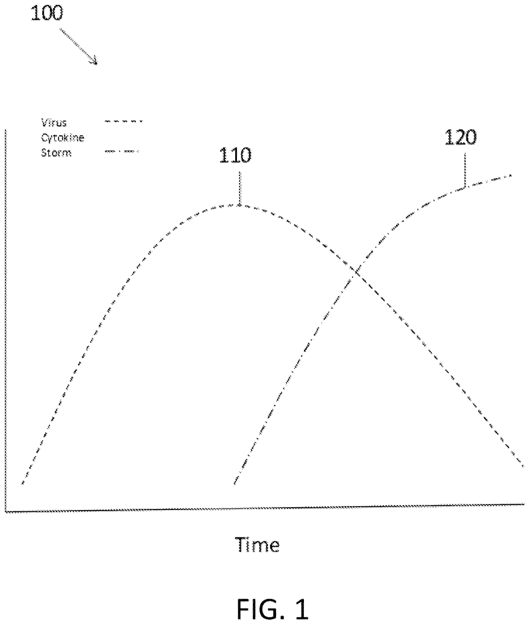

The direct viral destruction of patient's cells occurs early in the infection and tapers later in the infection as the cytokine storm destruction rises. This is shown in Fig 1, which is a disease severity versus time graph 100 depicting the early viral phase 110 and the late cytokine storm phase 120 of a COVID-19 infection according to some embodiments.

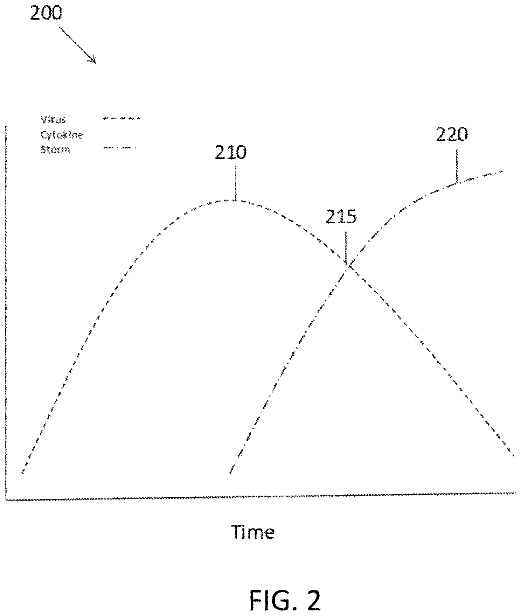

When patients present to a hospital, it may be difficult to know whether they are suffering more from the early viral phase of the COVID-19 infection, the later cytokine storm phase, or a middle combination of the two phases. Without wishing to be bound to theory, a combined medication composition may be beneficial to ensure treatment of both or either of the phases. If the medications are administered separately (*i.e.,* not combined), one medication may be prescribed when the other medication is more appropriate for the infection phase of the patient. Combining these two medications may avoid treatment errors. Fig. 2 is another disease severity versus time graph 200 depicting the early viral phase 210, the late cytokine storm phase 220, and an intermediate phase 215, which includes detrimental effects from both direct cell death from the virus and destruction caused by the cytokine storm. The combination of THC or an analog thereof and fluvoxamine or an analog thereof is expected to treat disease symptoms in all three of these phases 210, 215, 220.

In some embodiments, the methods, compositions, combinations and uses are for early treatment of ARDS or ARF, optionally in a subject afflicted with a COVID-19 infection or other coronavirus infection. Early treatment may, for example, be upon hospital presentation, upon positive diagnosis, or upon known exposure and at least one symptom associated with COVID-19.

In some embodiments, the methods, compositions, combinations and uses are for treatment of ARDS and/or ARF, optionally in a subject afflicted with a COVID-19 infection or other coronavirus infection, where the patient has an oxygen saturation that is less than 93%, a PaO₂/FiO₂ ratio of less than 300 mm Hg in room air, or a more than a 30% decrease in PaO₂/FiO₂ ratio in the previous 24 hours during hospitalization.

Without wishing to be bound by theory, THC reduces the secretion of multiple inflammatory cytokines by its action at CB2. It induces IL-10 secretion which reduces secretion of IL-6 and many other cytokines. It also causes apoptosis of activated leukocytes, which further acts to terminate the cytokine storm. Further, it may have action via the CB2 receptors of the splenic intermediate zone B cells, the lack of which prevents appropriate clearing of many different viruses in the spleen.

Fluvoxamine inhibits the secretion of viral particles from infected cells by its interaction at the sigma-one receptors on the endoplasmic reticulum. Fluvoxamine is thought to concentrate in pulmonary tissue. Fluvoxamine is theorized to act like other sigma-one receptor ligands and inhibit IL-6 secretion by its action at the endoplasmic reticulum.

Without wishing to be bound by theory, the presence of THC should help further suppress the cytokine storm by its suppression of IL-6 secretion by a different mechanism than THC. It is at least this synergy contributed to the 93% survival rate noted in the pivotal study.

THC was seen to reduce mortality by 68% in patients hospitalized for hypoxia caused by COVID-19 associated inflammation as shown in Example 1. It is theorized that this is due to termination of the cytokine storm by apoptosis of activated leukocytes, suppression of secretion of IL-6 and other cytokines, and by increasing viral clearance in the spleen. The survival rate was 93.5%. It is further theorized that fluvoxamine can increase the survival rate by using a different mechanism to suppress IL-6 secretion and suppress viral particle release. That fluvoxamine is believed to concentrate in pulmonary tissue makes it particularly useful in treating COVID-19, which most commonly initially targets lung tissue. It is theorized this synergistic suppression of IL-6 production and suppression of circulating virus can increase the COVID-19 survival rate by 1%-4%. If survival can achieve 97.5%, this approximates a cure.

### Exemplary Method for Treating ARF and/or ARDS in a subject afflicted with a COVID-19 Infection or other coronavirus infection

In some embodiments, a method for treating ARF and/or ARDS (optionally resulting from COVID-19 infection) using the compositions or combinations described herein include administering one or more of the compositions or combinations to a patient suffering from ARF and/or ARDS using a nebulizer. In some embodiments, the composition or combination may be administered to the patient twice 24 hours apart. While specific components of various compositions or combinations are described herein, one of skill in the art would understand that components may be substituted for specific components without going beyond the scope of the claims. For instance, various THC derivatives with greater anti-inflammatory/anti-IL-6 activity may be used in compositions or combinations. While specific concentrations of various components of the composition or combinations are described herein, one of skill in the art would understand that these concentrations can be varied to deliver therapeutically effective doses of each component in the composition or combination at the same time.

In some embodiments, the method for treating ARF and/or ARDS (optionally in a subject afflicted with a COVID-19 infection) may also include providing patients with standard of care treatments including glucocorticoid, hydroxychloroquine, azithromycin, heparin, antivirals, pressor support, and ventilation. In some embodiments, patients suffering from ARF and/or ARDS may be treated with the compositions or combinations described herein only if they show an O₂ saturation of less than 93%, a PaO₂/FiO₂ ratio of less than 300 mm Hg in room air, or a more than a 30% decrease in PaO₂/FiO₂ ratio in the previous 24 hours during hospitalization. In some embodiments, the method may include administering a bronchodilator about 10 minutes to about 15 minutes before administering the composition or combination to increase the absorption of the composition or combination by the patient.

While some embodiments describe delivery of compositions or combination by inhalation (via a nebulizer), the composition or combination described herein may be administered orally (*e.g.,* as an oral solution), intravenously, and/or via a suppository for the treatment of ARF and/or ARDS in a subject afflicted with a COVID-19 infection.

The terms "about," "substantially," and "approximately" as used herein mean a reasonable amount of deviation of the modified term such that the end result is not significantly changed. These terms of degree should be construed as including a deviation of at least ±5% or at least ±10% of the modified term if this deviation would not negate the meaning of the word it modifies. For example, "about 100 mg" can include 95 mg to 105 mg.

As used herein, the phrase "an effective amount" refers to an amount sufficient to bring about intended or beneficial results in a patient. An effective amount can be administered in one or more doses, applications, or administrations. Effective amounts for a particular substance are within the ordinary skill in the pharmaceutical arts.

As used herein, the phrase "pharmaceutically acceptable" refers to those compositions, materials, compounds, and/or dosage forms that are, within the scope of sound medical judgment, suitable for use in contact with human beings and animals, patients without excessive irritation, allergic response, toxicity, or other problems or complications with a reasonable risk/benefit ratio. Pharmaceutically acceptable ingredients are known in the pharmaceutical arts. Official publications known in the pharmaceutical arts, such as The United States Pharmacopeia, describe criteria for analysis of pharmaceutical acceptability of various components of interest.

As used herein, the term "dronabinol" refers to a compound having the chemical formula of C₂₁H₃₀O₂ and a molecular weight of about 314.46. Dronabinol is synthetic delta-9- tetrahydrocannabinol (delta-9-THC). Dronabinol may be a light-yellow resinous oil that is sticky at room temperature that hardens upon refrigeration. Dronabinol is insoluble in water and may be formulated in for example sesame oil. It has a pKa of 10.6 and an octanol-water partition coefficient: 6,000:1 at pH 7. Dronabinol formulations can include for example the following inactive ingredients: FD&C Yellow No. 6, gelatin, glycerin, purified water, sesame oil, titanium dioxide, iron oxide black, shellac glaze, isopropyl alcohol, n-butyl alcohol, propylene glycol, hydroxypropyl methyl cellulose and ammonium hydroxide, FD&C Blue No. 2 and/or FD&C Red No. 21.

As used herein, the term "analog" includes structurally related molecules that have a similar or better potency and/or other biological property as the base compound and includes families of molecules and derivatives, intermediates, fragments and/or combinations thereof. For example, analogs of THC includes THC: Δ9-tetrahydrocannabinol-C5 (Δ9-THC-C 5), Δ9-tetrahydrocannabinol-C4 (Δ9-THC-C 4), Δ9-tetrahydrocannabivarin (Δ9-THCV-C 3), Δ9-Tetrahydrocannabiorcol (Δ9-THCO C-1), Δ9-Tetrahydrocannabinolsäure (Δ9 THCA-C-5 A), Δ9-Tetrahydrocannabinolsäure B (Δ9 THCA-C-5 B), Δ9-Tetrahydrocannabinolsäure-C4 (Δ9 THCA-C-4 A and/or B), Δ9-Tetrahydrocannabivarinsäure A (Δ9-THCVA-C 3 A), Δ9-Tetrahydrocannabiorcolsäure (A9-THCOA-C 1 A and/or B), (-)-Δ8-trans-(6aR,10aR)-Δ8-tetrahydrocannabinol (Δ8-THC-C 5), (-)-A8-trans-(6aR, 10aR)-Tetrahydrocannabinolsäure A (Δ8-THCA-C 5 A); (-)-(6a S,10a R)-Δ9-tetrahydrocannabinol ((-)-cis-Δ9-THC-C 5). In some embodiments, the THC, derivative, or intermediate include tetrahydrocannabinolic acid (THCA), tetrahydrocannabivarin carboxylic acid (THCVA), tetrahydrocannabivarin (THCV), and combinations thereof. Examples of analogs of IFN include Type I IFN and Type III IFN. Type I IFNs include IFN-α and/or IFN-β. The IFN-α includes IFN-α1a, IFN-α2a, and IFN-α2b, with or without pegylation. The IFN-β includes IFN-β-1a and IFN-β-1b. Type III IFNs include IFN-λ. The IFN-λ includes IFN-λ-1 (IL-29), IFN-λ-2 (IL-28a), IFN-λ-3 (IL-28b), and IFN-λ-4 (similar to IFN-λ-3). An example of an analog of acetylcysteine includes N-acetylcysteine.

As used herein, the term "late-stage ARF and/or ARDS" also "late-stage ARF and/or late-stage ARDS" refers to a state in which a subject having ARF and/or ARDS requires hospitalization and/or ventilation and/or oxygen supplementation. Subjects with late-stage ARF and/or ARDS may have pulmonary edema, radiographic findings of ARF/ARDS (e.g., diffuse bilateral opacities), and/or hypoxia.

As used herein, the term "hypoxia or hypoxic" refers to when a subject has an O₂ saturation of less than about 93%, a PaO₂/FiO₂ ratio of less than 300 mm Hg in room air, or more than about a 30% decrease in PaO₂/FiO₂ ratio in the previous 24 hours.

As used herein "fluvoxamine" includes pharmaceutically acceptable salts thereof. As used herein, the term "fluvoxamine" refers to a chemical compound with the IUPAC name of 2-[(E)-[5-methoxy-1-[4-(trifluoromethyl)phenyl]pentylidene]amino]oxyethanamine.

As used herein "acetylcysteine" includes pharmaceutically acceptable salts thereof.

As used herein, the term "tetrahydrocannabinol (THC)" includes Δ9-tetrahydrocannabinol (Δ9-THC).

As used herein, the term "Δ9-tetrahydrocannabinol," "Δ9-THC," or the like refers to a chemical compound with the IPUAC name (-)-(6aR,10aR)-6,6,9-trimethyl-3-pentyl-6a,7,8,10a-tetrahydro-6H-benzo[c]-chromen-1-ol) and includes pharmaceutically acceptable salts thereof.

As used herein, the term "therapeutically effective amount" includes an amount of a substance and/or compound, or an amount of the combination of substances and/or compounds, *e.g.,* to treat or prevent a disorder, disease or condition in a patient, or one or more symptoms of a disorder, disease or condition in a patient. The combination of substances and/or compounds is preferably a synergistic combination, which occurs when the effect of the substances and/or compounds when administered in combination is greater than when the additive effect of the substances and/or compounds when administered alone. Synergy can include increased activity, lower cytotoxicity, or some other beneficial effect of the combination compared with the individual substances and/or compounds.

As used herein, and unless otherwise indicted the term "treating" or "treatment" means obtaining beneficial results and/or reversing, alleviating, inhibiting the progress of, or preventing the disorder, disease or condition to which such term applies, or one or more symptoms of such disorder, disease or condition. For example, decreasing mortality, decreasing time in ICU and/or hospital are beneficial results.

In this specification, amounts, concentrations, etc., of various substances and/or components are often presented in a range format. The disclosure of range formats is merely for brevity and convenience and are examples of the scope of the claimed inventions. Consequently, the disclosure of a range should be construed to have specifically disclosed all the possible sub-ranges as well as individual numerical values within that range. For example, description of a range such as 1% to 5% should be considered to have specifically disclosed sub ranges such as 1% to 4%, 2% to 5%, 2% to 3%, 3% to 4%, 4% to 5%, 3% to 5% etc., as well as individual numbers within that range, such as, 2%, 4%, 5% etc.

Various exemplary embodiments of the invention are described herein. Reference is made to these examples. They are provided to illustrate more broadly applicable aspects of the invention. Various changes may be made to the invention described and equivalents may be substituted without departing from the true spirit and scope of the invention. In addition, many modifications may be made to adapt a particular situation, material, composition of matter, process, process act(s) or step(s) to the objective(s), spirit, or scope of the present invention. Further, as will be appreciated by those with skill in the art that each of the individual variations described and illustrated herein has discrete components and features which may be readily separated from or combined with the features of any of the other several embodiments without departing from the scope or spirit of the present inventions. All such modifications are intended to be within the scope of claims associated with this disclosure.

Any of the devices described for carrying out the subject diagnostic or interventional procedures may be provided in packaged combination for use in executing such interventions. These supply "kits" may further include instructions for use and be packaged in sterile trays or containers as commonly employed for such purposes.

The invention includes methods that may be performed using the subject devices. The methods may comprise the act of providing such a suitable device. Such provision may be performed by the end user. In other words, the "providing" act merely requires the end user obtain, access, approach, position, set-up, activate, power-up or otherwise act to provide the requisite device in the subject method. Methods recited herein may be carried out in any order of the recited events which is logically possible, as well as in the recited order of events.

A single substance or component may meet more than a single functional requirement, provided that the single substance or component fulfills the more than one functional requirement as specified by claim language.

Exemplary aspects of the invention, together with details regarding material selection and manufacture have been set forth above. As for other details of the present invention, these may be appreciated in connection with the above-referenced patents and publications as well as generally known or appreciated by those with skill in the art. The same may hold true with respect to method-based aspects of the invention in terms of additional acts as commonly or logically employed.

The invention has been described in reference to several examples optionally incorporating various features. Various changes may be made to the invention described and equivalents (whether recited herein or not included for the sake of some brevity) may be substituted without departing from the true spirit and scope of the invention. In addition, where a range of values is provided, it is understood that every intervening value, between the upper and lower limit of that range and any other stated or intervening value in that stated range, is encompassed within the invention.

Also, it is contemplated that any optional feature of the inventive variations described may be set forth and claimed independently, or in combination with any one or more of the features described herein. Reference to a singular item, includes the possibility that there are plural of the same items present. More specifically, as used herein and in claims associated hereto, the singular forms "a," "an," "said," and "the" include plural referents unless the specifically stated otherwise or the content clearly dictates otherwise. Thus, for example, a composition containing "a compound" includes a mixture of two or more compounds. It should also be noted that the term "or" is generally employed in its sense including "and/or" unless the content clearly dictates otherwise. In other words, use of the articles allows for "at least one" of the subject item in the description above as well as claims associated with this disclosure. It is further noted that such claims may be drafted to exclude any optional element. As such, this statement is intended to serve as antecedent basis for use of such exclusive terminology as "solely" and "only" in connection with the recitation of claim elements, or use of a "negative" limitation.

As used in this application and claim(s), the word "consisting" and its derivatives, are intended to be close ended terms that specify the presence of stated features, elements, components, groups, integers, and/or steps, and also exclude the presence of other unstated features, elements, components, groups, integers and/or steps.

The term "composition" as used herein, a mixture comprising two or more compounds. In an embodiment, a composition is a composition of two or more distinct compounds. In a further embodiment, a composition can comprise two or more "forms" of the compounds, such as, salts, solvates, or, where applicable, stereoisomers of the compound in any ratio. A person of skill in the art would understand that a compound in a composition can also exist as a mixture of forms. For example, a compound may exist as a hydrate of a salt. All forms of the compounds disclosed herein are within the scope of the present disclosure.

Without the use of such exclusive terminology, the term "comprising" in claims associated with this disclosure shall allow for the inclusion of any additional elementirrespective of whether a given number of elements are enumerated in such claims, or the addition of a feature could be regarded as transforming the nature of an element set forth in such claims. Except as specifically defined herein, all technical and scientific terms used herein are to be given as broad a commonly understood meaning as possible while maintaining claim validity.

The breadth of the present invention is set out in the appended claims and the scope of claim language.

The above disclosure generally describes the present application. A more complete understanding can be obtained by reference to the following specific examples. These examples are described solely for the purpose of illustration. Changes in form and substitution of equivalents are contemplated as circumstances might suggest or render expedient. Although specific terms have been employed herein, such terms are intended in a descriptive sense.

The following examples are illustrative of the present disclosure:

### Examples

### Example 1: Clinical Study of THC

The loss of human life caused by COVID-19 has been well documented.¹ The disease is thought to have two phases.² The first phase consists of viral replication and spread, in which the virus infects the nasal epithelium, then the lung tissue via inhalation, then other organs via hematogenous spread. Some people are unable to contain the disease and go on to the second stage of the disease in which they develop a cytokine storm. The primary etiology of the cytokine storm appears to be failure of NK cells and cytotoxic T-Cells to kill infected cells. This failure leaves the infected cells and the cells trying to kill them stuck in the pro-inflammatory state secreting pro-inflammatory cytokines.³ IL-6 has been identified as the cytokine which appears to be the primary driver of the pro-inflammatory cytokine cascade.⁴

Tocilizumab is an antibody designed to antagonize the binding of IL-6 to its receptor. It has been successfully used to treat cytokine storm seen in other disease states.⁵ In May of 2020 it was used at many centers treating COVID-19 because it was hoped it would be successful in stopping the cytokine storm in those patients. It was not as effective as hoped. When it failed to be as effective as hoped,⁶ it was considered the model of cytokine storm which emphasized the central role of IL-6 was incorrect. It was then thought that the problem was with Tocilizumab. Tocilizumab is an antibody, and, thus, it has a size of about 150 kiloDaltons (kDa).7 Capillary apertures have a size on average of only about 40 kDa.⁸ It was reasoned that Tocilizumab may be trapped in the intravascular compartment⁹, unable to reach the tissues where the cytokine storm is raging.

The challenge was to find a small molecule with anti-IL-6 activity. The active fragments of Tocilizumab were first considered, but stabilized antibody fragments are likely still too large, having a size of about 47 kD.¹⁰ Methotrexate,¹¹ had a side-effect profile that was too high, and its use might have been too dangerous in these very sick patients. The inventor considered the endocannabinoid system which had been posited as a natural counterbalance to the body's inflammatory mediators.¹²

Drugs with activity on endocannabinoid receptors had a low, well-known side-effect profile, were small in size, already had FDA approval and had known anti-inflammatory effects.¹³ While the psychogenic effects for which endocannabinoids are famous occur primarily through the CB1 receptor,¹² the immune response is thought to be mediated primarily through the CB2 receptor,¹² a receptor that is present on nearly all white blood cells.¹⁴ There was limited published research available on the exact mechanism by which endocannabinoids exerted an anti-inflammatory effect,¹⁵ and in fact some research had suggested that endocannabinoids could be pro-inflammatory.¹⁶

An off-patent, previously FDA approved agonist of the CB1 and CB2 receptors to counteract the activity of IL-6 was administered to 77 sequentially admitted patients with COVID-19 who required supplemental oxygen. 72 patients survived, demonstrating a 94% survival rate in the treatment group versus an 80% survival rate with current standard of care treatment alone (p-value 0.02). Hospital days were reduced by 37% in the treated group when compared to the control group (p-value 0.01).

### Methods/Materials

Between October 10, 2020, and December 9, 2020, at one hospital in Las Vegas, Nevada, 97 consecutive COVID+ patients were seen by one infectious disease physician. All patient identifying data were removed prior to analysis. The study was exempt from IRB review pursuant to the terms of the U.S. Department of Health and Human Service's Policy for Protection of Human Research Subjects at 45 C.F.R. §46.104(d) Category 4. Inclusion criteria were age 18 years old or greater and the need for hospitalization for oxygen supplementation. Exclusion criteria included pregnancy. 13 patients who did not have hypoxia and two who were pregnant were excluded. The remaining 82 patients were offered dronabinol. Four patients refused. 78 patients received dronabinol. Patients were started on either 2.5 mg or 5 mg dronabinol orally BID. 68 patients received 5 mg orally BID from the day of first consult until the dayof discharge, with the exception of one patient with bipolar disorder who intermittently refused treatment but received treatment the majority of days of his stay. The patients who did not tolerate 5 mg orally BID were reduced to 2.5 mg orally BID. 10 patients received 2.5 mg dronabinol orally BID. They were either started on that dose due to perceived frailty by the consulting infectious disease physician or were started on it after either observed or reported side-effect from 5 mg orally BID. One of those patients had multiple comorbidities and withdrew herself from all treatments. She was therefore removed from analysis. She eventually succumbed to her medical issues. 9 patients completed treatment with 2.5 mg of dronabinol orally BID until the date of their discharge or death (one included patient, age 85). Therefore, 77 patients received dronabinol and were included in this study. The control data consisted of 44 consecutive COVID+ patients seen by the same infectious disease physician between June 6, 2020, and August 25, 2020, at one hospital in Las Vegas, Nevada. Patients in both groups received current standard of care treatment for COVID-19 patients requiring oxygen supplementation. COVID-19+ status was confirmed via nasal swab RNA testing.

The details of the control and treatment groups were compared by t-tests (age, CRP peak), Mann-Whitney test (number of comorbidities), or chi-square tests (gender and critical care). Due to the small number of patients who died in the study, a Fisher's exact test was used to test for differences in mortality rate between control and treatment groups. The mortality data was analyzed using per-protocol analysis. The length of stay in the hospital (LOS) was compared between treatment groups using a gamma regression model with a log link function (Basu *et al.* 2004). The 95% confidence intervals around mean LOS were estimated in log scale and back-transformed for reporting. Descriptive comparisons between patients who died and those who survived within each treatment group was done using means and 95% confidence intervals based on pooled standard errors due to the low mortality rate coupled with small sample size. Analyses were conducted in R3.6.3 (www.r-project.org) (R Core Team (2020). R: A language and environment for statistical computing. R Foundation for Statistical Computing, Vienna, Austria.

Standard of care treatment was to start patients with suspected COVID-19 infection on ceftriaxone and azithromycin medication until procalcitonin levels dropped below 0.20. The patients were all treated with dexamethasone and subcutaneous heparin. Once COVID-19 was confirmed, remdesivir and convalescent plasma were given. Respiratory support was offered per standard protocols. After September 2020, if the patient was 18 years or older, required oxygen, and was not pregnant, dronabinol was offered.

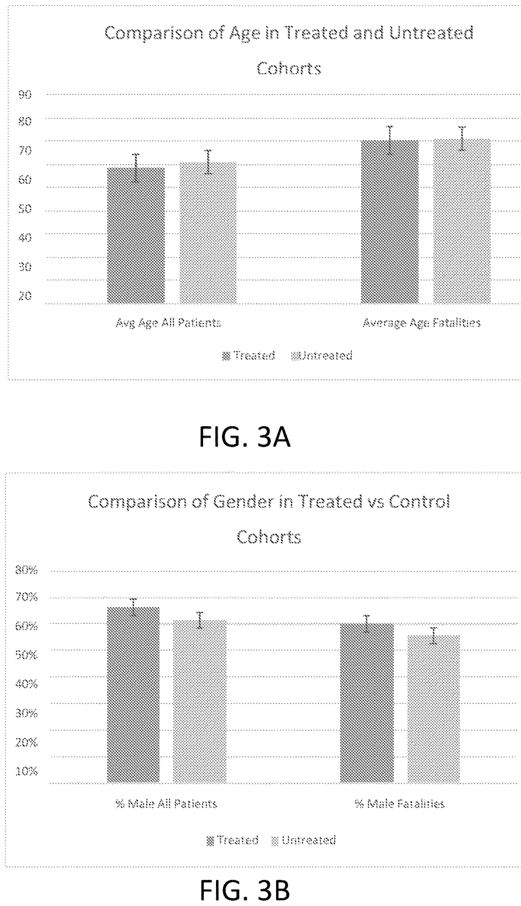

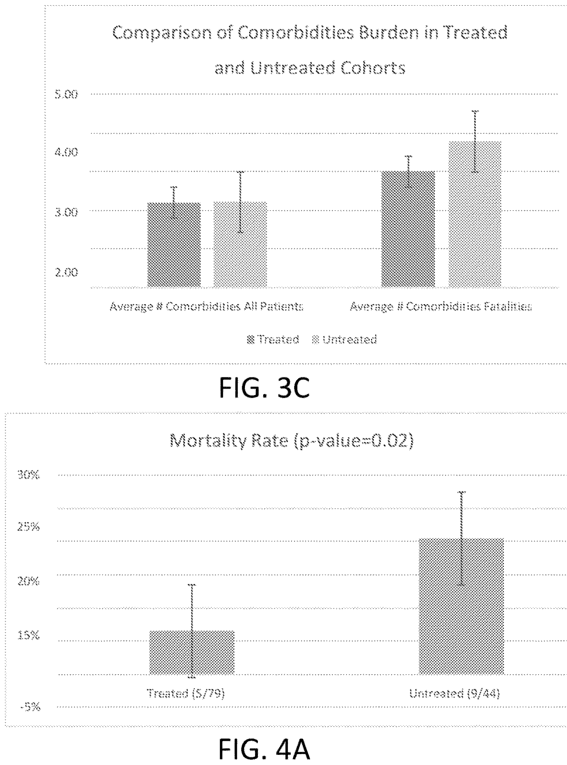

Treatment control groups were similar in age, gender and comorbidities demonstrating no statistically significant differences with the treated group (see Figs. 3A-3C).

### Results

There were 5 deaths among the 77 patients in the treatment group included in the study. Causes of death are explained in Table 1. One patient had a 15% ejection fraction prior to hospital admission and succumbed to heart failure. One patient developed a fatal tension pneumothorax while on a ventilator. One patient developed fatal gastrointestinal bleeding after receiving Decadron. Two patients died of pulmonary compromise directly attributed to COVD-19 induced lung damage. These patients are listed in Table 1. The remaining treated patients were able to be removed from supplemental oxygen and discharged home. The causes of mortality in the control group are listed in Table 2.

### All-cause mortality:

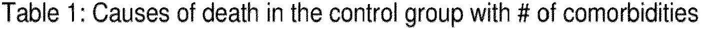

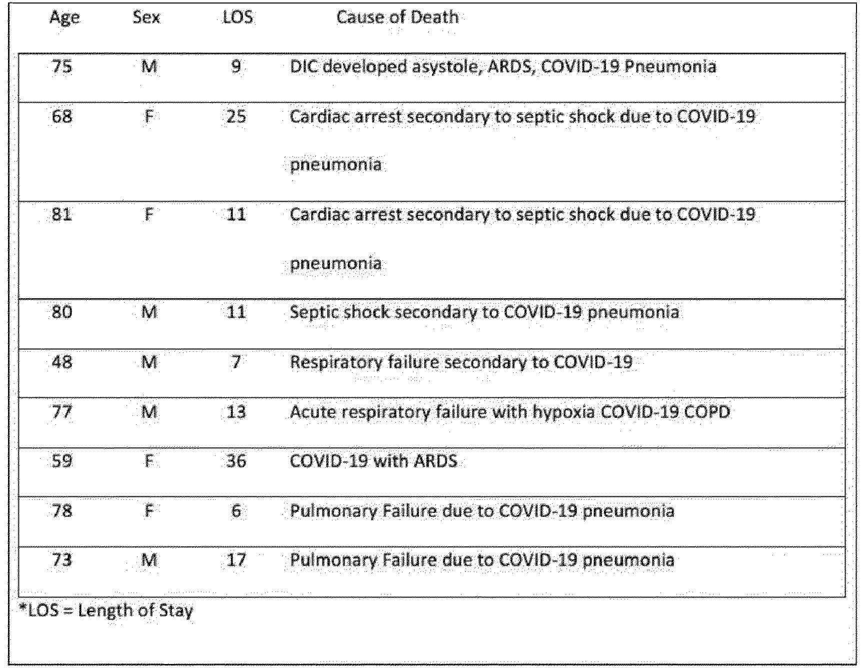

**Table 2 - Causes of death in the treatment group**

| AGE | M/F | LOS (Days) | Cause of Death |
|---|---|---|---|
| 85 | F | 28 | Acute respiratory failure with hypoxia - Lower GI Bleed, prior Idiopathic Pulmonary Fibrosis |
| 61 | M | 4 | Cardiopulmonary arrest secondary to Hypoxia from COVID-19 pneumonia - prior EF 15% |
| 48 | F | 21 | Cardiac arrest due to tension pneumothorax due to ventilator |
| 84 | M | 15 | Acute hypoxic respiratory failure secondary to COVID-19 pneumonia |
| 72 | M | 13 | Acute hypoxic respiratoryfailure secondary to COVID-19 pneumonia |
| 84 | F | 13 | Acute respiratory failure from pulmonary complications of Covid-19 pneumonia patient withdrew consent for treatment, therefore not included in analysis |

| | | | |
|---|---|---|---|
| *LOS = Length of Stay | | | |

Mortality rates, CRP levels and length of stay for patients in the group of patients treated withdronabinol are compared to controls and all are presented in Figs. 4A-C.

Mortality rate was reduced from 20.5% of hospitalized COVID-19 patients requiring oxygen supplementation in the untreated group, to 6.5% in the treated group. This represents a 68% reduction in mortality in the treated group (p-value=0.02) (Fig. 4A).

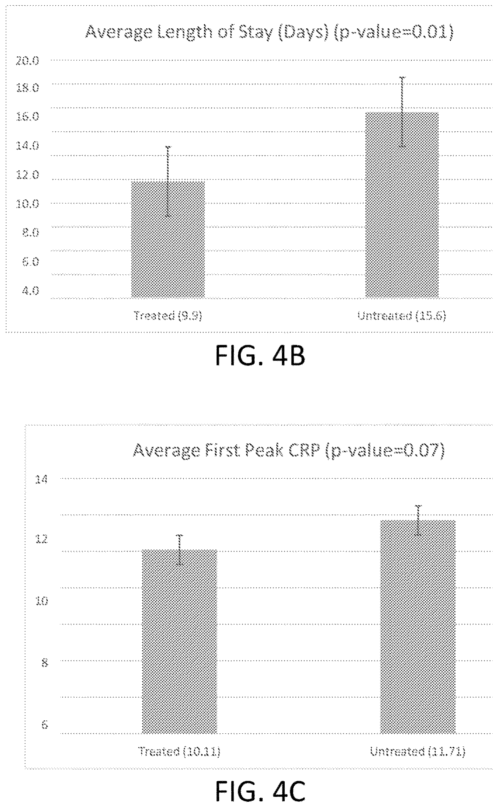

Length of stay in the hospital was also significantly reduced from 15.6 days in the untreated group to 9.9 days in the treated group. This is a reduction of 37% in length of stay in the treated group (p-value=0.01) (Fig. 4B).

A measure of degree of initial inflammation, average first peak C-reactive protein (CRP) levels were lower in the treatment group. This represents a difference of 14% (p-value of 0.07), which did not reach the level of statistical significance (p-value < 0.05) (Fig. 4C).

This study demonstrates a reduction in mortality for patients admitted to the hospital for COVID-19 who required supplemental oxygen and were treated with 2.5 mg or 5 mg dronabinol orally BID in addition to current standard of care treatment as compared to those who received only current standard of care treatment. (Fig. 4A)

Age appeared to play a significant role in determining survival as demonstrated by the average age of mortality being significantly higher than the average age of the patients in the study (Fig. 3A). It is believed that this is caused by two factors. First, older patients have diminished interferon-induced anti-viral immunity because they have impaired gut absorption of two nutrients critical to intracellular defense signaling pathways: zinc and Vitamin D.¹⁸ Second, older patients are more chronologically distant from their last MMR booster injection than younger patients and the necessary memory T-cells are more likely to have died or become dormant through age-related immunosenescence.¹⁹

Gender appeared to play a significant role in oxygen requirement, since more men were entered in the study than women, suggesting that fewer women with COVID-19 develop hypoxia. In this study, treatment with dronabinol seemed to temporize the effect of gender. A larger study would be required to confirm this. (Fig. 3B) The genes for toll-like receptor (TLR) 3,7 and 9, proteins responsible for intracellular recognition and presentation of viral proteins, are located on the X chromosome, giving women 2x the likelihood that men have of having functional copies.²⁰ Also, CD4+ T-cells and B-cells both have receptors for estrogen, suggesting estrogen signaling plays a role in enhancing humoral immunity.²⁰ The generation of humoral immunity appears critical in overcoming COVID-19 infection, as described later.

CRP is elevated in patients experiencing cytokine storm.³ The reduction in early peak CRP level and the reduction in the duration of symptoms as evidenced by the reduced length of stay coincided with the administration of dronabinol, suggesting dronabinol was the cause of the attenuation/termination of the cytokine storm in these patients. (Figs. 4B and 4C)

There is no evidence for or against the presumption that viral load was diminished since levels of viral load were not measured during this study. It is known that cytokine storm causes significant morbidity though damage to tissue by inflammatory processes. It is therefore possible that cessation of the cytokine storm alone was enough to reduce symptoms enough to relieve the patients' oxygen requirements and allow discharge. It is too soon to know if they will be readmitted again after discharge due to re-ignition of the cytokine storm after the dronabinol stops if viral reproduction continues.

So far there have been few re-admissions since October 2020. This suggests that for most patients, cessation of the cytokine storm allows the body to mount sufficient defense to halt viral reproduction and prevent re-escalation of the cytokine storm. Dronabinol activates CB1 and CB2 receptors. CB1 and CB2 receptors increase the production of human cytokine synthesis inhibitory factor (interleukin 10). Interleukin 10 (IL-10) suppresses production of IL-6, IFN-gamma, and IL-2, all pro-inflammatory cytokines. These cytokines draw cytotoxic CD8+ T-cells and NK cells to the site, which then disgorge pro-inflammatory cytokines of their own. The IL-10 simultaneously stimulates B-cell reproduction directly, and, by acting on CD4+ T-cells²¹, suppresses the innate immune response to intracellular pathogens. IL-10 may act as aswitch in the immune system to direct the immune response away from the innate immune response, which triggers activation of NK cells and cytotoxic CD8+ T-cells, and towards the adaptive immune response, consisting of induction of CD4+ T-cells to influence B-cells to reproduce and produce the antibodies needed to address the provocative agent.

This coincides with the observation that symptomatic patients respond well to convalescent plasma, implying that their own bodies have been unable to produce enough antibody to the virus to protect them. It is possible the IL-10 boost provided by dronabinol gives the body the kick-start it needs to begin producing sufficient antibodies on its own.

A second study could be designed in which COVID-19+ patients with cytokine storm are treated with dronabinol and IL-6 levels (more sensitive than CRP), IL-10 levels, viral load, and levels of anti-body to thevirus are checked frequently in treatment and control groups. Such a study could demonstrate whether or not dronabinol increases IL-10 production, decreases IL-6 production, induces antibody production specific to the virus, and thereby reduces circulating viral load. If such reductions correlated in symptomatic improvement, the mechanism of action of dronabinol would be more clearly and certainly elucidated. Additionally, it may be possible to better quantify the inflammatory response in the treated vs. untreated groups by creating measuring the area under the curves generated by plotting the IL-6 levels against duration of symptoms.

5 mg orally BID was chosen as the dose of dronabinol since maximal efficacy was desired and this was the maximum dose recommended by the FDA. Only one patient required lower dosing becauseof behavioral side-effects, and 3 total patients were on 2.5 mg dronabinol BID who did not appear to receive less benefit than those taking 5 mg orally BID.

In one of the patients who died (85yo LOS 28d) the level of CRP rose after several days of initial improvement in the CRP level, suggesting that perhaps viral replication continued and possibly overwhelmed the body's response to dronabinol. In the other patients the CRP levels exhibitedsignificant volatility without obvious response to the dronabinol.

It is theorized that this treatment deals primarily with the second stage of the disease, the cytokine storm.

### Example 2: Clinical Study of Combination of THC and Fluvoxamine

A composition or combination as described herein comprising Δ9-THC (e.g. dronabinol) and fluvoxamine will be used. A randomised clinical trial on patients admitted to the hospital for oxygen supplementation due to confirmed COVID-19 will be performed and one or more of the following outcomes will be evaluated:
- All-cause mortality rate;
- Number of says alive and free of hospitalisation;
- Clinical status of patients 14 days after randomization using the World Health Organization 6-point Ordinal Scale for Clinical Improvement (Table 3);
- Number of days of mechanical ventilation from randomization to day 28 or discharge;
- Evaluate plasma cytokine (or other marker) concentrations (e.g. IL-6, IL-1, IL-10, CRP) level changes at baseline and optionally days 2, 4, 6, 8, 10, and day 12 or day of hospital discharge;
- Number of days of hospitalization;
- Number of days in the intensive care unit (ICU) during hospitalization;
- Evaluate changes in serum anti-COVID-19 antibody levels and viral load in response to treatment for example at baseline, day 5, and day 28 or day of hospital discharge.

Patients in the intervention group will receive about 5.25 mg or about 10.5 mg of THC and for example about 148 mg fluvoxamine maleate optionally orally twice a day, until resolution of hypoxia or discharge from the hospital, in addition to standard of care.

Patients in the control group I will receive standard of care alone. A suitable ventilation strategy as determined by a skilled person can be used as part of the standard of care. Patients with negative laboratory tests included in the study will be evaluated by a blinded committee formed by two critical care physicians of the research group with experience treating COVID-19 patients. This committee will take into account the timing of testing, clinical symptoms, and analysis of chest image (computed tomography scan of the lungs, or chest X-ray) to define if the patient has COVID-19 infection with negative laboratory tests (probable COVID-19 infection) or if the patient possibly does not have COVID-19 infection. Patients with positive polymerase chain reaction (PCR) tests for SARS-CoV2 will be deemed to have confirmed COVID-19 infection. The main analysis will be based on the intention-to-treat principle, with additional sensitivity analysis regarding the COVID-19 infection status (confirmed versus not confirmed).

### Randomization and allocation concealment

Patients will be randomized in a 1:1 ratio to one of the two groups: standard treatment plus THC/fluvoxamine composition or combination (intervention group) and standard treatment without THC/fluvoxamine composition or combination (control group).

### Blinding

This trial is where the investigators, caregivers, and patients will be blinded regarding the intervention. All statistical analyses will be performed in a blinded manner in respect to group allocation.

### Data collection

Unidentified patient data will be collected through an electronic online data capture tool (REDCap). Demographic and baseline data, height, weight, use of THC or fluvoxamine prior to randomization, lab test results, and daily maximum oxygen supplementation requirement will be collected for all patients. The SOFA, PHQ-9, GAD-7 scores will be collected in addition to the Hunter Serotonin Toxicity Criteria. The use of mechanical ventilation or any other ventilation/oxygen support (high-flow nasal cannula, non-invasive ventilation, use of supplementary oxygen) will be collected daily until day 28 or until hospital discharge, whichever comes first. Vital status at ICU and hospital discharge and any other relevant clinical data such as nosocomial infections, insulin use for glycemic control, antibiotic use, and other therapies for COVID-19 (hydroxychloroquine, chloroquine, and azithromycin) will be collected. Data on mechanical ventilation will be collected in specific forms with data on the date and time of initiation and discontinuation of therapy.

At 3, 6, 9, and 12 months after entering the study, patients will be assessed by structured telephone interview and with a validated instrument in relation to health-related quality of life using the EQ-5D-3L instrument. Patient will also be asked if they are using any home oxygen or ventilatory support and if they have been readmitted to the hospital for any cause since discharge. If the patient is unable to respond to his/her clinical conditions, telephone follow-up will be carried out with the family member or person responsible for the patient's care.

Patients will be followed up to 28 days while in the hospital from treatment intervention with assessments daily up to 28 days and on day of discharge or death from hospital, whichever occurs first.

### Statistical Methods

Outcomes: The main analysis study population will comprise all patients who have been randomized (intention-to-treat population), using the group allocated as variable, regardless of the medication administered.

Patients discharged from the hospital alive before 28 days will be counted. Number of days of hospitalization will be presented as mean and standard deviation. The treatment effect will be presented as mean difference, with 95% confidence interval and P-value. A generalized linear model with beta-binomial distribution or zero/one inflated beta distribution can be used, with center as random effect and adjusted for age, use of THC and fluvoxamine composition or combination before randomization and PaO₂/FiO₂ ratio. In case of loss of follow-up, missing data on the primary outcome will be dealt with using the multiple imputations technique.

All-cause mortality rates at 28 days will be analyzed using a mixed Cox model with centers as random effects (frailty model). The treatment effect on SOFA score at baseline, 3, 7, 14, and 28 days after treatment intervention will be analyzed by a linear mixed model with centers as random effects. For the clinical status of patients, an ordinal logistic regression will be used, the results will be presented as proportional odds ratio comparing two combinations: Intervention versus Control. The probability ratios will be derived from a mixed logistic regression of proportional probabilities adjusted for age and PaO₂/FiO₂ ratio, with random intercepts for the center. The cumulative ordinal scores will be presented separately, as well as the main secondary results. Each odds ratio will be estimated using mixed logistic regression. The same models will be used to compare the effects of treatment on the follow-up. In case of the proportional odds assumption is not met, categories of the Ordinal Scale 1-4 will be grouped as a single category for the analysis. All secondary outcomes will be exploratory and no adjustment for multiple testing will be made.

Adverse events will be expressed as counts and percentages and compared between groups using Chi-squared test. The significance level for all analyses will be 0.05. There will be no adjustment for multiple testing. All the analyses will be performed using the R software (R Core Team, Vienna, Austria, 2020).

### Additional analysis:

### Sensitivity analysis:

Analyses can be performed to assess treatment effects on the primary and secondary outcomes considering only patients that received the proposed treatment in the intervention group and patients that did not receive the THC/fluvoxamine composition or combination in the control group. Additionally, a sensitivity analysis can be performed for the primary outcome in the following groups:
- Confirmed COVID-19 infection
- Confirmed and probable COVID-19 infection
- Patients which received the THC/fluvoxamine composition or combination and patients which did not receive the THC/fluvoxamine composition or combination
- Patients who received the proposed treatment in the intervention group and patients who did not receive the treatment in the control group.

**Table 3 The 6-point Ordinal Scale**

| | |
|---|---|
| 1 | Not hospitalized. |
| 2 | Hospitalized, not requiring supplemental oxygen. |
| 3 | Hospitalized, requiring supplemental oxygen. |
| 4 | Hospitalized, requiring non-invasive ventilation or nasal high-flow oxygen therapy |
| 5 | Hospitalized, requiring invasive mechanical ventilation or ECMO |
| 6 | Death |

### Example 3: Formulation of THC and Fluvoxamine

An exemplary formulation of Δ9-THC and fluvoxamine is provided herein. The FDA approved dronabinol formulation can be used, optionally where the carriers and/or excipients can be adjusted. Excipients such as glycerin, iron oxide yellow, gelatin and titanium dioxide can be removed from the dronabinol formulation.

Fluvoxamine can be used in the form of a pharmaceutically acceptable salt thereof. For the purpose of this exemplary formulation, fluvoxamine maleate is used.

Fluvoxamine and THC are dissolved or suspended in a suitable carrier such as sesame seed oil. It can be appreciated that since Δ9-THC is hydrophobic, a hydrophobic carrier such as sesame seed oil may be suitable. Fluvoxamine in contrast is more hydrophilic but can still be suspended in carriers such as sesame seed oil. Fluvoxamine is either dissolved or suspended in sesame seed oil in less than about 5 nm deflocculated particles. Fluvoxamine particles can be obtained by any suitable means known in the art. For this exemplary formulation, fluvoxamine is milled to about 0.5 microns. The fluvoxamine particles are suspended in sesame seed oil to create a coarse suspension.

Since sesame seed oil is currently used in a commercially available Δ9-THC formulation, it is not expected to adversely affect the pharmacokinetic properties of Δ9-THC. It can be appreciated that this formulation would not negatively affect the absorption of fluvoxamine since there are no contraindication to taking fluvoxamine including fluvoxamine maleate with food such as sesame seed oil. Further, it is believed that if lipids or oils could interfere with the bioavailability of fluvoxamine, this would have been demonstrated in the original bioavailability studies conducted in humans and resulted in a recommendation to restrict administration the medication with food.

THC and fluvoxamine can be formulated in a HPMC capsule. For this exemplary formulation, each capsule comprises about 5.25 mg of Δ9-THC and about 148 mg of fluvoxamine maleate. It is contemplated that other dosages and unit doses can be used as described herein. It is contemplated that other dosages and unit doses can be used as described herein such as a capsule containing 6.0 mg of THC and 120 mg of fluvoxamine maleate, a capsule containing 6.0 mg of THC and 180 mg of fluvoxamine maleate, or a capsule containing 12.5 mg THC and 180 mg of fluvoxamine maleate. Additionally, a capsule containing 6.25 mg of THC and 134 mg of fluvoxamine maleate or a capsule containing 12.5 mg of THC and 166 mg of fluvoxamine maleate can be used.

Based on the available storage and handling information of fluvoxamine and Δ9-THC, the capsule comprising the composition of Δ9-THC and fluvoxamine as described herein can for example be stored at a temperature of about 8°C and 15°C, or alternatively be stored in a refrigerator, while protecting from freezing.

### Example 4: Nanovesicle Formulation of THC and Fluvoxamine

THC and fluvoxamine can be formulated using nanovesicles. One or more of the active pharmaceutical ingredients (API) can be contained in nanovesicles, for example lipid bilayer spheres. In this instance, since Δ9-THC is dissolved in for example sesame seed oil, it may be beneficial to use reverse nanovesicle where the interior environment of the nanovesicle is hydrophilic.

### Example 5: Pharmacology of THC and Fluvoxamine

### Summary

### Nonclinical pharmacology

Δ9-THC activates the CB2 receptors present on leukocytes to downregulate response to IL-6. Further, without wishing to be bound to theory, THC likely potentiates CB2 receptor reliant viral clearing in the spleen. THC also decreases IL-6 secretion. Fluvoxamine binds the non-σ₁ opioid receptor preventing fusion of the endoplasmic reticulum with the cell membrane, which is believed to prevent secretion of both IL-6 and viral particles from infected cells.

### Pharmacokinetics

Δ9-THC is 90-95% absorbed after oral dose and 10-20% reaches systemic circulation with a large 2 compartment volume of distribution of 10 L/kg with significant lipid distribution. Fluvoxamine has 53% bioavailability after oral dose with a 25 L/kg distribution with 80% bound to plasma proteins.

### Toxicology

The lowest reported fatal dose of fluvoxamine is 1400 mg. Overdose can be managed with gastric lavage, activated charcoal and supportive care. Fluvoxamine has very rarely caused serotonin syndrome, which can be treated with cytoheptadine. It has very rarely caused neuroleptic malignant syndrome which can be treated with dantrolene. Δ9-THC has not had a reported fatality from overdosage. At high doses side effects of Δ9-THC can include urinary retention and postural hypotension which can be treated symptomatically.

### Clinical Experience

As described herein, the use of Δ9-THC in addition to standard of care in 77 consecutive patients hospitalised for hypoxia caused by COVID-19 showed a statistically significant 68% reduction in mortality in comparison to 44 consecutive patients who received only the standard of care.

### Pharmacodynamics

Δ9-THC-induced sympathomimetic activity may result in tachycardia and/or conjunctival injection. Its effects on blood pressure are inconsistent, but occasional subjects have experienced orthostatic hypotension and/or syncope upon abrupt standing.

Δ9-THC also demonstrates reversible effects on appetite, mood, cognition, memory, and perception. These phenomena appear to be dose-related, increasing in frequency with higher dosages, and subject to great interpatient variability. After oral administration, Δ9-THC has an onset of action of approximately 0.5 to 1 hours and peak effect at 2 to 4 hours. Duration of action for psychoactive effects is 4 to 6 hours, but the appetite stimulant effect of Δ9-THC may continue for 24 hours or longer after administration. Tachyphylaxis and tolerance develop to some of the pharmacologic effects of Δ9-THC and other cannabinoids with chronic use, suggesting an indirect effect on sympathetic neurons. In a study of the pharmacodynamics of chronic Δ9-THC exposure, healthy male volunteers (N = 12) received 210 mg/day Δ9-THC, administered orally in divided doses, for 16 days. An initial tachycardia induced by Δ9-THC was replaced successively by normal sinus rhythm and then bradycardia. A decrease in supine blood pressure, made worse by standing, was also observed initially. These volunteers developed tolerance to the cardiovascular and subjective adverse CNS effects of Δ9-THC within 12 days of treatment initiation. Tachyphylaxis and tolerance do not, however, appear to develop to the appetite stimulant effect of Δ9-THC Capsules. In studies involving patients with Acquired Immune Deficiency Syndrome (AIDS), the appetite stimulant effect of Δ9-THC Capsules has been sustained for up to five months in clinical trials, at dosages ranging from 2.5 mg/day to 20 mg/day.

The mechanism of action of fluvoxamine in obsessive compulsive disorder is presumed to be linked to its specific serotonin reuptake inhibition in brain neurons. Receptor binding studies have demonstrated that fluvoxamine is a potent serotonin reuptake inhibitor in vitro as well as in vivo. In preclinical studies, it was found that fluvoxamine inhibited neuronal uptake of serotonin. In in vitro studies fluvoxamine had no significant affinity for histaminergic, alpha or beta adrenergic, muscarinic, or dopaminergic receptors. Antagonism of some of these receptors is thought to be associated with various sedative, cardiovascular, anticholinergic, and extrapyramidal effects of some psychotropic drugs.

### Pharmacokinetics

Absorption and Distribution: Δ9-THC is almost completely absorbed (90 to 95%) after single oral doses. Due to the combined effects of first pass hepatic metabolism and high lipid solubility, only 10 to 20% of the administered dose reaches the systemic circulation. Δ9-THC has a large apparent volume of distribution, approximately 10 Ukg, because of its lipid solubility. The plasma protein binding of Δ9-THC and its metabolites is approximately 97%. The elimination phase of Δ9-THC can be described using a two-compartment model with an initial (alpha) half-life of about 4 hours and a terminal (beta) half-life of 25 to 36 hours. Because of its large volume of distribution, Δ9-THC and its metabolites may be excreted at low levels for prolonged periods of time. The pharmacokinetics of Δ9-THC after single doses (2.5, 5, and 10 mg) and multiple doses (2.5, 5, and 10 mg given twice a day; BID) have been studied in healthy women and men. A slight increase in dose proportionality on mean Cmax and AUC (0-12) of Δ9-THC was observed with increasing dose over the dose range studied.

A summary of multi-dose pharmacokinetics parameters of Δ9-THC in healthy volunteers (n=34; 20 to 45 years old) under fasted conditions is provided in Table 4.

**Table 4 Summary of pharmacokinetics of Δ9-THC in healthy volunteers**

| Mean (SD) PK Parameter Values | | | |
|---|---|---|---|
| BID Dose | Cmax ng/mL | Median Tmax (range), hr | AUC (0-12) ng*hr/mL |
| 2.5 mg | 1.32 (0.62) | 1.00 (0.50-4.00) | 2.88 (1.57) |
| 5 mg | 2.96 (1.81) | 2.50 (0.50-4.00) | 6.16 (1.85) |
| 10 mg | 7.88 (4.54) | 1.50 (0.50-3.50) | 15.2 (5.52) |

### Metabolism

### Δ9-THC

Δ9-THC undergoes extensive first-pass hepatic metabolism, primarily by microsomal hydroxylation, yielding both active and inactive metabolites. Δ9-THC and its principal active metabolite, 11-OH-delta-9-THC, are present in approximately equal concentrations in plasma. Concentrations of both parent drug and metabolite peak at approximately 0.5 to 4 hours after oral dosing and decline over several days. Values for clearance average about 0.2 L/kg-hr, but are highly variable due to the complexity of cannabinoid distribution. Elimination: Δ9-THC and its biotransformation products are excreted in both feces and urine. Biliary excretion is the major route of elimination with about half of a radio-labeled oral dose being recovered from the feces within 72 hours as contrasted with 10 to 15% recovered from urine. Less than 5% of an oral dose is recovered unchanged in the feces. Following single dose administration, low levels of Δ9-THC metabolites have been detected for more than 5 weeks in the urine and feces. In a study of Δ9-THC Capsules involving AIDS patients, urinary cannabinoid/creatinine concentration ratios were studied bi-weekly over a six-week period. The urinary cannabinoid/creatinine ratio was closely correlated with dose. No increase in the cannabinoid/creatinine ratio was observed after the first two weeks of treatment, indicating that steady state cannabinoid levels had been reached. This conclusion is consistent with predictions based on the observed terminal half-life of Δ9-THC. Special Populations: The pharmacokinetic profile of Δ9-THC has not been investigated in either pediatric or geriatric patients. Δ9-THC is metabolized primarily by the CYP2C9, CYP 2C19 and CYP3A4 P450 enzyme of the liver. Δ9-THC may inhibit CYP 2D6.

### Fluvoxamine

The absolute bioavailability of fluvoxamine maleate is 53%. Oral bioavailability is not significantly affected by food. In a dose proportionality study involving fluvoxamine maleate at 100, 200 and 300 mg/day for 10 consecutive days in 30 normal volunteers, steady state was achieved after about a week of dosing. Maximum plasma concentrations at steady state occurred within 3-8 hours of dosing and reached concentrations averaging 88, 283 and 546 ng/mL, respectively. Thus, fluvoxamine had nonlinear pharmacokinetics over this dose range, i.e., higher doses of fluvoxamine maleate produced disproportionately higher concentrations than predicted from the lower dose. Distribution/Protein Binding: The mean apparent volume of distribution for fluvoxamine is approximately 25 Ukg, suggesting extensive tissue distribution. Approximately 80% of fluvoxamine is bound to plasma protein, mostly albumin, over a concentration range of 20 to 2000 ng/mL. Metabolism: fluvoxamine maleate is extensively metabolized by the liver; the main metabolic routes are oxidative demethylation and deamination. Nine metabolites were identified following a 5 mg radiolabelled dose of fluvoxamine maleate, constituting approximately 85% of the urinary excretion products of fluvoxamine. The main human metabolite was fluvoxamine acid which, together with its N-acetylated analog, accounted for about 60% of the urinary excretion products. A third metabolite, fluvoxamine ethanol, formed by oxidative deamination, accounted for about 10%. fluvoxamine acid and fluvoxamine ethanol were tested in an in vitro assay of serotonin and norepinephrine reuptake inhibition in rats; they were inactive except for a weak effect of the former metabolite on inhibition of serotonin uptake (1-2 orders of magnitude less potent than the parent 3 compound). Approximately 2% of fluvoxamine was excreted in urine unchanged. Elimination: Following a 14C-labelled oral dose of fluvoxamine maleate (5 mg), an average of 94% of drug-related products was recovered in the urine within 71 hours. The mean plasma half-life of fluvoxamine at steady state after multiple oral doses of 100 mg/day in healthy, young volunteers was 15.6 hours. Fluvoxamine moderately inhibits the CYP3A4, CYP2C9, and CYP2D6 P450 enzyme of the liver. Fluvoxamine strongly inhibits CYP 1AC and CYP 2C19. Fluvoxamine is metabolized by CYP 2D6.

Elderly Subjects: In a study of fluvoxamine Tablets at 50 and 100 mg comparing elderly (ages 66- 73) and young subjects (ages 19-35), mean maximum plasma concentrations in the elderly were 40% higher. The multiple dose elimination half-life of fluvoxamine was 17.4 and 25.9 hours in the elderly compared to 13.6 and 15.6 hours in the young subjects at steady state for 50 and 100 mg doses, respectively. In elderly patients, the clearance of fluvoxamine was reduced by about 50% and, therefore, fluvoxamine Tablets should be slowly titrated during initiation of therapy. Pediatric Subjects: The multiple-dose pharmacokinetics of fluvoxamine were determined in male and female children (ages 6-11) and adolescents (ages 12-17). Steady-state plasma fluvoxamine concentrations were 2-3 fold higher in children than in adolescents. AUC and Cmax in children were 1.5- to 2.7-fold higher than that in adolescents. As in adults, both children and adolescents exhibited nonlinear multiple-dose pharmacokinetics. Female children showed significantly higher AUC (0-12) and Cmax compared to male children and, therefore, lower doses of fluvoxamine Tablets may produce therapeutic benefit. No gender differences were observed in adolescents. Steady-state plasma fluvoxamine concentrations were similar in adults and adolescents at a dose of 300 mg/day, indicating that fluvoxamine exposure was similar in these two populations. Dose adjustment in adolescents (up to the adult maximum dose of 300 mg) may be indicated to achieve therapeutic benefit.

Hepatic and Renal Disease: A cross study comparison (healthy subjects versus. patients with hepatic dysfunction) suggested a 30% decrease in fluvoxamine clearance in association with hepatic dysfunction. The mean minimum plasma concentrations in renally impaired patients (creatinine clearance of 5 to 45 mL/min) after 4 and 6 weeks of treatment (50 mg b.i.d., N = 13) were comparable to each other, suggesting no accumulation of fluvoxamine in these patients.

### Example 6: Preparation of a Capsule of THC and Fluvoxamine

An exemplary formulation of a capsule comprising Δ9-THC and fluvoxamine is shown in Table 5.

**Table 5 Exemplary Batch Formulation of Δ9-THC and Fluvoxamine**

| **Ingredient Listing** | **Qty** | **Uni t** | **NDC#** | **Processi ng factor: 0.1** | **Qty. to Measu re** |
|---|---|---|---|---|---|
| **Fluvoxamine** Maleate, USP | 14.8 | gm | CAS # 61718-82-9 | 0.1 | 16.28 gm |
| Dronabinol 20% in Sesame Oil | 2.625 | mL | CAS # 1972-08-3 | 0.1 | 2.9 mL |
| Sesame Oil, NF | 29.575 | mL | CAS # 8008-74-0 | 0.1 | 32.5 mL |
| Silicon Dioxide, NF | 1 | gm | CAS # 7631-86-9 | 0.1 | 1.1 gm |
| Sodium Chloride, USP | As Needed | gm | CAS # 7647-14-5 | n/a | n/a |

An exemplary manufacturing process for a capsule comprising Δ9-THC and fluvoxamine is described herein. It can be appreciated that specific steps in the manufacturing process can be substituted with other formulation and manufacture processes known in the art.

| | |
|---|---|
| Non-Sterile Preparation | Yes, oral non-aqueous oral formulation |
| Processing Error | To account for processing error during capsule filling measure an additional 10% of the required quantities of ingredients |
| Special Instructions | Protective apparel, such as a lab coat, disposable gloves, and face-masks should always be worn. |

Powder preparation: By geometric addition, combine and mix the following ingredients together to form a homogeneous powder blend: fluvoxamine maleate and silicon dioxide.
- End result: homogeneous powder blend.

### Liquid preparation:

- Slowly add Dronabinol Sesame Oil to the additional Sesame Oil in a glass beaker on a magnetic stir plate.
- Add magnetic stir bar to glass beaker containing Oil Suspension and increase the spin rate to allow for continuous, non-turbulent, uniform mixing.
- End Result: Homogenous liquid dispersion.

Powder to Medium Incorporation: Incrementally add the homogenous powder blend to the homogenous liquid dispersion.
- Specifications: Continuously mix, ensuring uniform distribution of ingredients.
- End Result: Homogenous liquid-like dispersion.

Validation Technique (average capsule weight): The final weight of each capsule should fall between 90 and 110% of the theoretically calculated weight, in accordance to USP 795 guidelines. The theoretical calculated weight can be determined by adding the amount of Fluvoxamine maleate (148 mg), Dronabinol Oil (24.1 mg), Sesame Oil (271.6 mg) and Silicone Dioxide (10 mg) in each capsule to the vegetable (Hypromellose) capsule shell weight (79 mg) for a total weight of 532.7 mg.

Product Transfer: Transfer the final product into the specified dispensing container.

Stability of capsule: if the formulation is used in an anhydrous form, it can be expected that the stability of the anhydrous formulation can be increased for example for at least 6 months.

Appearance of capsule: exemplary appearance of the capsule could be half red (iron oxide), half blue (iron oxide), and stamped with black (iron oxide) 2.25 numbering. However, other appearance of the capsule can be used as well.

### Example 7: Pharmacology of THC and Fluvoxamine

### Primary Pharmacodynamics

THC: Non-clinical work has shown that delta 9 THC, the API in dronabinol, acts as an agonist at CB1 and CB2 receptors. CB2 receptors are present on most leukocytes. They are believed to have an immune modulatory role. Specifically, they have been shown to suppress leukocyte response to IL-6. THC has been shown to stimulate CB2 receptors to suppress cytokine storm in mice when the cytokine storm was triggered by immunogenic bacterial proteins. This action appears to have been mediated by inducing apoptosis in leukocytes via a mitochondrial pathway. Comparison of the findings in these mice with the findings in bronchioalveloar washings taken from COVID-19 patients who required bronchoscopy suggest similar pathways. This suggests THC would be useful as an immunomodulator in humans, as demonstrated in the clinical study of Example 1.

THC may play a role as an antiviral as well. It is documented that CB2 receptor activity is necessary for proper marginal zone B-cell function (these B-cell reside at the border of the red pulp and white pulp of the spleen, the marginal zone)²⁹. CB2 receptor function has been shown to be required for modulating the action of B-cells which then pass antigens to macrophages which in turn pass the antigens to T-cells to modulate immunity³⁰. This activity has been shown to be vital/beneficial in clearing infections of several viruses, including cowpox, CMV, and adenovirus.⁷³¹ Taken together, this information suggests CB2 receptors are important for antiviral defense and that CB2 activation may promote antiviral defense. This suggests that THC may have activity as an antiviral. Activity such as this may have contributed to the dramatic clinical benefit observed in COVID-19 patients who received THC when compared to controls.

Fluvoxamine: Non-clinical work has shown that fluvoxamine has the strongest agonistic sigma1 binding affinity of all pharmaceutical chemicals tested so far. Sigma1 agonists have been shown to interfere with fusion of the endoplasmic reticulum with the cell membrane. This is theorized to interfere with the ability of viral particles to be released from an infected cell (antiviral activity). It has been demonstrated that this limits the ability of IL-6 to be secreted (anti-inflammatory activity). It has been theorized that the Sigma1 affinity makes the fluvoxamine lysosomotropic, and its presence in the lysosome prevents the required acidification necessary for fusion of the lysosome with the cell-membrane allowing release of intracellular contents. It is also theorized that fluvoxamine interferes with Ceramide-16 synthesis and possibly Ceramide-18 synthesis. It is believed that fluvoxamine accumulates in pulmonary tissue in the setting of COVID-19.³² A mouse model has shown beneficial effects of fluvoxamine in models of inflammation and sepsis.³³

### Synergy

Without wishing to be bound by theory, THC and fluvoxamine share at least the following common and/or complementary features in their mechanism of action:
- reduce inflammation through differing mechanisms both targeting IL-6
- reduce viral load by differing mechanism, both having shown clinical efficacy against COVID-19.
- each has secondary pharmacodynamics that ameliorate side effects of the other. Fluvoxamine's primary side-effect is nausea, many forms of which are well-treated by THC. THC can often cause anxiety, many forms of which are well treated by fluvoxamine.
- use of the API's together should improve outcomes and reduce side-effects.
- THC is a CB2 agonist. CB2 is important for B cell role in presentation of viral antigen to T cells, which assists in increasing viral clearance. As such, it is expected that THC can be an effective antiviral agent. On the other hand, fluvoxamine is an antiviral that acts through a mechanism of action different from CB2 agonism. Therefore, it is expected that the combination of THC and fluvoxamine can be synergistic as an antiviral combination therapy.

Caution must be used in dosing the drugs together since:
- THC is metabolized by CYP2C9, CYP2C19 and CYP34A, all of which are inhibited by fluvoxamine (CYP2C19 strongly and CYP2C9 and CYP34A moderately). Fluvoxamine is metabolized by CYP2D6 which may be inhibited by Δ9-THC.
- Both THC and fluvoxamine are highly bound by albumin, creating the potential that presence of one causes an increased free serum content of the other.
- The two factors above would argue for a decrease in the dosing of both drug when used together.

THC causes apoptosis of leukocytes in mice, causing reduction in the cytokine storm. Fluvoxamine is predicted to potentially inhibit apoptosis suggesting a higher dose of THC would be required than might otherwise be expected in order to ensure its effectiveness.

### Example 8: THC plus Fluvoxamine Outperforms THC Alone in Improving Outcomes in COIVD-19 Hospitalized Patients for Hypoxia

In Example 1, it was shown that mortality and length of hospital stay for COVID-19 patients hospitalized for hypoxia could be significantly reduced by the administration of oral dronabinol. The hypothesis that improved efficacy with lower dosage can be achieved by concomitant administration of fluvoxamine was then evaluated.

### Materials and Methods

From October 2020 through December of 2020 a single infectious disease specialist treated consecutive adult COVID-19-positive patients hospitalized for hypoxia with oral dronabinol in addition to then current standard of care which consisted of ceftriaxone, azithromycin, dexamethasone, remdesivir, subcutaneous heparin, and convalescent plasma in addition to respiratory support per standard ICU protocols. Nine patients received 2.5 mg twice orally daily and 68 patients received 5 mg orally twice daily. A retrospective control group of consecutively treated COVID-19-positive patients hospitalized for hypoxia from June 2020 through August 2020, was treated with the same standard of care. The patients were similar in terms of age, gender and number of co-morbidities except that the 2.5 mg group had a larger number of female patients, and the control group was younger. The patients in the group that received dronabinol had a 68% reduction in mortality and a 37% reduction in length of hospital stay when compared to patients that did not receive dronabinol. The data were found to be statistically significant by a PhD statistician from a local medical school. There was a 93.5% survival rate in the 5 mg treated group. The 2.5mg group had a 89% survival rate compared to the group who received none which had a 79.5% survival rate. Fluvoxamine is a generic selective serotonin reuptake inhibitor that has an indication in the US for the treatment of obsessive compulsive disorder, and in other countries has indications for use in the treatment of depression. Fluvoxamine is known to be the strongest agonist known of the sigma-1 non-opioid receptor. Activation of this receptor on a cell interferes with the cell's ability to fuse its endoplasmic reticulum with the cell membrane.³⁴ This is responsible for the drug's effectiveness in OCD. Without wishing to be bound by theory, it appears this is may also be effective in preventing release for viral particles from the cell and in preventing the release of IL-6 from the cell.

The two drugs together have synergistic effects on IL-6 suppression, as well as suppression of viral replication. The two drugs are metabolized by the same cytochrome enzyme Fluvoxamine inhibits it is believed the metabolism of dronabinol in the liver, indicating that dosing of dronabinol in the presence of fluvoxamine may be less than might otherwise be required to achieve a target blood concentration than that required if fluvoxamine were not present.

When administered together, fluvoxamine may allow for a larger dose of dronabinol to be tolerated by the patient than might be tolerated by the patient had fluvoxamine not been present since it suppresses anxiety, which is sometimes associated with dronabinol use.

The most common side effect of fluvoxamine is nausea present in >10% of patients who used the drug. ²² Dronabinol is an effective treatment for nausea ²² caused by many different etiologies and may allow better tolerance of higher doses of fluvoxamine than might be otherwise tolerated without the presence of dronabinol. This provides justification for starting patients at a higher dose of fluvoxamine than is done normally in the treatment of OCD.

From October 5, 2021, through October 15, 2021, twelve consecutive unvaccinated COVID-19-positive patients hospitalized for hypoxia were treated by a single physician. They received current standard of care which consisted of ceftriaxone, azithromycin, dexamethasone, remdesivir and Eliquis in addition to respiratory support per standard ICU protocols. The patients additionally received 2.5 mg dronabinol and 100 mg fluvoxamine maleate. The patients were similar in gender distribution to the group that received 2.5 mg dronabinol in 2020 but were significantly younger and had more co-morbidities. One patient was excluded from analysis due to pre-COVID-19 oxygen dependence, and because she was still in the hospital as of this writing on her 12th hospital day.

### Results

**Table 6: Comparative analysis of results for groups of patients administered 2.5 mg dronabinol, 5 mg dronabinol, or 2.5 mg dronabinol and 100 mg fluvoxamine.**

| Regimen | # Survived | Total # in Groups | % Female | Age | Co-morbidities (Avg) | LOS | Survival Rate |
|---|---|---|---|---|---|---|---|
| Control Group | 35 | 44 | 39% | 61 | 2.2 | 15.6 | 79.5% |
| 2.5 mg Dronabinol | 8 | 9 | 67 % | 74 | 3.0 | 17.4 | 88.9% |
| 5 mg Dronabinol | 64 | 68 | 29% | 73 | 3.3 | 8.8 | 94.1% |
| 2.5mg Dronabinol +100 mg Fluvoxamine | 11 | 11 | 73% | 49 | 4.8 | 6.5 | 100.0% |

Most of the morbidity and mortality caused by COVID-19 occurs because the infection triggers an overactive immune response in many patients. This immune response is called a cytokine storm due to the large expression of cytokines in the blood, interstitial fluid and alveolar lavage fluid of hospitalized COVID-19 patients. The primary cytokine driving this activity is interleukin-6 (IL-6).

Example 1 shows that CB2 receptor activation with oral dronabinol significantly reduced mortality and length of hospital stay in COVID-19-positive patients hospitalized for hypoxia. The data of Example 1 revealed that 6.5% of patients treated with dronabinol still died in spite of the treatment. Data on CRP levels when available indicated a trend towards initial reduction after starting dronabinol. In those patients who died, the CRP levels were seen to start to rise again in spite of continued dronabinol administration.

Without wishing to be bound by theory, the mechanism of action of fluvoxamine appears to be agonism of the intracellular sigma-1 receptor which is thought to prevent intracellular vesicles from fusing with the cell membrane and releasing their contents outside of the cell. Thus, without wishing to be bound by theory, in patients treated with fluvoxamine it is thought that intracellular vesicles containing IL-6 or fully formed viral particles are prevented from releasing IL-6 and causing further inflammation and intracellular vesicles containing fully formed viral particles are prevented from releasing those particles and causing further infection.

The data provided in Table 6 indicates a statistically significant finding (p=0.015 T-test) of reduced length of hospital stay and a trend toward reduced mortality in patients treated with low doses of fluvoxamine and dronabinol. These early data surpass the results of higher dose dronabinol alone treatment. This supports the hypothesis that dronabinol and fluvoxamine together are effective in treating COVID-19-positive patients hospitalized for hypoxia and that the addition of a low dose of fluvoxamine allows for a lower dose of dronabinol than was required previously in order to achieve the same effect. As shown in Table 6, administration of a low dose of THC (2.5 mg) in combination with a low dose of fluvoxamine (100 mg) results in improved outcomes as compared to administration of only a low dose of THC (2.5 mg) or administration of a higher dose of THC (5 mg) even though the group of patients administered the combination of 2.5 mg and 100 mg fluvoxamine had more co-morbidities than in either of the groups of patients administered only THC.

While the present disclosure has been described with reference to examples, it is to be understood that the scope of the claims should be given the broadest interpretation consistent with the description as a whole.

### References:

1. Covid19.who.int. 2020. WHO Coronavirus Disease (COVID-19) Dashboard. [online] Available at: <https://covid19.who.int/> [Accessed 20 December 2020].
2. Parasher A. COVID-19: Current understanding of its pathophysiology, clinical presentation and treatment. Postgrad Med J. 2020 Sep 25:postgradmedj-2020-138577. doi: 10.1136/postgradmedj-2020-138577. Epub ahead of print. PMID: 32978337.
3. Windsor, M., 2019. Here's A Playbook for Stopping Deadly Cytokine Storm Syndrome - The Reporter. [online] Uab.edu. Available at: <https://www.uab.edu/reporter/know-more/publications/item/8909-here-s-a-playbook-for-stopping-deadly-cytokine-storm-syndrome> [Accessed 14 May 2020].
4. Coomes EA, Haghbayan H. Interleukin-6 in Covid-19: A systematic review and meta-analysis. Rev Med Virol. 2020 Nov;30(6):1-9. doi: 10.1002/rmv.2141. Epub 2020 Aug 26. PMID: 32845568; PMCID: PMC7460877.
5. Tanaka T, Narazaki M, Kishimoto T. Immunotherapeutic implications of IL-6 blockade for cytokine storm. Immunotherapy. 2016 Jul;8(8):959-70. doi: 10.2217/imt-2016-0020. PMID: 27381687.
6. Guaraldi G, Meschiari M, Cozzi-Lepri A, Milic J, Tonelli R, Menozzi M, Franceschini E, Cuomo G, Orlando G, Borghi V, Santoro A, Di Gaetano M, Puzzolante C, Carli F, Bedini A, Corradi L, Fantini R, Castaniere I, Tabbì L, Girardis M, Tedeschi S, Giannella M, Bartoletti M, Pascale R, Dolci G, Brugioni L, Pietrangelo A, Cossarizza A, Pea F, Clini E, Salvarani C, Massari M, Viale PL, Mussini C. Tocilizumab in patients with severe COVID-19: a retrospective cohort study. Lancet Rheumatol. 2020 Aug;2(8):e474-e484. doi: 10.1016/S2665-9913(20)30173-9. Epub 2020 Jun 24. Erratum in: Lancet Rheumatol. 2020 Oct;2(10):e591. PMID: 32835257; PMCID: PMC7314456.
7. Sheppard M, Laskou F, Stapleton PP, Hadavi S, Dasgupta B. Tocilizumab (Actemra). Hum Vaccin Immunother. 2017 Sep 2;13(9):1972-1988. doi: 10.1080/21645515.2017.1316909. PMID: 28841363; PMCID: PMC5612212.
8. Vink H, Duling BR. Capillary endothelial surface layer selectively reduces plasma solute distribution volume. Am J Physiol Heart Circ Physiol. 2000 Jan;278(1):H285-9. doi: 10.1152/ajpheart.2000.278.1.H285. PMID: 10644610.
9. Ono S, Egawa G, Kabashima K. Regulation of blood vascular permeability in the skin. Inflamm Regen. 2017;37:11. Published 2017 Jul 10. doi:10.1186/s41232-017-0042-9.
10. BiologicsCorp. 2020. Fab Antibody Fragment Production - Fab/Fab' Expression - Biologicscorp. [online] Available at: <https://www.biologicscorp.com/fab-fragment-antibody/#.x99n7rmSnIU> [Accessed 20 December 2020].
11. Sung JY, Hong JH, Kang HS, Choi I, Lim SD, Lee JK, Seok JH, Lee JH, Hur GM. Methotrexate suppresses the interleukin-6 induced generation of reactive oxygen species in the synoviocytes of rheumatoid arthritis. Immunopharmacology. 2000 Apr;47(1):35-44. doi: 10.1016/s0162-3109(99)00185-x. PMID: 10708808.
12. Reggio PH. Endocannabinoid binding to the cannabinoid receptors: what is known and what remains unknown. Curr Med Chem. 2010;17(14):1468-1486. doi:10.2174/092986710790980005.
13. Marinol - FDA prescribing information, side effects and uses. Drugs.com. www.drugs.com/pro/marinol.html. Published March 1, 2020. Accessed December 20, 2020.
14. Atwood BK, Straiker A, Mackie K. CB2: therapeutic target-in-waiting. Prog Neuropsychopharmacol Biol Psychiatry. 2012;38(1):16-20. doi:10.1016/j.pnpbp.2011.12.001.
15. Nagarkatti P, Pandey R, Rieder SA, Hegde VL, Nagarkatti M. Cannabinoids as novel anti-inflammatory drugs. Future Med Chem. 2009;1(7):1333-1349. doi:10.4155/fmc.09.93.
16. Klein TW, Newton C, Widen R, Friedman H. Delta 9-tetrahydrocannabinol injection induces cytokine-mediated mortality of mice infected with Legionella pneumophila. J Pharmacol Exp Ther. 1993 Nov;267(2):635-40. PMID: 7504099.
17. Mohammed A, Alghetaa H, Sultan M, Singh NP, Nagarkatti P, Nagarkatti M. Administration of Δ9-Tetrahydrocannabinol (THC) Post-Staphylococcal Enterotoxin B Exposure Protects Mice from Acute Respiratory Distress Syndrome and Toxicity. Front Pharmacol. 2020;11:893. Published 2020 Jun 16. doi: 10.3389/fphar.2020.00893.
18. Aw D, Silva AB, Palmer DB. Immunosenescence: emerging challenges for an ageing population. Immunology. 2007;120(4):435-446. doi:10.1111/j.1365-2567.2007.02555.x.
19. Gold JE, Baumgartl WH, Okyay RA, Licht WE, Fidel PL Jr, Noverr MC, Tilley LP, Hurley DJ, Rada B, Ashford JW. Analysis of Measles-Mumps-Rubella (MMR) Titers of Recovered COVID-19 Patients. mBio. 2020 Nov 20;11(6):e02628-20. doi: 10.1128/mBio.02628-20. PMID: 33219096; PMCID: PMC7686805.
20. Pradhan A, Olsson PE. Sex differences in severity and mortality from COVID-19: are males more vulnerable? Biol Sex Differ. 2020 Sep 18;11(1):53. doi: 10.1186/s13293-020-00330-7. PMID: 32948238; PMCID: PMC7498997.
21. Li L, Krajewski S, Reed JC, Choi YS. The apoptosis and proliferation of SAC-activated B cells by IL-10 are associated with changes in Bcl-2, Bcl-xL, and Mcl-1 expression. Cell Immunol. 1997 May 25;178(1):33-41. doi: 10.1006/cimm.1997.1129. PMID: 9184696.
22. Joffre J, Yeh CC, Wong E, Thete M, Xu F, Zlatanova I, Lloyd E, Kobzik L, Legrand M, Hellman J. Activation of CB1R Promotes Lipopolysaccharide-Induced IL-10 Secretion by Monocytic Myeloid-Derived Suppressive Cells and Reduces Acute Inflammation and Organ Injury. J Immunol. 2020 Jun 15;204(12):3339-3350. doi: 10.4049/jimmunol.2000213. Epub 2020 May 8. PMID: 32385136; PMCID: PMC7276941.
23. Basu A, Manning WG, Mullahy J. Comparing alternative models: log versus Cox proportional hazard? Health Econ 2004;13:749-65.
24. Nagarkatti et al., Use of Cannabinoids to Treat Acute Respiratory Distress Syndrome and Cytokine Storm Associated with Coronavirus Disease-2019, November 6, 2020, https://www.frontiersin.org/articles/10.3389/fphar.2020.589438/full.
25. Rao et al., Tetrahydrocannabinol attenuates Staphylococcal enterotoxin B-induced inflammatory lung injury and prevents mortality in mice by modulation of miR-17-92 cluster and induction of T-regulatory cells, February 10, 2015, https://www.ncbi.nlm.nih.gov/pmc/articles/PMC4376457/.
26. Mohammed et al., Administration of Δ9-tetrahydrocannabinol (THC) post-staphylococcal enterotoxin B exposure protects mice from acute respiratory distress syndrome and toxicity, June 16, 2020.https://www.frontiersin.org/articles/10.3389/fphar.2020.00893/full.
27. Mohammed et al., Protective effects of Δ9-tetrahydrocannabinol against enterotoxin-induced acute respiratory distress syndrome is mediated by modulation of microbiota, August 4, 2020, https://pubmed.ncbi.nlm.nih.gov/32754917/.
28. Mohammed et al., Δ9-tetrahydrocannabinol prevents mortality from acute respiratory distress syndrome through the induction of apoptosis in immune cells, leading to cytokine storm suppression, August 28, 2020, https://www.ncbi.nlm.nih.gov/pmc/articles/PMC7503745/.
29. Basu S, Ray A, Dittel BN (December 2011). "Cannabinoid receptor 2 is critical for the homing and retention of marginal zone B lineage cells and for efficient T-independent immune responses." Journal of Immunology. 187 (11):5720-32. doi:10.4049/jimmunol.1102195. PMC 3226756. PMID 22048769.
30. Harvey BP, Gee RJ, Haberman AM, Schlomchik MJ, Mamula MJ. Antigen presentation and transfer between B cells and macrophages. Eur J Immunol. 2007 Jul;37(7):1739-51. doi: 10.1002/eji.200636452. PMID: 17534863.
31. den Haan, Joke M.M; Kraal. Georg (2012). "Innate Immune Functions of Macrophage Subpopulations in the Spleen." Journal of Innate immunity. 4 (5-6): 437-445.
32. Blaess M, Kaiser L, Sommerfeld O, Csuk R, Deigner HP. Drugs, Metabolites, and Lung Accumulating Small Lysosomotropic Molecules: Multiple Targeting Impedes SARS-CoV-2 Infection and Progress to COVID-19. Int J Mol Sci. 2021 Feb 11;22(4):1797. doi: 10.3390/ijms22041797. PMID: 33670304; PMCID: PMC7918659.
33. Rosen DA, Seki SM, Fernandes-Casteneda A, Beiter RM, Eccles JD, Woodfolk JA, Gaultier A. "Modulation of the Sigma-1 Receptor-IRE1 Pathway Is Beneficial in Preclinical Models of Inflammation and Sepsis." Sci. Transl. Med. 2019, 11, eaau5266.
34. Vela, Jose Miguel. Repurposing Sigma-I Receptor Ligands for COVID-19 Therapy?. Front Pharmacol 2020; 11:582310. doi: 10.3389/fphar.2020.582310.
35. Mohammed, A., FK Alghetaa, H., Miranda, K., Wilson, K., P. Singh, N., Cai, G., ... & Nagarkatti, M. (2020). Δ9-tetrahydrocannabinol prevents mortality from acute respiratory distress syndrome through the induction of apoptosis in immune cells, leading to cytokine storm suppression. International Journal of Molecular Sciences, 21(17), 6244.
36. Raj V, Park JG, Cho KH, Choi P, Kim T, Ham J, Lee J. Assessment of antiviral potencies of cannabinoids against SARS-CoV-2 using computational and in vitro approaches. Int J Biol Macromol. 2021 Jan 31;168:474-485. doi: 10.1016/j.ijbiomac.2020.12.020. Epub 2020 Dec 5. PMID: 33290767; PMCID: PMC7836687.
37. Ramlall, V., Zucker, J., & Tatonetti, N. (2020). Melatonin is significantly associated with survival of intubated COVID-19 patients. MedRxiv, 2020-10.
38. Kovalchuk et al. (2021-09-30). Cannabis Sativa (Hemp and Cannabis) Products for Viral Disease Prevention and Management (PCT WO 2021/191/888).
39. Molad, A. (2021-10-21). Composition for the Treatment of Respiratory Symptoms and Methods Thereof (PCT WO 2021/209998).
40. Baban et al. (2021-11-18). Cannabidiol as a Therapeutic Modality for COVID-19 (PCT WO 2021/231810).
41. losub-Ami et al. (2021-11-11). Cannabidiolic Acid Esters for Treating Respiratiory Distress Including Acute Respiratory Distress Syndrome and Coronavirus (PCT WO 2021/224924).
42. ZHOU QIONG et al. "Interferon-alpha2b Treatment for COVID-1 9", Frontiers in Immunology, vol. 11, 15 May 2020 (2020-05-1 5), Lausanne, ISSN: 1664-3224, DOI: 10.3389/flmmu.2020.01061.
43. RANG EL-MINDEZ et al. "N-acetylcysteine as a potential treatment for COVID-1 9", Future Microbiology, vol. 15, no. 11, 1 July 2020 (2020-07-01), pages 959-962, GB ISSN: 1746-0913, DOI: 1 0.221 7/fmb-2020-0074, 43. Strichman-Almashane et al. (2020-09-24). Methods and Compositions for Treating Autism Spectrum Disorder and Associated Disorders (PCT WO 2020/1888551).
44. Hoertel et al. "SSRIs and SNRIs and Risk of Death or Intubation in COVID-1 9: Results from an Observational Study," medRxiv, 14 July 2020 (2020-07-14), pages 1-37, DOI: 10.1101/2020.07.09.20143339,
45. Lenze et al. Clinicaltrials.Gov: "NCT04342663 A Double-blind, Placebo-controlled Clinical Trial of Fluvoxamine for Symptomatic Individuals With COVID-19 Infection." 13April2020 (2020-04-13), pages 1-8,

### ABSTRACT

A method of treating acute respiratory failure (ARF) and/or acute respiratory distress syndrome (ARDS) (optionally caused in a subject afflicted with COVID-19) in a subject in need thereof, which includes administering to the subject a therapeutically effective amount of a composition or combination including a tetrahydrocannabinol (THC), derivative, intermediate, metabolite, or fragment thereof, and/or combinations thereof and a fluvoxamine, derivative, intermediate, metabolite, or fragment thereof, and/or combinations thereof, and, optionally, one or more of melatonin, an interferon (IFN), derivative, intermediate, metabolite, or fragment thereof, and/or combinations thereof, and an acetylcysteine, derivative, intermediate, metabolite, or fragment thereof, and/or combinations thereof.

## Claims

1. A composition comprising tetrahydrocannabinol (THC) and fluvoxamine, for use in treating a subject with acute respiratory failure (ARF) and/or acute respiratory distress syndrome (ARDS), optionally caused by cytokine storm.

2. The composition for use in treating, as recited in claim 1, wherein the fluvoxamine is present in the composition in an amount of about 50 mg to about 450 mg, optionally about 75 mg to about 300 mg, optionally about 50 mg, optionally about 100 mg, optionally about 134 mg, optionally about 148 mg, optionally about 150 mg.

3. The composition for use in treating, as recited in claims 1 to 2, wherein the THC is present in the composition in a concentration of from about 0.5 mg/mL to about 30 mg/mL.

4. The composition for use in treating, as recited in claims 1 to 3, wherein the THC comprises synthetic delta-9-tetrahydrocannabinol.

5. The composition for use in treating, as recited in claims 1 to 4, wherein the composition further comprises at least one of an interferon (IFN) or an acetylcysteine.

6. The composition for use in treating, as recited in claims 1 to 5, wherein the composition further comprises melatonin.

7. The composition for use in treating, as recited in claims 1 to 6, wherein the fluvoxamine and the THC are administered simultaneously, separately, or sequentially.

8. A combination for use in treating a subject with acute respiratory failure (ARF) and/or acute respiratory distress syndrome (ARDS), optionally caused by cytokine storm, the combination treatment comprising:
tetrahydrocannabinol (THC); and
fluvoxamine.

9. The combination for use in treating, as recited in claim 8, wherein the fluvoxamine is present in the combination treatment in an amount of about 50 mg to about 450 mg, optionally about 75 mg to about 300 mg, optionally about 50 mg, optionally about 100 mg, optionally about 134 mg, optionally about 148 mg, optionally about 150 mg.

10. The combination for use in treating, as recited in claims 8 to 9, wherein the THC is present in the combination treatment in a concentration of from about 0.5 mg/mL to about 30 mg/mL.

11. The combination for use in treating, as recited in claims 8 to 10, wherein the fluvoxamine and the THC are administered simultaneously, separately, or sequentially.

12. The combination for use in treating, as recited in claims 8 to 11, wherein the combination treatment further comprises melatonin.

13. The combination for use in treating, as recited in claims 8 to 12, wherein the fluvoxamine is administered in an amount sufficient to deliver a daily dose of about 100 mg to about 300 mg, about 200 mg, about 268 mg, about 296 mg, or about 300 mg of the fluvoxamine.

14. The combination for use in treating, as recited in claims 8 to 13, wherein the THC is administered in an amount sufficient to deliver a daily dose of about 1 mg to about 30 mg.

15. The combination for use in treating, as recited in claims 8 to 14, wherein the combination treatment is administered via suppository, orally, sublingually, transdermally, intramuscularly, intravenously, or intranasally.

## Patentansprüche

1. Zusammensetzung enthaltend Tetrahydrocannabinol (THC) und Fluvoxamin zur Verwendung bei der Behandlung eines Patienten mit akutem Lungenversagen (ARF) und/oder akutem Atemnotsyndrom (ARDS), insbesondere verursacht durch einen Zytokinsturm.

2. Zusammensetzung zur Verwendung gemäß Anspruch 1, wobei das Fluvoxamin in der Zusammensetzung in einer Menge von etwa 50 mg bis etwa 450 mg, insbesondere etwa 75 mg bis etwa 300 mg, vorzugsweise etwa 50 mg, vorzugsweise etwa 100 mg, vorzugsweise etwa 134 mg, vorzugsweise etwa 148 mg, vorzugsweise etwa 150 mg, enthalten ist.

3. Zusammensetzung zur Verwendung gemäß den Ansprüchen 1 oder 2, wobei das THC in der Zusammensetzung in einer Konzentration von etwa 0,5 mg/ml bis etwa 30 mg/ml enthalten ist.

4. Zusammensetzung zur Verwendung gemäß einem der Ansprüche 1 bis 3, wobei das THC synthetisches delta-9-Tetrahydrocannabinol umfasst.

5. Zusammensetzung zur Verwendung gemäß einem der Ansprüche 1 bis 4, wobei die Zusammensetzung zusätzlich zumindest Interferon (IFN) oder Acetylcystein enthält.

6. Zusammensetzung zur Verwendung gemäß einem der Ansprüche 1 bis 5, wobei die Zusammensetzung zusätzlich Melatonin enthält.

7. Zusammensetzung zur Verwendung gemäß einem der Ansprüchen 1 bis 6, wobei das Fluvoxamin und das THC gleichzeitig, getrennt oder aufeinanderfolgend verabreicht werden.

8. Kombination zur Verwendung bei der Behandlung eines Patienten mit akutem Lungenversagen (ARF) und/oder akutem Atemnotsyndrom (ARDS), insbeondere verursacht durch einen Zytokinsturm, wobei die Kombinationsbehandlung umfasst:
Tetrahydrocannabinol (THC); und
Fluvoxamin.

9. Kombination zur Verwendung gemäß Anspruch 8, wobei das Fluvoxamin in der Kombinationsbehandlung in einer Menge von etwa 50 mg bis etwa 450 mg vorliegt,
insbesondere etwa 75 mg bis etwa 300 mg, vorzugsweise etwa 50 mg, vorzugsweise etwa 100 mg, vorzugsweise etwa 134 mg, vorzugsweise etwa 148 mg, vorzugsweise etwa 150 mg.

10. Kombination zur Verwendung gemäß den Ansprüchen 8 oder 9, wobei das THC in der Kombinationsbehandlung in einer Konzentration von etwa 0,5 mg/ml bis etwa 30 mg/ml vorliegt.

11. Kombination zur Verwendung gemäß einem der Ansprüchen 8 bis 10, wobei das Fluvoxamin und das THC gleichzeitig, getrennt oder aufeinanderfolgend verabreicht werden.

12. Kombination zur Verwendung gemäß einem der Ansprüchen 8 bis 11, wobei die Kombinationsbehandlung zusätzlich Melatonin umfasst.

13. Kombination zur Verwendung gemäß einem den Ansprüchen 8 bis 12, wobei das Fluvoxamin in einer Menge verabreicht wird, die ausreicht, um eine Tagesdosis von etwa 100 mg bis etwa 300 mg, etwa 200 mg, etwa 268 mg, etwa 296 mg oder etwa 300 mg des Fluvoxamin zu liefern.

14. Kombination zur Verwendung gemäß einem der Ansprüchen 8 bis 13, wobei das THC in einer Menge verabreicht wird, die ausreicht, um eine Tagesdosis von etwa 1 mg bis etwa 30 mg zu liefern.

15. Kombination zur Verwendung bei der Behandlung gemäß den Ansprüchen 8 bis 14, wobei die Kombinationsbehandlung als Zäpfchen, oral, sublingual, transdermal, intramuskulär,intravenös oder intranasal verabreicht wird.

## Revendications

1. Composition comprenant du tétrahydrocannabinol (THC) et de la fluvoxamine, utilisée pour traitement d'un sujet souffrant d'insuffisance respiratoire aiguë (IRA) et/ou de syndrome de respiratoire aiguë (SDRA), éventuellement causé par une tempête de cytokines.

2. Composition pour utilisation dans le traitement, telle que décrite dans la revendication 1, dans laquelle la fluvoxamine est présente dans la composition en une quantité d'environ 50 mg à environ 450 mg, éventuellement d'environ 75 mg à environ 300 mg, éventuellement d'environ 50 mg, éventuellement d'environ 100 mg, éventuellement environ 134 mg, éventuellement environ 148 mg, éventuellement environ 150 mg.

3. La composition à utiliser pour le traitement, telle que décrite dans les revendications 1 à 2, dans laquelle le THC est dans la composition à une concentration d'environ 0,5 mg/mL à environ 30 mg/mL.

4. La composition à utiliser pour le traitement, telle que décrite dans les revendications 1 à 3, dans laquelle le THC comprend du delta-9-tétrahydrocannabinol synthétique.

5. La composition à utiliser pour le traitement, telle que décrite dans les revendications 1 à 4, dans laquelle la composition comprend en outre au moins un interféron (IFN) ou un acétylcystéine.

6. La composition utilisée pour le traitement, telle que décrite dans les revendications 1 à 5, dans laquelle la composition comprend en outre de la mélatonine.

7. La composition utilisée pour le traitement, telle que décrite dans les revendications 1 à 6, dans laquelle la fluvoxamine et le THC sont administrés simultanément, séparément ou séquentiellement.

8. Combinaison destinée à être utilisée dans le traitement d'un sujet souffrant d'insuffisance respiratoire aiguë (IRA) et/ou syndrome de détresse respiratoire aiguë (SDRA), éventuellement causé par une tempête de cytokines, traitement combiné comprenant:
du tétrahydrocannabinol (THC); et
de la fluvoxamine.

9. Combinaison thérapeutique décrite dans la revendication 8, dans laquelle la fluvoxamine est présente dans le traitement combiné dans une quantité d'environ 50 mg à environ 450 mg, éventuellement d'environ 75 mg à environ 300 mg, éventuellement d'environ 50 mg, éventuellement d'environ 100 mg, éventuellement d'environ 134 mg, éventuellement environ 148 mg, éventuellement environ 150 mg.

10. La combinaison à utiliser pour le traitement, telle que décrite dans les revendications 8 à 9, dans laquelle le THC est dans le traitement combiné à une concentration d'environ 0,5 mg/mL à environ 30 mg/mL.

11. La combinaison utilisée pour le traitement, telle que décrite dans les revendications 8 à 10, dans laquelle la fluvoxamine et le THC sont administrés simultanément, séparément ou séquentiellement.

12. La combinaison utilisée pour le traitement, telle que décrite dans les revendications 8 à 11, dans laquelle la combinaison traitement comprend en outre de la mélatonine.

13. La combinaison utilisée pour le traitement, telle que décrite dans les revendications 8 à 12, dans laquelle la fluvoxamine est administrée en quantité suffisante pour délivrer une dose journalière d'environ 100 mg à environ 300 mg, d'environ 200 mg, d'environ 268 mg, d'environ 296 mg ou d'environ 300 mg de fluvoxamine.

14. La combinaison pour le traitement, telle que décrite dans les revendications 8 à 13, dans laquelle le THC est administré en quantité suffisante pour délivrer une dose quotidienne d'environ 1 mg à environ 30 mg.

15. La combinaison utilisée pour le traitement, telle que décrite dans les revendications 8 à 14, dans laquelle la combinaison traitement par voie intramusculaire, par voie intraveineuse ou par voie intranasale.
